# EUROPEAN PATENT APPLICATION

(11) **EP 1 949 785 A1**
(43) Date of publication of application: **30.07.2008**
(21) Application number: 07101275.1
(22) Date of filing: 26.01.2007
(51) Int. Cl.: A01H 5/00, C12N 15/82

(54) **Use of R-genes as a selection marker in plant transformation and use of cisgenes in plant transformation**

(71) Applicant: Coöperatie AVEBE U.A., 9641 GK Veendam (NL)
(72) Inventor: The designation of the inventor has not yet been filed
(74) Representative: van Loon, C.J.J.

(57) **Abstract**

The invention relates to the field of plant transformation, in particular plant transformation of a Solanaceae, preferably of potato. The present invention provides an alternative selection method in plant transformation processes. The invention further provides a plant that has been provided with additional nucleic acid sequences but which genetically modified plant essentially consists of cis plant sequences, for example a genetically modified potato plant that has been provided with additional (essentially) potato plant sequences. Such a transgenic plant is herein further referred to as a cis-gene plant.

### Annex to the application documents - subsequently filed sequences listing

SEQUENCE LISTING

<110>  Coöperatie AVEBE U.A.

<120>  Use of R-genes as a selection marker in plant transformation and
       use of cisgenes in plant transformation

<130>  P76880EP00

<140>  EP 07101275.1
<141>  2007-01-26

<160>  50

<170>  PatentIn version 3.3

<210>  1
<211>  4
<212>  PRT
<213>  Artificial

<220>
<223>  motif


<220>
<221>  VARIANT
<222>  (2)..(2)
<223>  C,D,E,F,G,H,I,K,L,M,N,P,Q,R,S,T,V,W,Y

<400>  1

Arg Ala Leu Arg
1


<210>  2
<211>  26
<212>  DNA
<213>  artificial

<220>
<223>  primer

<400>  2
agcttggcgc gcccgggtta attaag                                          26


<210>  3
<211>  26
<212>  DNA
<213>  Artificial

<220>
<223>  primer

<400>  3
aattcttaat taacccgggc gcgcca                                          26


<210>  4
<211>  20
<212>  DNA
<213>  Artificial

<220>
<223>  primer

<400>  4
ccaatatatc ctgtcaaaca                                                    20

<210>  5
<211>  20
<212>  DNA
<213>  Artificial

<220>
<223>  primer

<400>  5
catcatcatc ccaagtacaa                                                    20

<210>  6
<211>  20
<212>  DNA
<213>  Artificial

<220>
<223>  primer

<400>  6
aagggcaaac ataaccattc                                                    20

<210>  7
<211>  20
<212>  DNA
<213>  Artificial

<220>
<223>  primer

<400>  7
ggatatattg tggtgtaaac                                                    20

<210>  8
<211>  22
<212>  DNA
<213>  Artificial

<220>
<223>  primer

<400>  8
gtaatacgac tcactatagg gc                                                 22

<210>  9
<211>  27
<212>  DNA
<213>  Artificial

<220>
<223>  primer

<400>  9
taagtttaat cagaagttgg gtaggaa                                            27

<210>  10
<211>  19
<212>  DNA
<213>  Artificial

<220>
<223>  primer

<400>  10
actatagggc acgcgtggt                                                    19


<210>  11
<211>  27
<212>  DNA
<213>  Artificial

<220>
<223>  primer

<400>  11
gttgggtagg aagcctgctc ttggaaa                                           27


<210>  12
<211>  27
<212>  DNA
<213>  Artificial

<220>
<223>  primer

<400>  12
gtatgtatgt gtagttaatg gggtagt                                           27


<210>  13
<211>  27
<212>  DNA
<213>  Artificial

<220>
<223>  primer

<400>  13
acggtttcta aattaacgta gccaata                                           27


<210>  14
<211>  21
<212>  DNA
<213>  Artificial

<220>
<223>  primer

<400>  14
gcggtcctga tcaatcgtca c                                                 21


<210>  15
<211>  30
<212>  DNA
<213>  Artificial

<220>
<223>  primer

<400>  15
ggtacctgac aggatatatt ggcgggtaaa                                        30

<210>  16
<211>  32
<212>  DNA
<213>  Artificial

<220>
<223>  primer

<400>  16
ggtacctcta gagtttacac cacaatatat cc                                    32


<210>  17
<211>  22
<212>  DNA
<213>  Artificial

<220>
<223>  primer

<400>  17
gcgggtttaa cctacttcct tt                                               22


<210>  18
<211>  22
<212>  DNA
<213>  Artificial

<220>
<223>  primer

<400>  18
cggcgcgccc gggttaatta ag                                               22


<210>  19
<211>  30
<212>  DNA
<213>  Artificial

<220>
<223>  primer

<400>  19
gatccttaat taacccgggc gcgccggtac                                       30


<210>  20
<211>  20
<212>  DNA
<213>  Artificial

<220>
<223>  primer

<400>  20
ccaatatatc ctgtcaggta                                                  20


<210>  21
<211>  22
<212>  DNA
<213>  Artificial

<220>
<223>  primer

<400>  21
agctgtgaag gcaaagatga gg                                              22

<210>  22
<211>  22
<212>  DNA
<213>  Artificial

<220>
<223>  primer

<400>  22
cgtcactgcc atcatggtag at                                              22

<210>  23
<211>  21
<212>  DNA
<213>  Artificial

<220>
<223>  primer

<400>  23
taccgctacc acttgacatt c                                               21

<210>  24
<211>  21
<212>  DNA
<213>  Artificial

<220>
<223>  primer

<400>  24
gcaccccagg ctttacactt t                                               21

<210>  25
<211>  27
<212>  DNA
<213>  Artificial

<220>
<223>  primer

<400>  25
ctctagaagt tcgagacact ggctacg                                         27

<210>  26
<211>  28
<212>  DNA
<213>  Artificial

<220>
<223>  primer

<400>  26
ggatcctgga ggagatgagt aaaagtta                                        28

<210>  27
<211>  27
<212>  DNA
<213>  Artificial

<220>
<223>  primer

<400>  27
ggatccaaac gtatttacta gcgaact                                          27


<210>  28
<211>  26
<212>  DNA
<213>  Artificial

<220>
<223>  primer

<400>  28
ggtaccaaag agacaggtgc cgttat                                           26


<210>  29
<211>  20
<212>  DNA
<213>  Artificial

<220>
<223>  primer

<400>  29
ctctgtcaac agccaaatag                                                  20


<210>  30
<211>  27
<212>  DNA
<213>  Artificial

<220>
<223>  primer

<400>  30
tttactcatc tcctccagga tccttct                                          27


<210>  31
<211>  27
<212>  DNA
<213>  Artificial

<220>
<223>  primer

<400>  31
atctcctcca ggatccttct gctcctc                                          27


<210>  32
<211>  20
<212>  DNA
<213>  Artificial

<220>
<223>  primer

<400>  32
cgctttcaga atctattact                                                  20

<210> 33
<211> 27
<212> DNA
<213> Artificial

<220>
<223> primer

<400> 33
atttcatgcg catattcccg atcaaac 27


<210> 34
<211> 27
<212> DNA
<213> Artificial

<220>
<223> primer

<400> 34
tcccgatcaa acttaaatta ctagact 27


<210> 35
<211> 29
<212> DNA
<213> Artificial

<220>
<223> primer

<400> 35
ggatccatgg gttccaaggc aattatgtt 29


<210> 36
<211> 29
<212> DNA
<213> Artificial

<220>
<223> primer

<400> 36
ggatccgaat ggctttattc atacttgtt 29


<210> 37
<211> 27
<212> DNA
<213> Artificial

<220>
<223> primer

<400> 37
attgccttgg aacccatgga tccttct 27


<210> 38
<211> 27
<212> DNA
<213> Artificial

<220>
<223> primer

<400>  38
tggaacccat ggatccttct gctcctc                                          27


<210>  39
<211>  22
<212>  DNA
<213>  Artificial

<220>
<223>  primer

<400>  39
taagcgagaa atgaataaga ag                                               22


<210>  40
<211>  22
<212>  DNA
<213>  Artificial

<220>
<223>  primer

<400>  40
gcgagacaga tgaaacgaag ta                                               22


<210>  41
<211>  8461
<212>  DNA
<213>  Solanum bulbocastanum


<220>
<221>  CDS
<222>  (2944)..(5487)

<400>  41
gatcaaaaac ggattcgggg agtgaaaagc ttaccgttag cctcaagaac gatggaaaac      60

tatcaagagt cgtctggggt tcgttcctta gctctaaaaa tcgaaaagtg cagaataaag     120

acgttttgag gcttatttac gcactacaaa aaaatcatat attgctgcaa aatttgttgt     180

agctaaatat gaaaatttcc atggctaaac ttcaatattt tcttccttag cattcgtagc     240

aagatatagc atggctaaaa gttagctaca aactttacat ggttcatggc tgagaactaa     300

tatctattgc tacaaaattt ttgagttgta gctgtactga taaaaagttt ttgccacaca     360

aaatatatat atatatatag caaataactt tattcttttg ccacgtcaaa aatgatttat     420

agttgtcttt ttagatgcag ccacaaagct attacattgt agcaacatat ttaatttggt     480

ttccatgtaa acagaaattt gtagctattt gtatagctta gtcacgtggc aatagtactc     540

gtatttagtt atgactatta aaattcatgg caataacatg aagtatttac cacaagcatt     600

ttcgaattat agctaaaatt catatcttta gccatgaata tatataattt tagctaaaaa     660

tccatttgct acgaaagtca aaatttatat acatgttttt ttacgtgata tatatagaaa     720

atttgttgca ttcaatatat aattccatta ttattagaga ttttttcgca agctttatta     780

gttccaagag ctacataatg tagctatgaa ttaattttta agatttcaaa acacttccta     840

aattacgtat aattacaatt tacaatttta aaaatagttc taattatata taactcatgt     900

aatacactat cttgtttgag tacaaagcag tatagtcttc tcttatttct ccttccacaa 960
cgttcaattg aatcttagct tgatttacac cgatcttgtc atttagatgc tttaactcca 1020
ttgatatacc tatcaaaatg atgaagtatt tcaacaccaa caatcattac caactaatta 1080
tagatgagag tgtgtgttat ataatatttc aagaagacaa caaatttatt tatatatata 1140
tcattagcta aaaaatttga aagatacaaa caaaaatatg caagaaaaaa aagtaaacaa 1200
atctataaac ttttatttct taaaataatg ttcacctcaa ttgtgacatt tttggcggtt 1260
tgcgaattat gactcctctc aacactattt aaattttaaa atcttcacaa tctgctcata 1320
aataagatgt aatatttttt aaaagatcta aaataatcta aacccaaaaa tctaagtcaa 1380
ataagtgaaa cataaattcg aaaattgtaa ataagaagat atgaacatgc cttgaatata 1440
aaaaaaccac aaaaagaaat tagcaaaaca ttctacattt tgaaatgcca aagtcttctc 1500
tctcaacatt tatctcttga gcaagaagat ttccatgtaa acttcatgtc ctttacttta 1560
agcattactt ccgatattgt tcttaccttt gtcaaggaac ctagtccatt gacctatggt 1620
ggacacagat aagctaacaa catttaatac cctaactacc attaccacaa caggtagagt 1680
acgctgaatt tttctaagtt gtgtcaccat ttaaaggaca aaaatgactc aagagtaaaa 1740
tcaatgaagc atttgctgca ggcctccaaa agttttatcg atatattttt ttttaataat 1800
ttgctcgttc ttccaaatca gttgaaactt gagttgttaa aattgatttg gtacgtctgg 1860
attttttttc aaataatacc gctccatcaa atttagatta atatgatgta atatgcacaa 1920
ttagaattgc ggacaattgt aaccaattta atgaattcaa aattatttca ttgtaacaag 1980
caaatagtaa aaaataaaat tattattatg aacaaataaa aagggacaag gcataagtac 2040
tctcctagac tatgactgaa atctcagaaa cacacataaa cttaactagg gtcctattac 2100
cccctaaact aatttaaaat ggaataaata caccacaaac ggtgacatga catagagagt 2160
gtacacactc tattgaaggc aattgattag tgcacaaatt ggacacatgt cattttttta 2220
ttgataactt tattattagt tagtacacat atttattgat aataaaaatt atatatatat 2280
aattattttt atttctttct ttgtaaaata tttattttaa tttcttttta ctttaaactt 2340
tttttatttt aatttattat gttttcactt ttgattattt aaaaaaaatt tatgtgtatt 2400
ttttaaaaaa gtaattttaa agtgtccttt aattttaatg ttttaacttt tttatgtttt 2460
atttgaattt cttcacttat tttaatatcc gttcaattta atgaatccta aattatttca 2520
ttgtaacaag taaatagaaa aaaataaaat aagtcttatg aagaaataaa aacaaatata 2580
ttttttccac aagttgtgta atgattgttg aagatgcttc cattttttaa atcttttctc 2640
aatatatatt ttccacaagt tgtgcaatga ttgctgaagt tgctttattg ttttaagact 2700
tttctctata tgtgtttttc cccaagttgt ataatggttg ttgaagatgc tttaattaaa 2760
aaaaaaaacc ttttgtttag tggaaaattt caaaaagctt tagtacatct ttgtcgtttt 2820
atccaatcgt aattctttat tcagaaacca catgtttttt ttctaatctt acttttatgt 2880
ctatcaccca ttttccaata tacagcctac tctttttttc aatcaaaact agtattccta 2940

aag atg gct gat gcc ttt cta tca ttt gca gtt caa aaa ttg ggt gat 2988
Met Ala Asp Ala Phe Leu Ser Phe Ala Val Gln Lys Leu Gly Asp
1               5                   10                  15

ttc cta ata cag aaa gtt tcc ctg cgt aaa agt ctc aga gat gaa att 3036
Phe Leu Ile Gln Lys Val Ser Leu Arg Lys Ser Leu Arg Asp Glu Ile
                20                  25                  30

aga tgg ctg ata aat gag cta ctc ttc ata cgg tct ttc ctc aga gat 3084
Arg Trp Leu Ile Asn Glu Leu Leu Phe Ile Arg Ser Phe Leu Arg Asp
            35                  40                  45

gca gaa caa aag cag tgc gga gat caa aga gtt caa caa tgg gtg ttt 3132
Ala Glu Gln Lys Gln Cys Gly Asp Gln Arg Val Gln Gln Trp Val Phe
        50                  55                  60

gag atc aac tct att gct aat gat gct gtt gct ata ctc gag act tat 3180
Glu Ile Asn Ser Ile Ala Asn Asp Ala Val Ala Ile Leu Glu Thr Tyr
    65                  70                  75

agc ttt gag gct ggt aaa ggt gct agt cgt ctc aag gct tgc act tgc 3228
Ser Phe Glu Ala Gly Lys Gly Ala Ser Arg Leu Lys Ala Cys Thr Cys
80                  85                  90                  95

ata tgt agg aag gag aag aaa ttc tac aat gtt gcc gag gag att caa 3276
Ile Cys Arg Lys Glu Lys Lys Phe Tyr Asn Val Ala Glu Glu Ile Gln
                100                 105                 110

tca ctc aag caa cga atc atg gat atc tct cgc aaa cga gag act tat 3324
Ser Leu Lys Gln Arg Ile Met Asp Ile Ser Arg Lys Arg Glu Thr Tyr
            115                 120                 125

ggt att aca aat atc aat tat aat tca gga gaa agg cca agt aat cag 3372
Gly Ile Thr Asn Ile Asn Tyr Asn Ser Gly Glu Arg Pro Ser Asn Gln
        130                 135                 140

gtt aca aca ttg agg aga act acc tca tat gta gat gaa cag gat tac 3420
Val Thr Thr Leu Arg Arg Thr Thr Ser Tyr Val Asp Glu Gln Asp Tyr
    145                 150                 155

att ttt gtt ggc ttt cag gat gtt gta caa aca ttg cta gct caa ctt 3468
Ile Phe Val Gly Phe Gln Asp Val Val Gln Thr Leu Leu Ala Gln Leu
160                 165                 170                 175

ctg aaa gca gag cct cgt cga agc gtc ctc tcc att tat gga atg ggg 3516
Leu Lys Ala Glu Pro Arg Arg Ser Val Leu Ser Ile Tyr Gly Met Gly
                180                 185                 190

ggt tta ggc aag acc act ctt gcc aga aaa ctt tac acc agt cct gat 3564
Gly Leu Gly Lys Thr Thr Leu Ala Arg Lys Leu Tyr Thr Ser Pro Asp
            195                 200                 205

ata ctc aat agc ttt cct aca cgc gct tgg ata tgt gtc tct caa gag 3612
Ile Leu Asn Ser Phe Pro Thr Arg Ala Trp Ile Cys Val Ser Gln Glu
        210                 215                 220

tac aac aca atg gat ctt ctt agg act atc ata aaa tcc atc caa ggc 3660
Tyr Asn Thr Met Asp Leu Leu Arg Thr Ile Ile Lys Ser Ile Gln Gly
    225                 230                 235

tgc gcc aag gaa act cta gat ttg ttg gaa aag atg gca gaa ata gat 3708
Cys Ala Lys Glu Thr Leu Asp Leu Leu Glu Lys Met Ala Glu Ile Asp
240                 245                 250                 255

cta gaa aat cac ctt cgt gat cta ttg aaa gaa tgc aaa tac ctt gtg 3756
Leu Glu Asn His Leu Arg Asp Leu Leu Lys Glu Cys Lys Tyr Leu Val
                260                 265                 270

gtg gtt gat gat gta tgg cag aga gaa gca tgg gag agt ttg aaa aga 3804
Val Val Asp Asp Val Trp Gln Arg Glu Ala Trp Glu Ser Leu Lys Arg
            275                 280                 285

gca ttc ccg gat ggc aag aat gga agc aga gtc att att acc acg cgc 3852
Ala Phe Pro Asp Gly Lys Asn Gly Ser Arg Val Ile Ile Thr Thr Arg
        290                 295                 300

aaa gag gat gtc gct gaa aga gta gac cac aga ggt ttt gtt cat aaa 3900
Lys Glu Asp Val Ala Glu Arg Val Asp His Arg Gly Phe Val His Lys
    305                 310                 315

ctt cgt ttc cta agt caa gaa gaa agt tgg gat ctc ttt cgt agg aaa 3948
Leu Arg Phe Leu Ser Gln Glu Glu Ser Trp Asp Leu Phe Arg Arg Lys
320                 325                 330                 335

cta ctt gat gtt cga gca atg gtt cca gaa atg gaa agt tta gct aag 3996
Leu Leu Asp Val Arg Ala Met Val Pro Glu Met Glu Ser Leu Ala Lys
                340                 345                 350

gat atg gtg gaa aag tgt aga ggc tta cct ctt gca att gtt gta ttg 4044
Asp Met Val Glu Lys Cys Arg Gly Leu Pro Leu Ala Ile Val Val Leu
            355                 360                 365

agc gga cta ctt tcg cat aaa aag ggg cta aac caa tgg caa aag gtg 4092
Ser Gly Leu Leu Ser His Lys Lys Gly Leu Asn Gln Trp Gln Lys Val
        370                 375                 380

aaa gat cac ctt tgg aag aac att aaa gaa gat aaa tct att gaa atc 4140
Lys Asp His Leu Trp Lys Asn Ile Lys Glu Asp Lys Ser Ile Glu Ile
    385                 390                 395

tct aac ata cta tcc tta agc tac aat gat ttg tca act gcg ctc aag 4188
Ser Asn Ile Leu Ser Leu Ser Tyr Asn Asp Leu Ser Thr Ala Leu Lys
400                 405                 410                 415

cag tgt ttt ctc tac ttt ggt att ttt cca gaa gat caa gtg gta aag 4236
Gln Cys Phe Leu Tyr Phe Gly Ile Phe Pro Glu Asp Gln Val Val Lys
                420                 425                 430

gct gat gac ata ata cgg ttg tgg atg gcg gag ggt ttc ata ccc aga 4284
Ala Asp Asp Ile Ile Arg Leu Trp Met Ala Glu Gly Phe Ile Pro Arg
            435                 440                 445

gga gaa gaa aga atg gag gat gtg gct gac ggc ttc ttg aat gaa ctg 4332
Gly Glu Glu Arg Met Glu Asp Val Ala Asp Gly Phe Leu Asn Glu Leu
        450                 455                 460

ata aga cga agc ttg gtt caa gta gct aaa aca ttt tgg gaa aaa gtt 4380
Ile Arg Arg Ser Leu Val Gln Val Ala Lys Thr Phe Trp Glu Lys Val
    465                 470                 475

act gac tgt agg gtt cat gat tta ctt cgt gat ctt gcg ata caa aag 4428
Thr Asp Cys Arg Val His Asp Leu Leu Arg Asp Leu Ala Ile Gln Lys
480                 485                 490                 495

gca ttg gag gta aac ttc ttt gac gtt tat ggt cca aga agc cac tcc 4476
Ala Leu Glu Val Asn Phe Phe Asp Val Tyr Gly Pro Arg Ser His Ser
                500                 505                 510

ata tcc tct tta tgt atc aga cat ggc att cat agt gaa gga gaa agg 4524
Ile Ser Ser Leu Cys Ile Arg His Gly Ile His Ser Glu Gly Glu Arg
            515                 520                 525

tac ctc tca tca ctt gat ctt tct aac ttg aag ttg agg tca att atg 4572
Tyr Leu Ser Ser Leu Asp Leu Ser Asn Leu Lys Leu Arg Ser Ile Met
        530                 535                 540

ttc ttc gat cca gat ttt cgt aag atg agt cat ata aac ctc agg agt 4620
Phe Phe Asp Pro Asp Phe Arg Lys Met Ser His Ile Asn Leu Arg Ser
    545                 550                 555

gag ttc caa cat ctg tat gtg ttg tac ttg gat acg aat ttt ggg tat 4668
Glu Phe Gln His Leu Tyr Val Leu Tyr Leu Asp Thr Asn Phe Gly Tyr
560                 565                 570                 575

gtg tct atg gta cct gat gcc ata gga agt ttg tac cac ctc aag ttg 4716
Val Ser Met Val Pro Asp Ala Ile Gly Ser Leu Tyr His Leu Lys Leu
                580                 585                 590

tta aga ttg aga ggt atc cat gat att ccg tct tcc att ggc aac ctc 4764
Leu Arg Leu Arg Gly Ile His Asp Ile Pro Ser Ser Ile Gly Asn Leu
            595                 600                 605

aag aat tta caa aca ctt gtc gtt gta aat ggt tac aca ttt ttt tgc 4812
Lys Asn Leu Gln Thr Leu Val Val Val Asn Gly Tyr Thr Phe Phe Cys
        610                 615                 620

caa cta ccc tgc aag aca gct gac cta ata aat cta aga cat tta gtt 4860
Gln Leu Pro Cys Lys Thr Ala Asp Leu Ile Asn Leu Arg His Leu Val
    625                 630                 635

gtt caa tat tca gag cct tta aaa tgt ata aac aaa ctc act agt ctt 4908
Val Gln Tyr Ser Glu Pro Leu Lys Cys Ile Asn Lys Leu Thr Ser Leu
640                 645                 650                 655

caa gtt ctt gat ggt gtt gct tgt gat cag tgg aaa gat gtt gac cct 4956
Gln Val Leu Asp Gly Val Ala Cys Asp Gln Trp Lys Asp Val Asp Pro
                660                 665                 670

gtt gat tta gtc aat ctt cga gaa tta agc atg gat cgt atc agg agc 5004
Val Asp Leu Val Asn Leu Arg Glu Leu Ser Met Asp Arg Ile Arg Ser
            675                 680                 685

tct tac tcc cta aac aac att agc agc ttg aaa aac ctt agc act ctc 5052
Ser Tyr Ser Leu Asn Asn Ile Ser Ser Leu Lys Asn Leu Ser Thr Leu
        690                 695                 700

aaa ttg att tgt gga gaa cgt caa tca ttt gca tcc ctt gaa ttt gtt 5100
Lys Leu Ile Cys Gly Glu Arg Gln Ser Phe Ala Ser Leu Glu Phe Val
    705                 710                 715

aat tgt tgt gaa aag ctc cag aaa ttg tgg tta caa ggg aga ata gag 5148
Asn Cys Cys Glu Lys Leu Gln Lys Leu Trp Leu Gln Gly Arg Ile Glu
720                 725                 730                 735

gaa ctg cct cat ctg ttt tca aac tcc atc aca atg atg gtt ctg agt 5196
Glu Leu Pro His Leu Phe Ser Asn Ser Ile Thr Met Met Val Leu Ser
                740                 745                 750

ttc tca gaa ctg aca gaa gat ccg atg cct att ttg gga agg ttt cca 5244
Phe Ser Glu Leu Thr Glu Asp Pro Met Pro Ile Leu Gly Arg Phe Pro
            755                 760                 765

aac cta agg aat ctc aaa tta gat gga gct tat gaa gga aaa gaa ata 5292
Asn Leu Arg Asn Leu Lys Leu Asp Gly Ala Tyr Glu Gly Lys Glu Ile
        770                 775                 780

atg tgc agt gat aac agc ttc agt caa cta gag ttc ctt cat ctt cgt 5340
Met Cys Ser Asp Asn Ser Phe Ser Gln Leu Glu Phe Leu His Leu Arg
    785                 790                 795

gat ctt tgg aag cta gaa aga tgg gat tta ggc aca agt gcc atg cct 5388
Asp Leu Trp Lys Leu Glu Arg Trp Asp Leu Gly Thr Ser Ala Met Pro
800                 805                 810                 815

ctg att aaa ggt ctt ggt atc cat aac tgt cca aat tta aag gag att     5436
Leu Ile Lys Gly Leu Gly Ile His Asn Cys Pro Asn Leu Lys Glu Ile
            820                 825                 830

cct gag aga atg aaa gac atg gag ctg ttg aag cgg aat tat atg ttg     5484
Pro Glu Arg Met Lys Asp Met Glu Leu Leu Lys Arg Asn Tyr Met Leu
            835                 840                 845

tga agcttttctg ccaagcacat tggttattaa ttgagtggtt ttagtgttga     5537

tttcttatta ttgttttaag ctttttgagt gtgtaattgg tttgaacatt attgttttaa     5597

ttaattggtc tactgtatgt tctcatgctt atccacattt aagacaatgc tttatatgtt     5657

aaaatgaaat taaaaatact agtatatggt actctctctt gtccacaatt tcgtatattt     5717

tttgttcctc ttcataaaaa aaatggtaaa aaataccatt aaactatgtg ataggaacaa     5777

aaatgtcttc tattataatt taacttaaaa atgtctttac tgtcagtacc ttagttcaaa     5837

attgccctcg agtccgtagt tacaaaatgt cctttttcga ataaatatat atattttttt     5897

aaacacatct tcttcctaat taaaatatta ttaaagaaga ctattcttgt tttctttttt     5957

ctaaaaatca ctttaacaaa taaaaatgta ggaaatattt tgttttcttc ttaatctcac     6017

tttatcaatt aaaatagaat aactccatgt accctttcga catataatat atgtcatata     6077

tatatataag atcatagtat atgcatcatt aatttatatt tatataacga taaaaaataa     6137

tgataataaa ataagaaata tttttatttt ttatttcttt ctgaattgaa gtaatataaa     6197

catttgctaa ttttaaaaaa aaataataca aaaataatgt gttaaaaaga aaataataat     6257

atatttattt ggaaaatgag tatttttgat ccattaaata acagtaagtg tatttttaga     6317

ccaaagtatt gacaacaagg gtatttttgg atcaaacgac aaacggaggg tacttttgct     6377

cctttcgcat aatttaaggg tatttttaaa ccaaaatatt gacggtaaag gcatttttga     6437

gtcaaattat gaacgaaaga catttttatt tctttcacat agtttaagga catttttgac     6497

ccatttccct cctttatata aataatattt atgttaaatc aacagagaag aagctgtcaa     6557

ttgaagacat tcactttcat caacttggct tctccaagca tcaatcaact tggattattt     6617

caacattctg ttttttcaat gtttaatttc tttctatttt tggaaacatg tgttggaaga     6677

gaaccttttt tctggatttt gtgatgacct aattaacgaa acaaagttaa aaatgttctt     6737

aaattatgta aaatgaataa aaatatcctc agttaatagt ttgatccaaa aatattgttg     6797

tctctaataa ttgatctaaa aatgatatta ttgttactta ataagtgaaa accgtctttt     6857

tttcaattaa atatattatt tttctttttt taaaacacgc tcttttccta ataatgattt     6917

tttttcatta aaaaatattc atcctacttc aatttataaa aaatattact aataaataaa     6977

aatgtttttt atattattag aaagtttttt atcgttattt aaatgaaaat taatgacggg     7037

gttactatga ggacatataa tggaagaagt tgagaactcg cttagtgtga agcgagaata     7097

actaaaaaaa aaaaaaaaac ttacaaactc gcttggtgcg gagcgatttt tgggggaaaa     7157

tagagagcaa atcgctcata ggtagcgaga aaaaaaagat aaaataaaag agaaaatcgc     7217

tcgtaagtca acaagatgat caattttttt atgccgttaa cgatagttat agcacaaaca     7277

tatctcgctc cttctctagc aaagtgtccc ttttgattaa accaaaaatt gaaagaccct 7337

ttatgtattt taaagaaaaa aagtgatgtt tttaactttg aattcgaaat ttaatcatcc 7397

caatataatt cataaacgaa tttttacatc aattttaaaa taaagaataa aaaaaagaaa 7457

gataatatat actagcaggg aactacatgt gattactaca aaagataaat tcaatttcag 7517

gtggtatttg gatttgaatt gtcttacctt gctatcataa cattattttt gtttttatcc 7577

attaaaaaaa tgatgcattt atatatttat tactagtaaa gtaatatctt taatgtgtca 7637

acacataagt atccctcaat tagtaaaatg ttaggacttt tttcatgtga gaaacccaac 7697

ctcattgaaa aaggaaatta atacatttta actcaacttt taattaatta atgtcaagtt 7757

tgataaaaat aaataaaaaa acaatcgtag acaatctcta attattagaa ttttacaata 7817

tgcatattta atgggttata taaattttga gttggccttc tttttttctt cttgtgattc 7877

taagtcctcc actttatttt tatttttata tttataatta aatatttttt actcgattca 7937

cagaccgagt tggaccagtc caatcttgat taagcctcac gagttgacga gcttatttag 7997

gcttggctaa ataatttcgt tcttaaatga acttttaatt ttttttgagc tcaatcctat 8057

caaatcgcag attaggttgg atttgggtga aacaatggac caaagtccaa actaacagct 8117

ccaaaatcta cgaggtttag aaatagaaag tcttcttata tgttatgtat atctaacaaa 8177

ttatatgtta tgtatgatat tgtataaata gttatttaat gtatcaatat tgtataataa 8237

catatagtta tgtatttata atgtataact atatatgata ttgtataaat agttatttaa 8297

tgtatcgata ttgtatagta gcatatagtt atgtatttat aatgtatgta taactatgta 8357

tgatattgta taaatagatt ttggacttgg gaagtctgca gcaagcaaag gaagaggtcc 8417

aggtagcaac acttttatct taatgaatac accaaatgat gatc 8461

<210> 42
<211> 847
<212> PRT
<213> Solanum bulbocastanum

<400> 42

Met Ala Asp Ala Phe Leu Ser Phe Ala Val Gln Lys Leu Gly Asp Phe
1               5                   10                  15

Leu Ile Gln Lys Val Ser Leu Arg Lys Ser Leu Arg Asp Glu Ile Arg
            20                  25                  30

Trp Leu Ile Asn Glu Leu Leu Phe Ile Arg Ser Phe Leu Arg Asp Ala
        35                  40                  45

Glu Gln Lys Gln Cys Gly Asp Gln Arg Val Gln Gln Trp Val Phe Glu
    50                  55                  60

Ile Asn Ser Ile Ala Asn Asp Ala Val Ala Ile Leu Glu Thr Tyr Ser
65                  70                  75                  80

Phe Glu Ala Gly Lys Gly Ala Ser Arg Leu Lys Ala Cys Thr Cys Ile
                85                  90                  95

Cys Arg Lys Glu Lys Lys Phe Tyr Asn Val Ala Glu Glu Ile Gln Ser
            100                 105                 110

Leu Lys Gln Arg Ile Met Asp Ile Ser Arg Lys Arg Glu Thr Tyr Gly
        115                 120                 125

Ile Thr Asn Ile Asn Tyr Asn Ser Gly Glu Arg Pro Ser Asn Gln Val
    130                 135                 140

Thr Thr Leu Arg Arg Thr Thr Ser Tyr Val Asp Glu Gln Asp Tyr Ile
145                 150                 155                 160

Phe Val Gly Phe Gln Asp Val Val Gln Thr Leu Leu Ala Gln Leu Leu
                165                 170                 175

Lys Ala Glu Pro Arg Arg Ser Val Leu Ser Ile Tyr Gly Met Gly Gly
            180                 185                 190

Leu Gly Lys Thr Thr Leu Ala Arg Lys Leu Tyr Thr Ser Pro Asp Ile
        195                 200                 205

Leu Asn Ser Phe Pro Thr Arg Ala Trp Ile Cys Val Ser Gln Glu Tyr
    210                 215                 220

Asn Thr Met Asp Leu Leu Arg Thr Ile Ile Lys Ser Ile Gln Gly Cys
225                 230                 235                 240

Ala Lys Glu Thr Leu Asp Leu Leu Glu Lys Met Ala Glu Ile Asp Leu
                245                 250                 255

Glu Asn His Leu Arg Asp Leu Leu Lys Glu Cys Lys Tyr Leu Val Val
            260                 265                 270

Val Asp Asp Val Trp Gln Arg Glu Ala Trp Glu Ser Leu Lys Arg Ala
        275                 280                 285

Phe Pro Asp Gly Lys Asn Gly Ser Arg Val Ile Ile Thr Thr Arg Lys
    290                 295                 300

Glu Asp Val Ala Glu Arg Val Asp His Arg Gly Phe Val His Lys Leu
305                 310                 315                 320

Arg Phe Leu Ser Gln Glu Glu Ser Trp Asp Leu Phe Arg Arg Lys Leu
                325                 330                 335

Leu Asp Val Arg Ala Met Val Pro Glu Met Glu Ser Leu Ala Lys Asp
            340                 345                 350

Met Val Glu Lys Cys Arg Gly Leu Pro Leu Ala Ile Val Val Leu Ser
        355                 360                 365

Gly Leu Leu Ser His Lys Lys Gly Leu Asn Gln Trp Gln Lys Val Lys
    370                 375                 380

Asp His Leu Trp Lys Asn Ile Lys Glu Asp Lys Ser Ile Glu Ile Ser
385                 390                 395                 400

Asn Ile Leu Ser Leu Ser Tyr Asn Asp Leu Ser Thr Ala Leu Lys Gln
                405                 410                 415

Cys Phe Leu Tyr Phe Gly Ile Phe Pro Glu Asp Gln Val Val Lys Ala
            420                 425                 430

Asp Asp Ile Ile Arg Leu Trp Met Ala Glu Gly Phe Ile Pro Arg Gly
        435                 440                 445

Glu Glu Arg Met Glu Asp Val Ala Asp Gly Phe Leu Asn Glu Leu Ile
    450                 455                 460

Arg Arg Ser Leu Val Gln Val Ala Lys Thr Phe Trp Glu Lys Val Thr
465                 470                 475                 480

Asp Cys Arg Val His Asp Leu Leu Arg Asp Leu Ala Ile Gln Lys Ala
                485                 490                 495

Leu Glu Val Asn Phe Phe Asp Val Tyr Gly Pro Arg Ser His Ser Ile
            500                 505                 510

Ser Ser Leu Cys Ile Arg His Gly Ile His Ser Glu Gly Glu Arg Tyr
        515                 520                 525

Leu Ser Ser Leu Asp Leu Ser Asn Leu Lys Leu Arg Ser Ile Met Phe
    530                 535                 540

Phe Asp Pro Asp Phe Arg Lys Met Ser His Ile Asn Leu Arg Ser Glu
545                 550                 555                 560

Phe Gln His Leu Tyr Val Leu Tyr Leu Asp Thr Asn Phe Gly Tyr Val
                565                 570                 575

Ser Met Val Pro Asp Ala Ile Gly Ser Leu Tyr His Leu Lys Leu Leu
            580                 585                 590

Arg Leu Arg Gly Ile His Asp Ile Pro Ser Ser Ile Gly Asn Leu Lys
        595                 600                 605

Asn Leu Gln Thr Leu Val Val Val Asn Gly Tyr Thr Phe Phe Cys Gln
    610                 615                 620

Leu Pro Cys Lys Thr Ala Asp Leu Ile Asn Leu Arg His Leu Val Val
625                 630                 635                 640

Gln Tyr Ser Glu Pro Leu Lys Cys Ile Asn Lys Leu Thr Ser Leu Gln
                645                 650                 655

Val Leu Asp Gly Val Ala Cys Asp Gln Trp Lys Asp Val Asp Pro Val
            660                 665                 670

Asp Leu Val Asn Leu Arg Glu Leu Ser Met Asp Arg Ile Arg Ser Ser
        675                 680                 685

Tyr Ser Leu Asn Asn Ile Ser Ser Leu Lys Asn Leu Ser Thr Leu Lys
    690                 695                 700

Leu Ile Cys Gly Glu Arg Gln Ser Phe Ala Ser Leu Glu Phe Val Asn
705                 710                 715                 720

Cys Cys Glu Lys Leu Gln Lys Leu Trp Leu Gln Gly Arg Ile Glu Glu
                725                 730                 735

Leu Pro His Leu Phe Ser Asn Ser Ile Thr Met Met Val Leu Ser Phe
            740                 745                 750

Ser Glu Leu Thr Glu Asp Pro Met Pro Ile Leu Gly Arg Phe Pro Asn
        755                 760                 765

Leu Arg Asn Leu Lys Leu Asp Gly Ala Tyr Glu Gly Lys Glu Ile Met
    770                 775                 780

Cys Ser Asp Asn Ser Phe Ser Gln Leu Glu Phe Leu His Leu Arg Asp
785                 790                 795                 800

Leu Trp Lys Leu Glu Arg Trp Asp Leu Gly Thr Ser Ala Met Pro Leu
                805                 810                 815

Ile Lys Gly Leu Gly Ile His Asn Cys Pro Asn Leu Lys Glu Ile Pro
            820                 825                 830

Glu Arg Met Lys Asp Met Glu Leu Leu Lys Arg Asn Tyr Met Leu
        835                 840                 845

<210>  43
<211>  852
<212>  DNA
<213>  Solanum tuberosum


<220>
<221>  CDS
<222>  (1)..(103)

<220>

<221>  Intron
<222>  (104)..(607)

<220>
<221>  CDS
<222>  (608)..(738)

<220>
<221>  3'UTR
<222>  (742)..(852)

<400>  43
atg ggt tcc aag gca att atg ttt ctt ggt ctt ttt ttg gct att ttc      48
Met Gly Ser Lys Ala Ile Met Phe Leu Gly Leu Phe Leu Ala Ile Phe
1               5                   10                  15

tta atg ata agc tct gag gtt gct gct agg gag ttg gca gct gag act      96
Leu Met Ile Ser Ser Glu Val Ala Ala Arg Glu Leu Ala Ala Glu Thr
            20                  25                  30

tcc aat g gtaagcattt cgtttcattt gttccctctc ttagggtgtg ttcgataaaa     153
Ser Asn

aaaatgtttt tcatgaaaaa aaatttgtaa gaaaataagt gactttttta tttacatttt     213

tgtaaaataa ttggaaacga aatcccaaaa tttcaattaa taatgatata gtttgatcat     273

aacacttaat atacattcct tttgtttcat tttatatgtc tcatttacta tgcataaagt     333

ttaagaatat accataaact ttccaatctt tgctaaacta aactaaaggt gtgtaaatta     393

tattataaat gtcctaagaa ttttgtgatc ttatgttaca ttgaaattaa aaaatttaca     453

attaaatata tattttagga aaaggagtac tcattttgaa atataataaa aaggaaagta     513

cgacatataa attgaagctc aattcagaga ctagtcatga ctcatatcgt acgtgttagc     573

tagtttaatt aatcttcaca attcatcgat gcag cg  gta aac gtt gat gga cat     627
                                      Ala Val Asn Val Asp Gly His
                                                          40

tat cat ggt ggc ggc tat ggt aag cac tat gga aaa cct aag aaa tgc      675
Tyr His Gly Gly Gly Tyr Gly Lys His Tyr Gly Lys Pro Lys Lys Cys
        45                  50                  55

tat aga tgc cac aaa aaa tac tgc tgc tct tat gaa gaa tat gtg gct      723
Tyr Arg Cys His Lys Lys Tyr Cys Cys Ser Tyr Glu Glu Tyr Val Ala
    60                  65                  70

gac cag act cac aac taaataatta attgtgtgta ttattattgt aaacttttga      778
Asp Gln Thr His Asn
    75

aattaagtga caagataata ataatcttgc tacttaagac cctttgcttg taacaagtat     838

gaataaagcc attc                                                       852

<210>  44
<211>  78
<212>  PRT
<213>  Solanum tuberosum

<400>  44

Met Gly Ser Lys Ala Ile Met Phe Leu Gly Leu Phe Leu Ala Ile Phe
1               5                   10                  15

Leu Met Ile Ser Ser Glu Val Ala Ala Arg Glu Leu Ala Ala Glu Thr
            20                  25                  30

Ser Asn Ala Val Asn Val Asp Gly His Tyr His Gly Gly Gly Tyr Gly
        35                  40                  45

Lys His Tyr Gly Lys Pro Lys Lys Cys Tyr Arg Cys His Lys Lys Tyr
    50                  55                  60

Cys Cys Ser Tyr Glu Glu Tyr Val Ala Asp Gln Thr His Asn
65                  70                  75

<210>  45
<211>  2913
<212>  DNA
<213>  Solanum stoloniferum

<220>
<221>  CDS
<222>  (1)..(2913)

<400>  45
atg gct gaa gct ttc att caa gtt ctg tta gac aat ctc act tct ttc      48
Met Ala Glu Ala Phe Ile Gln Val Leu Leu Asp Asn Leu Thr Ser Phe
1               5                   10                  15

ctc aaa ggg gaa ctt aca ttg ctt ttc ggt ttt caa gat gag ttc caa      96
Leu Lys Gly Glu Leu Thr Leu Leu Phe Gly Phe Gln Asp Glu Phe Gln
            20                  25                  30

agg ctt tca agc atg ttt tct aca atc caa gcc gtc ctt gaa gat gct      144
Arg Leu Ser Ser Met Phe Ser Thr Ile Gln Ala Val Leu Glu Asp Ala
        35                  40                  45

cag gag aag caa ctc aac aac aag cct cta gaa aat tgg ttg caa aaa      192
Gln Glu Lys Gln Leu Asn Asn Lys Pro Leu Glu Asn Trp Leu Gln Lys
    50                  55                  60

ctc aat gct gct aca tac gaa gtc gat gac atc ttg gat gaa tat aaa      240
Leu Asn Ala Ala Thr Tyr Glu Val Asp Asp Ile Leu Asp Glu Tyr Lys
65                  70                  75                  80

acc aag gcc aca aga ttc tcc cag tct gaa tat ggc cgt tat cat cca      288
Thr Lys Ala Thr Arg Phe Ser Gln Ser Glu Tyr Gly Arg Tyr His Pro
                85                  90                  95

aag gtt atc cct ttc cgt cac aag gtc ggg aaa agg atg gac caa gtg      336
Lys Val Ile Pro Phe Arg His Lys Val Gly Lys Arg Met Asp Gln Val
            100                 105                 110

atg aaa aaa cta aag gca att gct gag gaa aga aag aat ttt cat ttg      384
Met Lys Lys Leu Lys Ala Ile Ala Glu Glu Arg Lys Asn Phe His Leu
        115                 120                 125

cac gaa aaa att gta gag aga caa gct gtt aga cgg gaa aca ggt tct      432
His Glu Lys Ile Val Glu Arg Gln Ala Val Arg Arg Glu Thr Gly Ser
    130                 135                 140

gta tta acc gaa ccg cag gtt tat gga aga gac aaa gag aaa gat gag      480
Val Leu Thr Glu Pro Gln Val Tyr Gly Arg Asp Lys Glu Lys Asp Glu
145                 150                 155                 160

ata gtg aaa atc cta ata aac aat gtt agt gat gcc caa cac ctt tca 528
Ile Val Lys Ile Leu Ile Asn Asn Val Ser Asp Ala Gln His Leu Ser
                165                 170                 175

gtc ctc cca ata ctt ggt atg ggg gga tta gga aaa acg act ctt gcc 576
Val Leu Pro Ile Leu Gly Met Gly Gly Leu Gly Lys Thr Thr Leu Ala
            180                 185                 190

caa atg gtc ttc aat gac cag aga gtt act gag cat ttc cat tcc aaa 624
Gln Met Val Phe Asn Asp Gln Arg Val Thr Glu His Phe His Ser Lys
        195                 200                 205

ata tgg att tgt gtc tcg gaa gat ttt gat gag aag agg tta ata aag 672
Ile Trp Ile Cys Val Ser Glu Asp Phe Asp Glu Lys Arg Leu Ile Lys
    210                 215                 220

gca att gta gaa tct att gaa gga agg cca cta ctt ggt gag atg gac 720
Ala Ile Val Glu Ser Ile Glu Gly Arg Pro Leu Leu Gly Glu Met Asp
225                 230                 235                 240

ttg gct cca ctt caa aag aag ctt cag gag ttg ctg aat gga aaa aga 768
Leu Ala Pro Leu Gln Lys Lys Leu Gln Glu Leu Leu Asn Gly Lys Arg
                245                 250                 255

tac ttg ctt gtc tta gat gat gtt tgg aat gaa gat caa cag aag tgg 816
Tyr Leu Leu Val Leu Asp Asp Val Trp Asn Glu Asp Gln Gln Lys Trp
            260                 265                 270

gca aat tta aga gca gtc ttg aag gtt gga gca agt ggt gct tct gtt 864
Ala Asn Leu Arg Ala Val Leu Lys Val Gly Ala Ser Gly Ala Ser Val
        275                 280                 285

cta acc act act cgt ctt gaa aag gtt gga tca att atg gga aca ttg 912
Leu Thr Thr Thr Arg Leu Glu Lys Val Gly Ser Ile Met Gly Thr Leu
    290                 295                 300

caa cca tat gaa ctg tca aat ctg tct caa gaa gat tgt tgg ttg ttg 960
Gln Pro Tyr Glu Leu Ser Asn Leu Ser Gln Glu Asp Cys Trp Leu Leu
305                 310                 315                 320

ttc atg caa cgt gca ttt gga cac caa gaa gaa ata aat cca aac ctt 1008
Phe Met Gln Arg Ala Phe Gly His Gln Glu Glu Ile Asn Pro Asn Leu
                325                 330                 335

gtg gca atc gga aag gag att gtg aaa aaa agt ggt ggt gtg cct cta 1056
Val Ala Ile Gly Lys Glu Ile Val Lys Lys Ser Gly Gly Val Pro Leu
            340                 345                 350

gca gcc aaa act ctt gga ggt att ttg tgc ttc aag aga gaa gaa aga 1104
Ala Ala Lys Thr Leu Gly Gly Ile Leu Cys Phe Lys Arg Glu Glu Arg
        355                 360                 365

gca tgg gaa cat gtg aga gac agt ccg att tgg aat ttg cct caa gat 1152
Ala Trp Glu His Val Arg Asp Ser Pro Ile Trp Asn Leu Pro Gln Asp
    370                 375                 380

gaa agt tct att ctg cct gcc ctg agg ctt agt tac cat caa ctt cca 1200
Glu Ser Ser Ile Leu Pro Ala Leu Arg Leu Ser Tyr His Gln Leu Pro
385                 390                 395                 400

ctt gat ttg aaa caa tgc ttt gcg tat tgt gcg gtg ttc cca aag gat 1248
Leu Asp Leu Lys Gln Cys Phe Ala Tyr Cys Ala Val Phe Pro Lys Asp
                405                 410                 415

gcc aaa atg gaa aaa gaa aag cta atc tct ctc tgg atg gcg cat ggt 1296
Ala Lys Met Glu Lys Glu Lys Leu Ile Ser Leu Trp Met Ala His Gly
            420                 425                 430

ttt ctt tta tca aaa gga aac atg gag cta gag gat gtg ggt gat gaa 1344
Phe Leu Leu Ser Lys Gly Asn Met Glu Leu Glu Asp Val Gly Asp Glu
        435                 440                 445

gta tgg aaa gaa tta tac ttg agg tct ttt ttc caa gag att gaa gtt 1392
Val Trp Lys Glu Leu Tyr Leu Arg Ser Phe Phe Gln Glu Ile Glu Val
    450                 455                 460

aaa gat ggt aaa act tat ttc aag atg cat gat ctc atc cat gat ttg 1440
Lys Asp Gly Lys Thr Tyr Phe Lys Met His Asp Leu Ile His Asp Leu
465                 470                 475                 480

gca aca tct ctg ttt tca gca aac aca tca agc agc aat atc cgt gaa 1488
Ala Thr Ser Leu Phe Ser Ala Asn Thr Ser Ser Ser Asn Ile Arg Glu
                485                 490                 495

ata aat aaa cac agt tac aca cat atg atg tcc att ggt ttc gcc gaa 1536
Ile Asn Lys His Ser Tyr Thr His Met Met Ser Ile Gly Phe Ala Glu
            500                 505                 510

gtg gtg ttt ttt tac act ctt ccc ccc ttg gaa aag ttt atc tcg tta 1584
Val Val Phe Phe Tyr Thr Leu Pro Pro Leu Glu Lys Phe Ile Ser Leu
        515                 520                 525

aga gtg ctt aat cta ggt gat tcg aca ttt aat aag tta cca tct tcc 1632
Arg Val Leu Asn Leu Gly Asp Ser Thr Phe Asn Lys Leu Pro Ser Ser
    530                 535                 540

att gga gat cta gta cat tta aga tac ttg aac ctg tat ggc agt ggc 1680
Ile Gly Asp Leu Val His Leu Arg Tyr Leu Asn Leu Tyr Gly Ser Gly
545                 550                 555                 560

atg cgt agt ctt cca aag cag tta tgc aag ctt caa aat ctg caa act 1728
Met Arg Ser Leu Pro Lys Gln Leu Cys Lys Leu Gln Asn Leu Gln Thr
                565                 570                 575

ctt gat cta caa tat tgc acc aag ctt tgt tgt ttg cca aaa gaa aca 1776
Leu Asp Leu Gln Tyr Cys Thr Lys Leu Cys Cys Leu Pro Lys Glu Thr
            580                 585                 590

agt aaa ctt ggt agt ctc cga aat ctt tta ctt gat ggt agc cag tca 1824
Ser Lys Leu Gly Ser Leu Arg Asn Leu Leu Leu Asp Gly Ser Gln Ser
        595                 600                 605

ttg act tgt atg cca cca agg ata gga tca ttg aca tgc ctt aag act 1872
Leu Thr Cys Met Pro Pro Arg Ile Gly Ser Leu Thr Cys Leu Lys Thr
    610                 615                 620

cta ggt caa ttt gtt gtt gga agg aag aaa ggt tat caa ctt ggt gaa 1920
Leu Gly Gln Phe Val Val Gly Arg Lys Lys Gly Tyr Gln Leu Gly Glu
625                 630                 635                 640

cta gga aac cta aat ctc tat ggc tca att aaa atc tcg cat ctt gag 1968
Leu Gly Asn Leu Asn Leu Tyr Gly Ser Ile Lys Ile Ser His Leu Glu
                645                 650                 655

aga gtg aag aat gat aag gac gca aaa gaa gcc aat tta tct gca aaa 2016
Arg Val Lys Asn Asp Lys Asp Ala Lys Glu Ala Asn Leu Ser Ala Lys
            660                 665                 670

ggg aat ctg cat tct tta agc atg agt tgg aat aac ttt gga cca cat 2064
Gly Asn Leu His Ser Leu Ser Met Ser Trp Asn Asn Phe Gly Pro His
        675                 680                 685

ata tat gaa tca gaa gaa gtt aaa gtg ctt gaa gcc ctc aaa cca cac 2112
Ile Tyr Glu Ser Glu Glu Val Lys Val Leu Glu Ala Leu Lys Pro His
    690                 695                 700

tcc aat ctg act tct tta aaa atc tat ggc ttc aga gga atc cat ctc 2160
Ser Asn Leu Thr Ser Leu Lys Ile Tyr Gly Phe Arg Gly Ile His Leu
705                 710                 715                 720

cca gag tgg atg aat cac tca gta ttg aaa aat att gtc tct att cta 2208
Pro Glu Trp Met Asn His Ser Val Leu Lys Asn Ile Val Ser Ile Leu
                725                 730                 735

att agc aac ttc aga aac tgc tca tgc tta cca ccc ttt ggt gat ctg 2256
Ile Ser Asn Phe Arg Asn Cys Ser Cys Leu Pro Pro Phe Gly Asp Leu
            740                 745                 750

cct tgt cta gaa agt cta gag tta cac tgg ggg tct gcg gat gtg gag 2304
Pro Cys Leu Glu Ser Leu Glu Leu His Trp Gly Ser Ala Asp Val Glu
        755                 760                 765

tat gtt gaa gaa gtg gat att gat gtt cat tct gga ttc ccc aca aga 2352
Tyr Val Glu Glu Val Asp Ile Asp Val His Ser Gly Phe Pro Thr Arg
    770                 775                 780

ata agg ttt cca tcc ttg agg aaa ctt gat ata tgg gac ttt ggt agt 2400
Ile Arg Phe Pro Ser Leu Arg Lys Leu Asp Ile Trp Asp Phe Gly Ser
785                 790                 795                 800

ctg aaa gga ttg ctg aaa aag gaa gga gaa gag caa ttc cct gtg ctt 2448
Leu Lys Gly Leu Leu Lys Lys Glu Gly Glu Glu Gln Phe Pro Val Leu
                805                 810                 815

gaa gag atg ata att cac gag tgc cct ttt ctg acc ctt tct tct aat 2496
Glu Glu Met Ile Ile His Glu Cys Pro Phe Leu Thr Leu Ser Ser Asn
            820                 825                 830

ctt agg gct ctt act tcc ctc aga att tgc tat aat aaa gta gct act 2544
Leu Arg Ala Leu Thr Ser Leu Arg Ile Cys Tyr Asn Lys Val Ala Thr
        835                 840                 845

tca ttc cca gaa gag atg ttc aaa aac ctt gca aat ctc aaa tac ttg 2592
Ser Phe Pro Glu Glu Met Phe Lys Asn Leu Ala Asn Leu Lys Tyr Leu
    850                 855                 860

aca atc tct cgg tgc aat aat ctc aaa gag ctg cct acc agc ttg gct 2640
Thr Ile Ser Arg Cys Asn Asn Leu Lys Glu Leu Pro Thr Ser Leu Ala
865                 870                 875                 880

agt ctg aat gct ttg aaa agt cta aaa att caa ttg tgt tgc gca cta 2688
Ser Leu Asn Ala Leu Lys Ser Leu Lys Ile Gln Leu Cys Cys Ala Leu
                885                 890                 895

gag agt ctc cct gag gaa ggg ctg gaa ggt tta tct tca ctc aca gag 2736
Glu Ser Leu Pro Glu Glu Gly Leu Glu Gly Leu Ser Ser Leu Thr Glu
            900                 905                 910

tta ttt gtt gaa cac tgt aac atg cta aaa tgt tta cca gag gga ttg 2784
Leu Phe Val Glu His Cys Asn Met Leu Lys Cys Leu Pro Glu Gly Leu
        915                 920                 925

cag cac cta aca acc ctc aca agt tta aaa att cgg gga tgt cca caa 2832
Gln His Leu Thr Thr Leu Thr Ser Leu Lys Ile Arg Gly Cys Pro Gln
    930                 935                 940

ctg atc aag cgg tgt gag aag gga ata gga gaa gac tgg cac aaa att 2880
Leu Ile Lys Arg Cys Glu Lys Gly Ile Gly Glu Asp Trp His Lys Ile
945                 950                 955                 960

tct cac att cct aat gtg aat ata tat aat taa 2913
Ser His Ile Pro Asn Val Asn Ile Tyr Asn
                965                 970

<210>  46
<211>  970
<212>  PRT
<213>  Solanum stoloniferum

<400>  46

Met Ala Glu Ala Phe Ile Gln Val Leu Leu Asp Asn Leu Thr Ser Phe
1               5                   10                  15

Leu Lys Gly Glu Leu Thr Leu Leu Phe Gly Phe Gln Asp Glu Phe Gln
            20                  25                  30

Arg Leu Ser Ser Met Phe Ser Thr Ile Gln Ala Val Leu Glu Asp Ala
        35                  40                  45

Gln Glu Lys Gln Leu Asn Asn Lys Pro Leu Glu Asn Trp Leu Gln Lys
    50                  55                  60

Leu Asn Ala Ala Thr Tyr Glu Val Asp Asp Ile Leu Asp Glu Tyr Lys
65                  70                  75                  80

Thr Lys Ala Thr Arg Phe Ser Gln Ser Glu Tyr Gly Arg Tyr His Pro
                85                  90                  95

Lys Val Ile Pro Phe Arg His Lys Val Gly Lys Arg Met Asp Gln Val
            100                 105                 110

Met Lys Lys Leu Lys Ala Ile Ala Glu Glu Arg Lys Asn Phe His Leu
        115                 120                 125

His Glu Lys Ile Val Glu Arg Gln Ala Val Arg Arg Glu Thr Gly Ser
    130                 135                 140

Val Leu Thr Glu Pro Gln Val Tyr Gly Arg Asp Lys Glu Lys Asp Glu
145                 150                 155                 160

Ile Val Lys Ile Leu Ile Asn Asn Val Ser Asp Ala Gln His Leu Ser
                165                 170                 175

Val Leu Pro Ile Leu Gly Met Gly Gly Leu Gly Lys Thr Thr Leu Ala
            180                 185                 190

Gln Met Val Phe Asn Asp Gln Arg Val Thr Glu His Phe His Ser Lys
        195                 200                 205

Ile Trp Ile Cys Val Ser Glu Asp Phe Asp Glu Lys Arg Leu Ile Lys
    210                 215                 220

Ala Ile Val Glu Ser Ile Glu Gly Arg Pro Leu Leu Gly Glu Met Asp
225                 230                 235                 240

Leu Ala Pro Leu Gln Lys Lys Leu Gln Glu Leu Leu Asn Gly Lys Arg
                245                 250                 255

Tyr Leu Leu Val Leu Asp Asp Val Trp Asn Glu Asp Gln Gln Lys Trp
            260                 265                 270

Ala Asn Leu Arg Ala Val Leu Lys Val Gly Ala Ser Gly Ala Ser Val
        275                 280                 285

Leu Thr Thr Thr Arg Leu Glu Lys Val Gly Ser Ile Met Gly Thr Leu
    290                 295                 300

Gln Pro Tyr Glu Leu Ser Asn Leu Ser Gln Glu Asp Cys Trp Leu Leu
305                 310                 315                 320

Phe Met Gln Arg Ala Phe Gly His Gln Glu Glu Ile Asn Pro Asn Leu
                325                 330                 335

Val Ala Ile Gly Lys Glu Ile Val Lys Lys Ser Gly Gly Val Pro Leu
            340                 345                 350

Ala Ala Lys Thr Leu Gly Gly Ile Leu Cys Phe Lys Arg Glu Glu Arg
        355                 360                 365

Ala Trp Glu His Val Arg Asp Ser Pro Ile Trp Asn Leu Pro Gln Asp
    370                 375                 380

Glu Ser Ser Ile Leu Pro Ala Leu Arg Leu Ser Tyr His Gln Leu Pro
385                 390                 395                 400

Leu Asp Leu Lys Gln Cys Phe Ala Tyr Cys Ala Val Phe Pro Lys Asp
                405                 410                 415

Ala Lys Met Glu Lys Glu Lys Leu Ile Ser Leu Trp Met Ala His Gly
            420                 425                 430

Phe Leu Leu Ser Lys Gly Asn Met Glu Leu Glu Asp Val Gly Asp Glu
        435                 440                 445

Val Trp Lys Glu Leu Tyr Leu Arg Ser Phe Phe Gln Glu Ile Glu Val
    450                 455                 460

Lys Asp Gly Lys Thr Tyr Phe Lys Met His Asp Leu Ile His Asp Leu
465                 470                 475                 480

Ala Thr Ser Leu Phe Ser Ala Asn Thr Ser Ser Ser Asn Ile Arg Glu
                485                 490                 495

Ile Asn Lys His Ser Tyr Thr His Met Met Ser Ile Gly Phe Ala Glu
            500                 505                 510

Val Val Phe Phe Tyr Thr Leu Pro Pro Leu Glu Lys Phe Ile Ser Leu
        515                 520                 525

Arg Val Leu Asn Leu Gly Asp Ser Thr Phe Asn Lys Leu Pro Ser Ser
    530                 535                 540

Ile Gly Asp Leu Val His Leu Arg Tyr Leu Asn Leu Tyr Gly Ser Gly
545                 550                 555                 560

Met Arg Ser Leu Pro Lys Gln Leu Cys Lys Leu Gln Asn Leu Gln Thr
                565                 570                 575

Leu Asp Leu Gln Tyr Cys Thr Lys Leu Cys Cys Leu Pro Lys Glu Thr
            580                 585                 590

Ser Lys Leu Gly Ser Leu Arg Asn Leu Leu Leu Asp Gly Ser Gln Ser
        595                 600                 605

Leu Thr Cys Met Pro Pro Arg Ile Gly Ser Leu Thr Cys Leu Lys Thr
    610                 615                 620

Leu Gly Gln Phe Val Val Gly Arg Lys Lys Gly Tyr Gln Leu Gly Glu
625                 630                 635                 640

Leu Gly Asn Leu Asn Leu Tyr Gly Ser Ile Lys Ile Ser His Leu Glu
                645                 650                 655

Arg Val Lys Asn Asp Lys Asp Ala Lys Glu Ala Asn Leu Ser Ala Lys
            660                 665                 670

Gly Asn Leu His Ser Leu Ser Met Ser Trp Asn Asn Phe Gly Pro His
        675                 680                 685

Ile Tyr Glu Ser Glu Glu Val Lys Val Leu Glu Ala Leu Lys Pro His
    690                 695                 700

Ser Asn Leu Thr Ser Leu Lys Ile Tyr Gly Phe Arg Gly Ile His Leu
705                 710                 715                 720

Pro Glu Trp Met Asn His Ser Val Leu Lys Asn Ile Val Ser Ile Leu
                725                 730                 735

Ile Ser Asn Phe Arg Asn Cys Ser Cys Leu Pro Pro Phe Gly Asp Leu
            740                 745                 750

Pro Cys Leu Glu Ser Leu Glu Leu His Trp Gly Ser Ala Asp Val Glu
        755                 760                 765

Tyr Val Glu Glu Val Asp Ile Asp Val His Ser Gly Phe Pro Thr Arg
    770                 775                 780

Ile Arg Phe Pro Ser Leu Arg Lys Leu Asp Ile Trp Asp Phe Gly Ser
785                 790                 795                 800

Leu Lys Gly Leu Leu Lys Lys Glu Gly Glu Glu Gln Phe Pro Val Leu
                805                 810                 815

Glu Glu Met Ile Ile His Glu Cys Pro Phe Leu Thr Leu Ser Ser Asn
            820                 825                 830

Leu Arg Ala Leu Thr Ser Leu Arg Ile Cys Tyr Asn Lys Val Ala Thr
        835                 840                 845

Ser Phe Pro Glu Glu Met Phe Lys Asn Leu Ala Asn Leu Lys Tyr Leu
    850                 855                 860

Thr Ile Ser Arg Cys Asn Asn Leu Lys Glu Leu Pro Thr Ser Leu Ala
865                 870                 875                 880

Ser Leu Asn Ala Leu Lys Ser Leu Lys Ile Gln Leu Cys Cys Ala Leu
                885                 890                 895

Glu Ser Leu Pro Glu Glu Gly Leu Glu Gly Leu Ser Ser Leu Thr Glu
            900                 905                 910

Leu Phe Val Glu His Cys Asn Met Leu Lys Cys Leu Pro Glu Gly Leu
        915                 920                 925

Gln His Leu Thr Thr Leu Thr Ser Leu Lys Ile Arg Gly Cys Pro Gln
    930                 935                 940

Leu Ile Lys Arg Cys Glu Lys Gly Ile Gly Glu Asp Trp His Lys Ile
945                 950                 955                 960

Ser His Ile Pro Asn Val Asn Ile Tyr Asn
                965                 970

<210>  47
<211>  2913
<212>  DNA
<213>  Solanum patita

<220>
<221>  CDS
<222>  (1)..(2913)

<400>  47
atg gct gaa gct ttc att caa gtt ctg tta gac aat ctc act tct ttc      48
Met Ala Glu Ala Phe Ile Gln Val Leu Leu Asp Asn Leu Thr Ser Phe
1               5                   10                  15

ctc aaa ggg gaa ctt aca ttg ctt ttc ggt ttt caa gat gag ttc caa      96
Leu Lys Gly Glu Leu Thr Leu Leu Phe Gly Phe Gln Asp Glu Phe Gln
            20                  25                  30

agg ctt tca agc atg ttt tct aca atc caa gcc gtc ctt gaa gat gct 144
Arg Leu Ser Ser Met Phe Ser Thr Ile Gln Ala Val Leu Glu Asp Ala
        35                  40                  45

cag gag aag caa ctc aac aac aag cct cta gaa aat tgg ttg caa aaa 192
Gln Glu Lys Gln Leu Asn Asn Lys Pro Leu Glu Asn Trp Leu Gln Lys
    50                  55                  60

ctc aat gct gct aca tac gaa gtc gat gac atc ttg gat gaa tat aaa 240
Leu Asn Ala Ala Thr Tyr Glu Val Asp Asp Ile Leu Asp Glu Tyr Lys
65                  70                  75                  80

acc aag gcc aca aga ttc tcc cag tct gaa tat ggc cgt tat cat cca 288
Thr Lys Ala Thr Arg Phe Ser Gln Ser Glu Tyr Gly Arg Tyr His Pro
                85                  90                  95

aag gtt atc cct ttc cgt cac aag gtc ggg aaa agg atg gac caa gtg 336
Lys Val Ile Pro Phe Arg His Lys Val Gly Lys Arg Met Asp Gln Val
            100                 105                 110

atg aaa aaa cta aag gca att gct gag gaa aga aag aat ttt cat ttg 384
Met Lys Lys Leu Lys Ala Ile Ala Glu Glu Arg Lys Asn Phe His Leu
        115                 120                 125

cac gaa aaa att gta gag aga caa gct gtt aga cgg gaa aca ggt tct 432
His Glu Lys Ile Val Glu Arg Gln Ala Val Arg Arg Glu Thr Gly Ser
    130                 135                 140

gta tta acc gaa ccg cag gtt tat gga aga gac aaa gag aaa gat gag 480
Val Leu Thr Glu Pro Gln Val Tyr Gly Arg Asp Lys Glu Lys Asp Glu
145                 150                 155                 160

ata gtg aaa atc cta ata aac aat gtt agt gat gcc caa cac ctt tca 528
Ile Val Lys Ile Leu Ile Asn Asn Val Ser Asp Ala Gln His Leu Ser
                165                 170                 175

gtc ctc cca ata ctt ggt atg ggg gga tta gga aaa acg act ctt gcc 576
Val Leu Pro Ile Leu Gly Met Gly Gly Leu Gly Lys Thr Thr Leu Ala
            180                 185                 190

caa atg gtc ttc aat gac cag aga gtt act gag cat ttc cat tcc aaa 624
Gln Met Val Phe Asn Asp Gln Arg Val Thr Glu His Phe His Ser Lys
        195                 200                 205

ata tgg att tgt gtc tcg gaa gat ttt gat gag aag agg tta ata aag 672
Ile Trp Ile Cys Val Ser Glu Asp Phe Asp Glu Lys Arg Leu Ile Lys
    210                 215                 220

gca att gta gaa tct att gaa gga agg cca cta ctt ggt gag atg gac 720
Ala Ile Val Glu Ser Ile Glu Gly Arg Pro Leu Leu Gly Glu Met Asp
225                 230                 235                 240

ttg gct cca ctt caa aag aag ctt cag gag ttg ctg aat gga aaa aga 768
Leu Ala Pro Leu Gln Lys Lys Leu Gln Glu Leu Leu Asn Gly Lys Arg
                245                 250                 255

tac ttg ctt gtc tta gat gat gtt tgg aat gaa gat caa cag aag tgg 816
Tyr Leu Leu Val Leu Asp Asp Val Trp Asn Glu Asp Gln Gln Lys Trp
            260                 265                 270

gct aat tta aga gca gtc ttg aag gtt gga gca agt ggt gct tct gtt 864
Ala Asn Leu Arg Ala Val Leu Lys Val Gly Ala Ser Gly Ala Ser Val
        275                 280                 285

cta acc act act cgt ctt gaa aag gtt gga tca att atg gga aca ttg 912
Leu Thr Thr Thr Arg Leu Glu Lys Val Gly Ser Ile Met Gly Thr Leu
    290                 295                 300

caa cca tat gaa ctg tca aat ctg tct caa gaa gat tgt tgg ttg ttg 960
Gln Pro Tyr Glu Leu Ser Asn Leu Ser Gln Glu Asp Cys Trp Leu Leu
305                 310                 315                 320

ttc atg caa cgt gca ttt gga cac caa gaa gaa ata aat cca aac ctt 1008
Phe Met Gln Arg Ala Phe Gly His Gln Glu Glu Ile Asn Pro Asn Leu
                325                 330                 335

gtg gca atc gga aag gag att gtg aaa aaa agt ggt ggt gtg cct cta 1056
Val Ala Ile Gly Lys Glu Ile Val Lys Lys Ser Gly Gly Val Pro Leu
            340                 345                 350

gca gcc aaa act ctt gga ggt att ttg tgc ttc aag aga gaa gaa aga 1104
Ala Ala Lys Thr Leu Gly Gly Ile Leu Cys Phe Lys Arg Glu Glu Arg
        355                 360                 365

gca tgg gaa cat gtg aga gac agt ccg att tgg aat ttg cct caa gat 1152
Ala Trp Glu His Val Arg Asp Ser Pro Ile Trp Asn Leu Pro Gln Asp
    370                 375                 380

gaa agt tct att ctg cct gcc ctg agg ctt agt tac cat caa ctt cca 1200
Glu Ser Ser Ile Leu Pro Ala Leu Arg Leu Ser Tyr His Gln Leu Pro
385                 390                 395                 400

ctt gat ttg aaa caa tgc ttt gcg tat tgt gcg gtg ttc cca aag gat 1248
Leu Asp Leu Lys Gln Cys Phe Ala Tyr Cys Ala Val Phe Pro Lys Asp
                405                 410                 415

gcc aaa atg gaa aaa gaa aag cta atc tct ctc tgg atg gcg cat ggt 1296
Ala Lys Met Glu Lys Glu Lys Leu Ile Ser Leu Trp Met Ala His Gly
            420                 425                 430

ttt ctt tta tca aaa gga aac atg gag cta gag gat gtg ggc gat gaa 1344
Phe Leu Leu Ser Lys Gly Asn Met Glu Leu Glu Asp Val Gly Asp Glu
        435                 440                 445

gta tgg aaa gaa tta tac ttg agg tct ttt ttc caa gag att gaa gtt 1392
Val Trp Lys Glu Leu Tyr Leu Arg Ser Phe Phe Gln Glu Ile Glu Val
    450                 455                 460

aaa gat ggt aaa act tat ttc aag atg cat gat ctc atc cat gat ttg 1440
Lys Asp Gly Lys Thr Tyr Phe Lys Met His Asp Leu Ile His Asp Leu
465                 470                 475                 480

gca aca tct ctg ttt tca gca aac aca tca agc agc aat atc cgt gaa 1488
Ala Thr Ser Leu Phe Ser Ala Asn Thr Ser Ser Ser Asn Ile Arg Glu
                485                 490                 495

ata aat aaa cac agt tac aca cat atg atg tcc att ggt ttc gcc gaa 1536
Ile Asn Lys His Ser Tyr Thr His Met Met Ser Ile Gly Phe Ala Glu
            500                 505                 510

gtg gtg ttt ttt tac act ctt ccc ccc ttg gaa aag ttt atc tcg tta 1584
Val Val Phe Phe Tyr Thr Leu Pro Pro Leu Glu Lys Phe Ile Ser Leu
        515                 520                 525

aga gtg ctt aat cta ggt gat tcg aca ttt aat aag tta cca tct tcc 1632
Arg Val Leu Asn Leu Gly Asp Ser Thr Phe Asn Lys Leu Pro Ser Ser
    530                 535                 540

att gga gat cta gta cat tta aga tac ttg aac ctg tat ggc agt ggc 1680
Ile Gly Asp Leu Val His Leu Arg Tyr Leu Asn Leu Tyr Gly Ser Gly
545                 550                 555                 560

atg cgt agt ctt cca aag cag tta tgc aag ctt caa aat ctg caa act 1728
Met Arg Ser Leu Pro Lys Gln Leu Cys Lys Leu Gln Asn Leu Gln Thr
                565                 570                 575

ctt gat cta caa tat tgc acc aag ctt tgt tgt ttg cca aaa gaa aca 1776
Leu Asp Leu Gln Tyr Cys Thr Lys Leu Cys Cys Leu Pro Lys Glu Thr
            580                 585                 590

agt aaa ctt ggt agt ctc cga aat ctt tta ctt gat ggt agc cag tca 1824
Ser Lys Leu Gly Ser Leu Arg Asn Leu Leu Leu Asp Gly Ser Gln Ser
        595                 600                 605

ttg act tgt atg cca cca agg ata gga tca ttg aca tgc ctt aag act 1872
Leu Thr Cys Met Pro Pro Arg Ile Gly Ser Leu Thr Cys Leu Lys Thr
    610                 615                 620

cta ggt caa ttt gtt gtt gga agg aag aaa ggt tat caa ctt ggt gaa 1920
Leu Gly Gln Phe Val Val Gly Arg Lys Lys Gly Tyr Gln Leu Gly Glu
625                 630                 635                 640

cta gga aac cta aat ctc tat ggc tca att aaa atc tcg cat ctt gag 1968
Leu Gly Asn Leu Asn Leu Tyr Gly Ser Ile Lys Ile Ser His Leu Glu
                645                 650                 655

aga gtg aag aat gat agg gac gca aaa gaa gcc aat tta tct gca aaa 2016
Arg Val Lys Asn Asp Arg Asp Ala Lys Glu Ala Asn Leu Ser Ala Lys
            660                 665                 670

ggg aat ctg cat tct tta agc atg agt tgg aat aac ttt gga cca cat 2064
Gly Asn Leu His Ser Leu Ser Met Ser Trp Asn Asn Phe Gly Pro His
        675                 680                 685

ata tat gaa tca gaa gaa gtt aaa gtg ctt gaa gcc ctc aaa cca cac 2112
Ile Tyr Glu Ser Glu Glu Val Lys Val Leu Glu Ala Leu Lys Pro His
    690                 695                 700

tcc aat ctg act tct tta aaa atc tat ggc ttc aga gga atc cat ctc 2160
Ser Asn Leu Thr Ser Leu Lys Ile Tyr Gly Phe Arg Gly Ile His Leu
705                 710                 715                 720

cca gag tgg atg aat cac tca gta ttg aaa aat att gtc tct att cta 2208
Pro Glu Trp Met Asn His Ser Val Leu Lys Asn Ile Val Ser Ile Leu
                725                 730                 735

att agc aac ttc aga aac tgc tca tgc tta cca ccc ttt ggt gat ctg 2256
Ile Ser Asn Phe Arg Asn Cys Ser Cys Leu Pro Pro Phe Gly Asp Leu
            740                 745                 750

cct tgt cta gaa agt cta gag tta cac tgg ggg tct gcg gat gtg gag 2304
Pro Cys Leu Glu Ser Leu Glu Leu His Trp Gly Ser Ala Asp Val Glu
        755                 760                 765

tat gtt gaa gaa gtg gat att gat gtt cat tct gga ttc ccc aca aga 2352
Tyr Val Glu Glu Val Asp Ile Asp Val His Ser Gly Phe Pro Thr Arg
    770                 775                 780

ata agg ttt cca tcc ttg agg aaa ctt gat ata tgg gac ttt ggt agt 2400
Ile Arg Phe Pro Ser Leu Arg Lys Leu Asp Ile Trp Asp Phe Gly Ser
785                 790                 795                 800

ctg aaa gga ttg ctg aaa aag gaa gga gaa gag caa ttc cct gtg ctt 2448
Leu Lys Gly Leu Leu Lys Lys Glu Gly Glu Glu Gln Phe Pro Val Leu
                805                 810                 815

gaa gag ctg ata att cac gag tgc cct ttt ctg acc ctt tct tct aat 2496
Glu Glu Leu Ile Ile His Glu Cys Pro Phe Leu Thr Leu Ser Ser Asn
            820                 825                 830

ctt agg gct ctt act tcc ctc aga att tgc tat aat aaa gta gct act 2544
Leu Arg Ala Leu Thr Ser Leu Arg Ile Cys Tyr Asn Lys Val Ala Thr
        835                 840                 845

tca ttc cca gaa gag atg ttc aaa aac ctt gca aat ctc aaa tac ttg 2592
Ser Phe Pro Glu Glu Met Phe Lys Asn Leu Ala Asn Leu Lys Tyr Leu
    850                 855                 860

aca atc tct cgg tgc aat aat ctc aaa gag ctg cct acc agc ttg gct 2640
Thr Ile Ser Arg Cys Asn Asn Leu Lys Glu Leu Pro Thr Ser Leu Ala
865                 870                 875                 880

agt ctg aat gct ttg aaa agt cta aaa att caa ttg tgt tgc gca cta 2688
Ser Leu Asn Ala Leu Lys Ser Leu Lys Ile Gln Leu Cys Cys Ala Leu
                885                 890                 895

gag agt ctc cct gag gaa ggg ctg gaa ggt tta tct tca ctc aca gag 2736
Glu Ser Leu Pro Glu Glu Gly Leu Glu Gly Leu Ser Ser Leu Thr Glu
            900                 905                 910

tta ttt gtt gaa cac tgt aac atg cta aaa tgt tta cca gag gga ttg 2784
Leu Phe Val Glu His Cys Asn Met Leu Lys Cys Leu Pro Glu Gly Leu
        915                 920                 925

cag cac cta aca acc ctc aca agt tta aaa att cgg gga tgt cca caa 2832
Gln His Leu Thr Thr Leu Thr Ser Leu Lys Ile Arg Gly Cys Pro Gln
    930                 935                 940

ctg atc aag cgg tgt gag aag gga ata gga gaa gac tgg cac aaa att 2880
Leu Ile Lys Arg Cys Glu Lys Gly Ile Gly Glu Asp Trp His Lys Ile
945                 950                 955                 960

tct cac att cct aat gtg aat ata tat aat taa 2913
Ser His Ile Pro Asn Val Asn Ile Tyr Asn
                965                 970

<210> 48
<211> 970
<212> PRT
<213> Solanum patita

<400> 48

Met Ala Glu Ala Phe Ile Gln Val Leu Leu Asp Asn Leu Thr Ser Phe
1               5                   10                  15

Leu Lys Gly Glu Leu Thr Leu Leu Phe Gly Phe Gln Asp Glu Phe Gln
            20                  25                  30

Arg Leu Ser Ser Met Phe Ser Thr Ile Gln Ala Val Leu Glu Asp Ala
        35                  40                  45

Gln Glu Lys Gln Leu Asn Asn Lys Pro Leu Glu Asn Trp Leu Gln Lys
    50                  55                  60

Leu Asn Ala Ala Thr Tyr Glu Val Asp Asp Ile Leu Asp Glu Tyr Lys
65                  70                  75                  80

Thr Lys Ala Thr Arg Phe Ser Gln Ser Glu Tyr Gly Arg Tyr His Pro
                85                  90                  95

Lys Val Ile Pro Phe Arg His Lys Val Gly Lys Arg Met Asp Gln Val
            100                 105                 110

Met Lys Lys Leu Lys Ala Ile Ala Glu Glu Arg Lys Asn Phe His Leu
        115                 120                 125

His Glu Lys Ile Val Glu Arg Gln Ala Val Arg Arg Glu Thr Gly Ser
    130                 135                 140

Val Leu Thr Glu Pro Gln Val Tyr Gly Arg Asp Lys Glu Lys Asp Glu
145                 150                 155                 160

Ile Val Lys Ile Leu Ile Asn Asn Val Ser Asp Ala Gln His Leu Ser
                165                 170                 175

Val Leu Pro Ile Leu Gly Met Gly Gly Leu Gly Lys Thr Thr Leu Ala
            180                 185                 190

Gln Met Val Phe Asn Asp Gln Arg Val Thr Glu His Phe His Ser Lys
        195                 200                 205

Ile Trp Ile Cys Val Ser Glu Asp Phe Asp Glu Lys Arg Leu Ile Lys
    210                 215                 220

Ala Ile Val Glu Ser Ile Glu Gly Arg Pro Leu Leu Gly Glu Met Asp
225                 230                 235                 240

Leu Ala Pro Leu Gln Lys Lys Leu Gln Glu Leu Leu Asn Gly Lys Arg
                245                 250                 255

Tyr Leu Leu Val Leu Asp Asp Val Trp Asn Glu Asp Gln Gln Lys Trp
            260                 265                 270

Ala Asn Leu Arg Ala Val Leu Lys Val Gly Ala Ser Gly Ala Ser Val
        275                 280                 285

Leu Thr Thr Thr Arg Leu Glu Lys Val Gly Ser Ile Met Gly Thr Leu
    290                 295                 300

Gln Pro Tyr Glu Leu Ser Asn Leu Ser Gln Glu Asp Cys Trp Leu Leu
305                 310                 315                 320

Phe Met Gln Arg Ala Phe Gly His Gln Glu Glu Ile Asn Pro Asn Leu
                325                 330                 335

Val Ala Ile Gly Lys Glu Ile Val Lys Lys Ser Gly Gly Val Pro Leu
            340                 345                 350

Ala Ala Lys Thr Leu Gly Gly Ile Leu Cys Phe Lys Arg Glu Glu Arg
        355                 360                 365

Ala Trp Glu His Val Arg Asp Ser Pro Ile Trp Asn Leu Pro Gln Asp
    370                 375                 380

Glu Ser Ser Ile Leu Pro Ala Leu Arg Leu Ser Tyr His Gln Leu Pro
385                 390                 395                 400

Leu Asp Leu Lys Gln Cys Phe Ala Tyr Cys Ala Val Phe Pro Lys Asp
                405                 410                 415

Ala Lys Met Glu Lys Glu Lys Leu Ile Ser Leu Trp Met Ala His Gly
            420                 425                 430

Phe Leu Leu Ser Lys Gly Asn Met Glu Leu Glu Asp Val Gly Asp Glu
        435                 440                 445

Val Trp Lys Glu Leu Tyr Leu Arg Ser Phe Phe Gln Glu Ile Glu Val
    450                 455                 460

Lys Asp Gly Lys Thr Tyr Phe Lys Met His Asp Leu Ile His Asp Leu
465                 470                 475                 480

Ala Thr Ser Leu Phe Ser Ala Asn Thr Ser Ser Ser Asn Ile Arg Glu
                485                 490                 495

Ile Asn Lys His Ser Tyr Thr His Met Met Ser Ile Gly Phe Ala Glu
            500                 505                 510

Val Val Phe Phe Tyr Thr Leu Pro Pro Leu Glu Lys Phe Ile Ser Leu
        515                 520                 525

Arg Val Leu Asn Leu Gly Asp Ser Thr Phe Asn Lys Leu Pro Ser Ser
    530                 535                 540

Ile Gly Asp Leu Val His Leu Arg Tyr Leu Asn Leu Tyr Gly Ser Gly
545                 550                 555                 560

Met Arg Ser Leu Pro Lys Gln Leu Cys Lys Leu Gln Asn Leu Gln Thr
                565                 570                 575

Leu Asp Leu Gln Tyr Cys Thr Lys Leu Cys Cys Leu Pro Lys Glu Thr
            580                 585                 590

Ser Lys Leu Gly Ser Leu Arg Asn Leu Leu Leu Asp Gly Ser Gln Ser
        595                 600                 605

Leu Thr Cys Met Pro Pro Arg Ile Gly Ser Leu Thr Cys Leu Lys Thr
    610                 615                 620

Leu Gly Gln Phe Val Val Gly Arg Lys Lys Gly Tyr Gln Leu Gly Glu
625                 630                 635                 640

Leu Gly Asn Leu Asn Leu Tyr Gly Ser Ile Lys Ile Ser His Leu Glu
                645                 650                 655

Arg Val Lys Asn Asp Arg Asp Ala Lys Glu Ala Asn Leu Ser Ala Lys
            660                 665                 670

Gly Asn Leu His Ser Leu Ser Met Ser Trp Asn Asn Phe Gly Pro His
        675                 680                 685

Ile Tyr Glu Ser Glu Glu Val Lys Val Leu Glu Ala Leu Lys Pro His
    690                 695                 700

Ser Asn Leu Thr Ser Leu Lys Ile Tyr Gly Phe Arg Gly Ile His Leu
705                 710                 715                 720

Pro Glu Trp Met Asn His Ser Val Leu Lys Asn Ile Val Ser Ile Leu
                725                 730                 735

Ile Ser Asn Phe Arg Asn Cys Ser Cys Leu Pro Pro Phe Gly Asp Leu
            740                 745                 750

Pro Cys Leu Glu Ser Leu Glu Leu His Trp Gly Ser Ala Asp Val Glu
        755                 760                 765

Tyr Val Glu Glu Val Asp Ile Asp Val His Ser Gly Phe Pro Thr Arg
    770                 775                 780

Ile Arg Phe Pro Ser Leu Arg Lys Leu Asp Ile Trp Asp Phe Gly Ser
785                 790                 795                 800

Leu Lys Gly Leu Leu Lys Lys Glu Gly Glu Glu Gln Phe Pro Val Leu
                805                 810                 815

Glu Glu Leu Ile Ile His Glu Cys Pro Phe Leu Thr Leu Ser Ser Asn
            820                 825                 830

Leu Arg Ala Leu Thr Ser Leu Arg Ile Cys Tyr Asn Lys Val Ala Thr
        835                 840                 845

Ser Phe Pro Glu Glu Met Phe Lys Asn Leu Ala Asn Leu Lys Tyr Leu
    850                 855                 860

Thr Ile Ser Arg Cys Asn Asn Leu Lys Glu Leu Pro Thr Ser Leu Ala
865                 870                 875                 880

Ser Leu Asn Ala Leu Lys Ser Leu Lys Ile Gln Leu Cys Cys Ala Leu
                885                 890                 895

Glu Ser Leu Pro Glu Glu Gly Leu Glu Gly Leu Ser Ser Leu Thr Glu
            900                 905                 910

Leu Phe Val Glu His Cys Asn Met Leu Lys Cys Leu Pro Glu Gly Leu
        915                 920                 925

Gln His Leu Thr Thr Leu Thr Ser Leu Lys Ile Arg Gly Cys Pro Gln
    930                 935                 940

Leu Ile Lys Arg Cys Glu Lys Gly Ile Gly Glu Asp Trp His Lys Ile
945                 950                 955                 960

Ser His Ile Pro Asn Val Asn Ile Tyr Asn
                965                 970

<210>  49
<211>  2913
<212>  DNA
<213>  Solanum bulbocastanum

<220>
<221>  CDS
<222>  (1)..(2913)

<400>  49
atg gct gaa gct ttc att caa gtt ctg cta gac aat ctc act tct ttc      48
Met Ala Glu Ala Phe Ile Gln Val Leu Leu Asp Asn Leu Thr Ser Phe
1               5                   10                  15

ctc aaa ggg gaa ctt gta ttg ctt ttc ggt ttt caa gat gag ttc caa      96
Leu Lys Gly Glu Leu Val Leu Leu Phe Gly Phe Gln Asp Glu Phe Gln
            20                  25                  30

agg ctt tca agc atg ttt tct aca att caa gcc gtc ctt gaa gat gct      144
Arg Leu Ser Ser Met Phe Ser Thr Ile Gln Ala Val Leu Glu Asp Ala
        35                  40                  45

cag gag aag caa ctc aac aac aag cct cta gaa aat tgg ttg caa aaa      192
Gln Glu Lys Gln Leu Asn Asn Lys Pro Leu Glu Asn Trp Leu Gln Lys
    50                  55                  60

ctc aat gct gct aca tat gaa gtc gat gac atc ttg gat gaa tat aaa      240
Leu Asn Ala Ala Thr Tyr Glu Val Asp Asp Ile Leu Asp Glu Tyr Lys
65                  70                  75                  80

acc aag gcc aca aga ttc tcc cag tct gaa tat ggc cgt tat cat cca      288
Thr Lys Ala Thr Arg Phe Ser Gln Ser Glu Tyr Gly Arg Tyr His Pro
                85                  90                  95

aag gtt atc cct ttc cgt cac aag gtc ggg aaa agg atg gac caa gtg      336
Lys Val Ile Pro Phe Arg His Lys Val Gly Lys Arg Met Asp Gln Val
            100                 105                 110

atg aaa aaa cta aag gca att gct gag gaa aga aag aat ttt cat ttg      384
Met Lys Lys Leu Lys Ala Ile Ala Glu Glu Arg Lys Asn Phe His Leu
        115                 120                 125

cac gaa aaa att gta gag aga caa gct gtt aga cgg gaa aca ggt tct      432
His Glu Lys Ile Val Glu Arg Gln Ala Val Arg Arg Glu Thr Gly Ser
    130                 135                 140

gta tta acc gaa ccg cag gtt tat gga aga gac aaa gag aaa gat gag      480
Val Leu Thr Glu Pro Gln Val Tyr Gly Arg Asp Lys Glu Lys Asp Glu
145                 150                 155                 160

ata gtg aaa atc cta ata aac aat gtt agt gat gcc caa cac ctt tca      528
Ile Val Lys Ile Leu Ile Asn Asn Val Ser Asp Ala Gln His Leu Ser
                165                 170                 175

gtc ctc cca ata ctt ggt atg ggg gga tta gga aaa acg act ctt gcc      576

Val Leu Pro Ile Leu Gly Met Gly Gly Leu Gly Lys Thr Thr Leu Ala
                180                 185                 190

caa atg gtc ttc aat gac cag aga gtt act gag cat ttc cat tcc aaa      624
Gln Met Val Phe Asn Asp Gln Arg Val Thr Glu His Phe His Ser Lys
        195                 200                 205

ata tgg att tgt gtc tcg gaa gat ttt gat gag aag agg tta ata aag      672
Ile Trp Ile Cys Val Ser Glu Asp Phe Asp Glu Lys Arg Leu Ile Lys
    210                 215                 220

gca att gta gaa tct att gaa gga agg cca cta ctt ggt gag atg gac      720
Ala Ile Val Glu Ser Ile Glu Gly Arg Pro Leu Leu Gly Glu Met Asp
225                 230                 235                 240

ttg gct cca ctt caa aag aag ctt cag gag ttg ctg aat gga aaa aga      768
Leu Ala Pro Leu Gln Lys Lys Leu Gln Glu Leu Leu Asn Gly Lys Arg
                245                 250                 255

tac ttg ctt gtc tta gat gat gtt tgg aat gaa gat caa cag aag tgg      816
Tyr Leu Leu Val Leu Asp Asp Val Trp Asn Glu Asp Gln Gln Lys Trp
            260                 265                 270

gct aat tta aga gca gtc ttg aag gtt gga gca agt ggt gct tct gtt      864
Ala Asn Leu Arg Ala Val Leu Lys Val Gly Ala Ser Gly Ala Ser Val
        275                 280                 285

cta acc act act cgt ctt gaa aag gtt gga tca att atg gga aca ttg      912
Leu Thr Thr Thr Arg Leu Glu Lys Val Gly Ser Ile Met Gly Thr Leu
    290                 295                 300

caa cca tat gaa ctg tca aat ctg tct caa gaa gat tgt tgg ttg ttg      960
Gln Pro Tyr Glu Leu Ser Asn Leu Ser Gln Glu Asp Cys Trp Leu Leu
305                 310                 315                 320

ttc atg caa cgt gca ttt gga cac caa gaa gaa ata aat cca aac ctt     1008
Phe Met Gln Arg Ala Phe Gly His Gln Glu Glu Ile Asn Pro Asn Leu
                325                 330                 335

gtg gca atc gga aag gag att gtg aaa aaa agt ggt ggt gtg cct cta     1056
Val Ala Ile Gly Lys Glu Ile Val Lys Lys Ser Gly Gly Val Pro Leu
            340                 345                 350

gca gcc aaa act ctt gga ggt att ttg tgc ttc aag aga gaa gaa aga     1104
Ala Ala Lys Thr Leu Gly Gly Ile Leu Cys Phe Lys Arg Glu Glu Arg
        355                 360                 365

gca tgg gaa cat gtg aga gac agt ccg att tgg aat ttg cct caa gat     1152
Ala Trp Glu His Val Arg Asp Ser Pro Ile Trp Asn Leu Pro Gln Asp
    370                 375                 380

gaa agt tct att ctg cct gcc ctg agg ctt agt tac cat caa ctt cca     1200
Glu Ser Ser Ile Leu Pro Ala Leu Arg Leu Ser Tyr His Gln Leu Pro
385                 390                 395                 400

ctt gat ttg aaa caa tgc ttt gcg tat tgt gcg gtg ttc cca aag gat     1248
Leu Asp Leu Lys Gln Cys Phe Ala Tyr Cys Ala Val Phe Pro Lys Asp
                405                 410                 415

gcc aaa atg gaa aaa gaa aag cta atc tct ctc tgg atg gcg cat ggt     1296
Ala Lys Met Glu Lys Glu Lys Leu Ile Ser Leu Trp Met Ala His Gly
            420                 425                 430

ttt ctt tta tca aaa gga aac atg gag cta gag gat gtg ggc gat gaa     1344
Phe Leu Leu Ser Lys Gly Asn Met Glu Leu Glu Asp Val Gly Asp Glu
        435                 440                 445

gta tgg aaa gaa tta tac ttg agg tct ttt ttc caa gag att gaa gtt     1392

Val Trp Lys Glu Leu Tyr Leu Arg Ser Phe Phe Gln Glu Ile Glu Val
    450                 455                 460

aaa gat ggt aaa act tat ttc aag atg cat gat ctc atc cat gat ttg 1440
Lys Asp Gly Lys Thr Tyr Phe Lys Met His Asp Leu Ile His Asp Leu
465                 470                 475                 480

gca aca tct ctg ttt tca gca aac aca tca agc agc aat atc cgt gaa 1488
Ala Thr Ser Leu Phe Ser Ala Asn Thr Ser Ser Ser Asn Ile Arg Glu
                485                 490                 495

ata aat aaa cac agt tac aca cat atg atg tcc att ggt ttc gcc gaa 1536
Ile Asn Lys His Ser Tyr Thr His Met Met Ser Ile Gly Phe Ala Glu
            500                 505                 510

gtg gtg ttt ttt tac act ctt ccc ccc ttg gaa aag ttt atc tcg tta 1584
Val Val Phe Phe Tyr Thr Leu Pro Pro Leu Glu Lys Phe Ile Ser Leu
        515                 520                 525

aga gtg ctt aat cta ggt gat tcg aca ttt aat aag tta cca tct tcc 1632
Arg Val Leu Asn Leu Gly Asp Ser Thr Phe Asn Lys Leu Pro Ser Ser
    530                 535                 540

att gga gat cta gta cat tta aga tac ttg aac ctg tat ggc agt ggc 1680
Ile Gly Asp Leu Val His Leu Arg Tyr Leu Asn Leu Tyr Gly Ser Gly
545                 550                 555                 560

atg cgt agt ctt cca aag cag tta tgc aag ctt caa aat ctg caa act 1728
Met Arg Ser Leu Pro Lys Gln Leu Cys Lys Leu Gln Asn Leu Gln Thr
                565                 570                 575

ctt gat cta caa tat tgc acc aag ctt tgt tgt ttg cca aaa gaa aca 1776
Leu Asp Leu Gln Tyr Cys Thr Lys Leu Cys Cys Leu Pro Lys Glu Thr
            580                 585                 590

agt aaa ctt ggt agt ctc cga aat ctt tta ctt gat ggt agc cag tca 1824
Ser Lys Leu Gly Ser Leu Arg Asn Leu Leu Leu Asp Gly Ser Gln Ser
        595                 600                 605

ttg act tgt atg cca cca agg ata gga tca ttg aca tgc ctt aag act 1872
Leu Thr Cys Met Pro Pro Arg Ile Gly Ser Leu Thr Cys Leu Lys Thr
    610                 615                 620

cta ggt caa ttt gtt gtt gga agg aag aaa ggt tat caa ctt ggt gaa 1920
Leu Gly Gln Phe Val Val Gly Arg Lys Lys Gly Tyr Gln Leu Gly Glu
625                 630                 635                 640

cta gga aac cta aat ctc tat ggc tca att aaa atc tcg cat ctt gag 1968
Leu Gly Asn Leu Asn Leu Tyr Gly Ser Ile Lys Ile Ser His Leu Glu
                645                 650                 655

aga gtg aag aat gat aag gac gca aaa gaa gcc aat tta tct gca aaa 2016
Arg Val Lys Asn Asp Lys Asp Ala Lys Glu Ala Asn Leu Ser Ala Lys
            660                 665                 670

ggg aat ctg cat tct tta agc atg agt tgg aat aac ttt gga cca cat 2064
Gly Asn Leu His Ser Leu Ser Met Ser Trp Asn Asn Phe Gly Pro His
        675                 680                 685

ata tat gaa tca gaa gaa gtt aaa gtg ctt gaa gcc ctc aaa cca cac 2112
Ile Tyr Glu Ser Glu Glu Val Lys Val Leu Glu Ala Leu Lys Pro His
    690                 695                 700

tcc aat ctg act tct tta aaa atc tat ggc ttc aga gga atc cat ctc 2160
Ser Asn Leu Thr Ser Leu Lys Ile Tyr Gly Phe Arg Gly Ile His Leu
705                 710                 715                 720

cca gag tgg atg aat cac tca gta ttg aaa aat att gtc tct att cta 2208

Pro Glu Trp Met Asn His Ser Val Leu Lys Asn Ile Val Ser Ile Leu
                725                 730                 735

att agc aac ttc aga aac tgc tca tgc tta cca ccc ttt ggt gat ctg      2256
Ile Ser Asn Phe Arg Asn Cys Ser Cys Leu Pro Pro Phe Gly Asp Leu
            740                 745                 750

cct tgt cta gaa agt cta gag tta cac tgg ggg tct gcg gat gtg gag      2304
Pro Cys Leu Glu Ser Leu Glu Leu His Trp Gly Ser Ala Asp Val Glu
        755                 760                 765

tat gtt gaa gaa gtg gat att gat gtt cat tct gga ttc ccc aca aga      2352
Tyr Val Glu Glu Val Asp Ile Asp Val His Ser Gly Phe Pro Thr Arg
    770                 775                 780

ata agg ttt cca tcc ttg agg aaa ctt gat ata tgg gac ttt ggt agt      2400
Ile Arg Phe Pro Ser Leu Arg Lys Leu Asp Ile Trp Asp Phe Gly Ser
785                 790                 795                 800

ctg aaa gga ttg ctg aaa aag gaa gga gaa gag caa ttc cct gtg ctt      2448
Leu Lys Gly Leu Leu Lys Lys Glu Gly Glu Glu Gln Phe Pro Val Leu
                805                 810                 815

gaa gag atg ata att cac gag tgc cct ttt ctg acc ctt tct tct aat      2496
Glu Glu Met Ile Ile His Glu Cys Pro Phe Leu Thr Leu Ser Ser Asn
            820                 825                 830

ctt agg gct ctt act tcc ctc aga att tgc tat aat aaa gta gct act      2544
Leu Arg Ala Leu Thr Ser Leu Arg Ile Cys Tyr Asn Lys Val Ala Thr
        835                 840                 845

tca ttc cca gaa gag atg ttc aaa aac ctt gca aat ctc aaa tac ttg      2592
Ser Phe Pro Glu Glu Met Phe Lys Asn Leu Ala Asn Leu Lys Tyr Leu
    850                 855                 860

aca atc tct cgg tgc aat aat ctc aaa gag ctg cct acc agc ttg gct      2640
Thr Ile Ser Arg Cys Asn Asn Leu Lys Glu Leu Pro Thr Ser Leu Ala
865                 870                 875                 880

agt ctg aat gct ttg aaa agt cta aaa att caa ttg tgt tgc gca cta      2688
Ser Leu Asn Ala Leu Lys Ser Leu Lys Ile Gln Leu Cys Cys Ala Leu
                885                 890                 895

gag agt ctc cct gag gaa ggg ctg gaa ggt tta tct tca ctc aca gag      2736
Glu Ser Leu Pro Glu Glu Gly Leu Glu Gly Leu Ser Ser Leu Thr Glu
            900                 905                 910

tta ttt gtt gaa cac tgt aac atg cta aaa tgt tta cca gag gga ttg      2784
Leu Phe Val Glu His Cys Asn Met Leu Lys Cys Leu Pro Glu Gly Leu
        915                 920                 925

cag cac cta aca acc ctc aca agt tta aaa att cgg gga tgt cca caa      2832
Gln His Leu Thr Thr Leu Thr Ser Leu Lys Ile Arg Gly Cys Pro Gln
    930                 935                 940

ctg atc aag cgg tgt gag aag gga ata gga gaa gac tgg cac aaa att      2880
Leu Ile Lys Arg Cys Glu Lys Gly Ile Gly Glu Asp Trp His Lys Ile
945                 950                 955                 960

tct cac att cct aat gtg aat ata tat att taa                          2913
Ser His Ile Pro Asn Val Asn Ile Tyr Ile
                965                 970

<210>  50
<211>  970
<212>  PRT
<213>  Solanum bulbocastanum

<400> 50

Met Ala Glu Ala Phe Ile Gln Val Leu Leu Asp Asn Leu Thr Ser Phe
1               5                   10                  15

Leu Lys Gly Glu Leu Val Leu Leu Phe Gly Phe Gln Asp Glu Phe Gln
            20                  25                  30

Arg Leu Ser Ser Met Phe Ser Thr Ile Gln Ala Val Leu Glu Asp Ala
        35                  40                  45

Gln Glu Lys Gln Leu Asn Asn Lys Pro Leu Glu Asn Trp Leu Gln Lys
    50                  55                  60

Leu Asn Ala Ala Thr Tyr Glu Val Asp Asp Ile Leu Asp Glu Tyr Lys
65                  70                  75                  80

Thr Lys Ala Thr Arg Phe Ser Gln Ser Glu Tyr Gly Arg Tyr His Pro
                85                  90                  95

Lys Val Ile Pro Phe Arg His Lys Val Gly Lys Arg Met Asp Gln Val
            100                 105                 110

Met Lys Lys Leu Lys Ala Ile Ala Glu Glu Arg Lys Asn Phe His Leu
        115                 120                 125

His Glu Lys Ile Val Glu Arg Gln Ala Val Arg Arg Glu Thr Gly Ser
    130                 135                 140

Val Leu Thr Glu Pro Gln Val Tyr Gly Arg Asp Lys Glu Lys Asp Glu
145                 150                 155                 160

Ile Val Lys Ile Leu Ile Asn Asn Val Ser Asp Ala Gln His Leu Ser
                165                 170                 175

Val Leu Pro Ile Leu Gly Met Gly Gly Leu Gly Lys Thr Thr Leu Ala
            180                 185                 190

Gln Met Val Phe Asn Asp Gln Arg Val Thr Glu His Phe His Ser Lys
        195                 200                 205

Ile Trp Ile Cys Val Ser Glu Asp Phe Asp Glu Lys Arg Leu Ile Lys
    210                 215                 220

Ala Ile Val Glu Ser Ile Glu Gly Arg Pro Leu Leu Gly Glu Met Asp
225                 230                 235                 240

Leu Ala Pro Leu Gln Lys Lys Leu Gln Glu Leu Leu Asn Gly Lys Arg
                245                 250                 255

Tyr Leu Leu Val Leu Asp Asp Val Trp Asn Glu Asp Gln Gln Lys Trp

            260                 265                 270

Ala Asn Leu Arg Ala Val Leu Lys Val Gly Ala Ser Gly Ala Ser Val
        275                 280                 285

Leu Thr Thr Thr Arg Leu Glu Lys Val Gly Ser Ile Met Gly Thr Leu
    290                 295                 300

Gln Pro Tyr Glu Leu Ser Asn Leu Ser Gln Glu Asp Cys Trp Leu Leu
305                 310                 315                 320

Phe Met Gln Arg Ala Phe Gly His Gln Glu Glu Ile Asn Pro Asn Leu
                325                 330                 335

Val Ala Ile Gly Lys Glu Ile Val Lys Lys Ser Gly Gly Val Pro Leu
            340                 345                 350

Ala Ala Lys Thr Leu Gly Gly Ile Leu Cys Phe Lys Arg Glu Glu Arg
        355                 360                 365

Ala Trp Glu His Val Arg Asp Ser Pro Ile Trp Asn Leu Pro Gln Asp
    370                 375                 380

Glu Ser Ser Ile Leu Pro Ala Leu Arg Leu Ser Tyr His Gln Leu Pro
385                 390                 395                 400

Leu Asp Leu Lys Gln Cys Phe Ala Tyr Cys Ala Val Phe Pro Lys Asp
                405                 410                 415

Ala Lys Met Glu Lys Glu Lys Leu Ile Ser Leu Trp Met Ala His Gly
            420                 425                 430

Phe Leu Leu Ser Lys Gly Asn Met Glu Leu Glu Asp Val Gly Asp Glu
        435                 440                 445

Val Trp Lys Glu Leu Tyr Leu Arg Ser Phe Phe Gln Glu Ile Glu Val
    450                 455                 460

Lys Asp Gly Lys Thr Tyr Phe Lys Met His Asp Leu Ile His Asp Leu
465                 470                 475                 480

Ala Thr Ser Leu Phe Ser Ala Asn Thr Ser Ser Ser Asn Ile Arg Glu
                485                 490                 495

Ile Asn Lys His Ser Tyr Thr His Met Met Ser Ile Gly Phe Ala Glu
            500                 505                 510

Val Val Phe Phe Tyr Thr Leu Pro Pro Leu Glu Lys Phe Ile Ser Leu
        515                 520                 525

Arg Val Leu Asn Leu Gly Asp Ser Thr Phe Asn Lys Leu Pro Ser Ser

530                 535                 540

Ile Gly Asp Leu Val His Leu Arg Tyr Leu Asn Leu Tyr Gly Ser Gly
545                 550                 555                 560

Met Arg Ser Leu Pro Lys Gln Leu Cys Lys Leu Gln Asn Leu Gln Thr
                565                 570                 575

Leu Asp Leu Gln Tyr Cys Thr Lys Leu Cys Cys Leu Pro Lys Glu Thr
            580                 585                 590

Ser Lys Leu Gly Ser Leu Arg Asn Leu Leu Leu Asp Gly Ser Gln Ser
        595                 600                 605

Leu Thr Cys Met Pro Pro Arg Ile Gly Ser Leu Thr Cys Leu Lys Thr
    610                 615                 620

Leu Gly Gln Phe Val Val Gly Arg Lys Lys Gly Tyr Gln Leu Gly Glu
625                 630                 635                 640

Leu Gly Asn Leu Asn Leu Tyr Gly Ser Ile Lys Ile Ser His Leu Glu
                645                 650                 655

Arg Val Lys Asn Asp Lys Asp Ala Lys Glu Ala Asn Leu Ser Ala Lys
            660                 665                 670

Gly Asn Leu His Ser Leu Ser Met Ser Trp Asn Asn Phe Gly Pro His
        675                 680                 685

Ile Tyr Glu Ser Glu Glu Val Lys Val Leu Glu Ala Leu Lys Pro His
    690                 695                 700

Ser Asn Leu Thr Ser Leu Lys Ile Tyr Gly Phe Arg Gly Ile His Leu
705                 710                 715                 720

Pro Glu Trp Met Asn His Ser Val Leu Lys Asn Ile Val Ser Ile Leu
                725                 730                 735

Ile Ser Asn Phe Arg Asn Cys Ser Cys Leu Pro Pro Phe Gly Asp Leu
            740                 745                 750

Pro Cys Leu Glu Ser Leu Glu Leu His Trp Gly Ser Ala Asp Val Glu
        755                 760                 765

Tyr Val Glu Glu Val Asp Ile Asp Val His Ser Gly Phe Pro Thr Arg
    770                 775                 780

Ile Arg Phe Pro Ser Leu Arg Lys Leu Asp Ile Trp Asp Phe Gly Ser
785                 790                 795                 800

Leu Lys Gly Leu Leu Lys Lys Glu Gly Glu Glu Gln Phe Pro Val Leu

                805                 810                 815

Glu Glu Met Ile Ile His Glu Cys Pro Phe Leu Thr Leu Ser Ser Asn
            820                 825                 830

Leu Arg Ala Leu Thr Ser Leu Arg Ile Cys Tyr Asn Lys Val Ala Thr
        835                 840                 845

Ser Phe Pro Glu Glu Met Phe Lys Asn Leu Ala Asn Leu Lys Tyr Leu
    850                 855                 860

Thr Ile Ser Arg Cys Asn Asn Leu Lys Glu Leu Pro Thr Ser Leu Ala
865                 870                 875                 880

Ser Leu Asn Ala Leu Lys Ser Leu Lys Ile Gln Leu Cys Cys Ala Leu
                885                 890                 895

Glu Ser Leu Pro Glu Glu Gly Leu Glu Gly Leu Ser Ser Leu Thr Glu
            900                 905                 910

Leu Phe Val Glu His Cys Asn Met Leu Lys Cys Leu Pro Glu Gly Leu
        915                 920                 925

Gln His Leu Thr Thr Leu Thr Ser Leu Lys Ile Arg Gly Cys Pro Gln
    930                 935                 940

Leu Ile Lys Arg Cys Glu Lys Gly Ile Gly Glu Asp Trp His Lys Ile
945                 950                 955                 960

Ser His Ile Pro Asn Val Asn Ile Tyr Ile
                965                 970

## Description

The invention relates to the field of plant transformation, in particular plant transformation of a Solanaceae, preferably of potato.

Transformation of plants using transforming bacteria such as Agrobacterium spp. to obtain transgenic plants expressing a heterologous gene or a gene fragment of interest has been known since the late seventies and early eighties of the last century when it was found that the Ti (tumor inducing) plasmid of said bacterium could be used as a vector in genetic engineering of plants. The wild-type plasmid induces plant cells to produce tumor cells, but it can be modified so that it can carry foreign gene constructs into cells without necessarily making the recipient cells tumorous. During tumor induction, a specific segment of the Ti plasmid, called the T-DNA (transferred DNA), integrates into the host plant nuclear DNA. In genetic engineering of plants, said T-DNA is modified and now carries the foreign gene construct to be integrated in the plant's DNA so that a transgenic plant can be obtained.

A transformation procedure for obtaining transgenic plants generally consists of infection of a plant cell with a transforming bacterium, which generally comprises an essentially non-tumorigenic Agrobacterium strain, which bacterium is provided with a recombinant nucleic acid comprising a T-DNA vector construct allowing for transfer of said construct into the genome of a plant cell, said construct essentially comprising the desired nucleic acid, gene or gene fragment that one wishes to see expressed in a finally transformed plant and a selective marker nucleic acid or selection gene. This desired heterologous gene and the marker are in general located on a plasmid or vector in a piece of the T-DNA, which is the DNA flanked by at least one, or located between two imperfect direct repeats of most often 24 basepairs length, called the T-DNA borders. Transfer of the heterologous gene/selection gene construct into the plant cell takes place in a process whereby vir genes (located on the same or different plasmid) are involved to accommodate transfer and integration of the T-DNA/gene construct. Vir-proteins (D1 and D2) cause nicking of the border repeats at a precise site whereby the T-DNA construct is cut at the T-DNA borders from the plasmid and inserted into the plant genome.

By growing the thus treated plant cells in an appropriate environment, whole plants can be regenerated, some of which carry the desired gene. To select for the desired transformed cells in the background of untransformed cells DNA encoding said selection gene and means for its expression is generally physically linked to the gene of interest on the T-DNA to permit the recovery of transformants. The presence of such a selection or marker sequence in the T-DNA is generally seen as an absolute requirement for efficient recovery; otherwise tenths- to hundreds-of-thousands putative transformants would need to be screened for the presence and functional insertion of the desired heterologous gene. Instead, putative transformants are almost always grown in a selective medium or under selective pressure appropriate for the selective marker chosen to give the transformed cells a selective advantage over the, initially over-abundantly present, non-transformed cells.

Historically, a plant selection marker is a dominant gene which, after expression, confers resistance to a selective agent that is added to the regeneration medium, but which itself is not essential for cell growth. Such a selective agent is for example an antibiotic, herbicide, amino acid or amino acid analog added to a plant or plant culturing medium in a toxic concentration. Among the selective markers or selection genes that are most widely used in plant transformation are the bacterial neomycin phosphotransferase genes (nptI, nptII and nptIII genes) conferring resistance to the selective agent kanamycin, suggested in EP131623 and the bacterial aphIV gene suggested in EP186425 conferring resistance to hygromycin. EP 275957 discloses the use of an acetyl transferase gene from *Streptomyces viridochromogenes* that confers resistance to the herbicide phosphinotricin. Plant genes conferring relative resistance to the herbicide glyphosate are suggested in EP218571. The resistance is based on the expression of a gene encoding 5-enolshikimate-3-phosphate synthase (EPSPS) that is relatively tolerant to N-phosphomethylglycine. Certain amino acids such as lysine, threonine, or the lysine derivative amino ethyl cysteine (AEC) and tryptophan analogs like 5-methyl tryptophan can also be used as selective agents due to their ability to inhibit cell growth when applied at high concentration. In this selection system expression of the selectable marker gene results in overproduction of amino acids by transgenic cells which permits the transgenic to grow under selection.

Another class of selectable markers are those that support growth and proliferation of the transformed plant cells under conditions that are insufficient for the growth of non-transformed cells, e.g. conditions lacking a plant growth hormone. A selection marker gene can encode an enzyme that after expression converts an encryptic carbon source into a carbon source that supports growth and proliferation of the transformed plant cells under conditions that contain minimal nutrients and in which the carbon source is replaced by an encryptic or latent carbon source that cannot be utilized by non-transformed cells. An example of such a positive selection marker is the phosphomannose isomerase that converts non-utilizable mannose-6-phosphate into fructose-6-phosphate that can be used by plant cells as a carbon source (suggested in US6143562) or xylose isomerase from *Streptomyces rubiginosus* (Haldrup A., et al. 1998, Plant Cell Report 18:76-81).

Yet another class of selection marker genes allows the screening of presumably transformed plant cells rather than direct genetic selection of transformed cells for resistance to a toxic substance such as an antibiotic. For this reason they are often also called screenable markers. The genes are referred to as reporter genes and include for instance beta-glucuronidase (GUS), beta-galactosidase, luciferase, and green fluorescent protein (GFP).

Alternatively, a screenable marker may provide some other visible reactive response. For instance, it may cause a distinctive appearance or growth pattern relative to plants or plant cells not expressing a screenable marker gene in the presence of a substance, which can either be applied to the plant or plant cells directly or a substance which is present in the plant or in the plant cell growth media.

Generally, the plants or plant cells containing such screenable marker genes have a distinctive phenotype for purpose of identification, i.e., they can be distinguished from non-transformed cells. The characteristic phenotype allows the identification of cells, cell groups, tissues, organs, plant parts or whole plants containing the construct. An example of a morphological abnormality induction (MAI) marker gene is the isopentenyl transferase gene from Agrobacterium (Keller et al. WO 00/37060). Isopentenyl transferase is a rate-limiting enzyme in the biosynthesis of cytokinin, which is a plant growth hormone. A plant cell into which the ipt gene is introduced produces cytokinin, with the result that the proliferation and differentiation of the cell containing the ipt gene are confused to induce various morphological abnormalities.

The presence of antibiotic resistance genes and other selective markers sequences in the final transgenic plant obtained is in most cases considered undesirable. These sequences, albeit thought to be necessary for the transformation processes, do in general not positively contribute to the final transformed plant and in fact lessen its desirability to the consumer for a number of reasons. Consumer groups express concern about the widespread distribution of resistance markers in food products referring to a theoretical risk of a horizontal transfer of transgene selection genes into gut bacteria.

Environmental groups are concerned about the risk of cross-pollination between the transgenic plant and related species which can lead to a transfer of resistance traits into weeds, jeopardizing the long-term use of transgenic crops and causing potential ecological problems.

A number of systems have so far been developed to facilitate the removal of selective marker genes. Co-transformation of two different constructs can result in transgenic lines that have integrated both transgenes and after a genetic cross the selective marker can be segregated away from the gene of interest. Removal of selective marker genes by co-transformation is suggested in e.g. WO95/16031, US 6265638 and WO00/18939. A co-transformation system however requires that the marker gene is located at an unlinked location meaning screening of many more independent transformation events. Moreover, many cross pollinating and in particular vegetatively propagated crops (like apple or strawberry), and especially tuberous crops like potato and cassava, are highly heterozygous and removal of the marker sequence by genetic segregation would require many years to find a clone with suitable field performance. Several transposable element systems and site-specific recombination systems have been employed for marker removal (Sugita K, et al. 2000 Plant J 22: 461-469; Zuo J, et al. 2001 Nature Biotech 19: 157-161). EP 716147 describes a vector for introducing a desired gene into plants, comprising the gene of interest and at least a morphological abnormality induction (MAI) marker gene placed on a removable DNA element i.e. a transposable element or site-specific recombination system. Plants transformed with the MAI containing gene construct can be easily detected by eye by their abnormal morphology of the shoots. Likewise, the loss of the MAI gene's function after transposition of the transposable element or after site-specific removal of the recombination system can be easily detected as this results in normal looking shoots. Such transgenic plants can be produced free of marker genes without having to undergo the crossing step. These systems require the expression of a transposase or recombinase that mediates the deletion of regions bracketed between recombination or transposase target sequences, and the subsequent removal of the marker gene by genetic segregation. Also these systems are time consuming as well as impractical for species that are mainly propagated vegetatively. Moreover, deleted fragments can reinsert into other genomic positions. Another approach to induce DNA deletions is based on intrachromosomal homologous recombination between two homologous sequences. Intrachromosomal homologous recombination frequencies are often too low for an efficient application of this system to produce deletions of transgene regions. Using the attP region of bacteriophage λ this recombination frequency could be increased (Zubko E, et al. 2000 Nature Biotech 18: 442-445). However, the process is still unpredictable and too inefficient to generate hundreds to thousands of independent transformation events to find the clone with suitable agronomical performance. A system to recover transgenic cells without the use of selective markers has been described. In WO98/51806 a method is disclosed for the recovery of transformed cells without the use of selectable markers by enrichment of transgenic sectors using nodal culture and non-selective screening assays. This method involves the culturing of the transformed plant cells or tissue comprising a non-selectively assayable transgene until nodes comprising meristematic tissue have developed. Subsequently, the plant tissue is assayed using a non-selective assay, such as enzyme assays or ELISA's. Assay-positive plants that are recovered with this method are chimeric and have transformed sectors. To enrich these transformed sectors from the assay-positive tissue nodal explants are prepared and cultured such that shoots are formed. Shoots and leaves are to be assayed again using a non-selective assay in the hope that plants are recovered with enriched transformed sectors so that eventually, after several rounds of assaying, near-uniform transgenic plants can be obtained.

WO 03/010319 describes a method for obtaining marker-free plants comprising a recombinant nucleic acid comprising a T-DNA construct allowing for transfer of said construct into the genome of a plant cell, said construct provided with a foreign nucleic acid that is free of nucleic acid encoding a selective marker. This method has proven to be very useful. However, there is still a need for further alternative transformation methods.

The present invention provides an alternative selection method in plant transformation processes.

Besides the fact that the presence of antibiotic resistance genes and other selective markers sequences in the final transgenic plant obtained is in most cases considered undesirable, there is a more general reluctance to the presence of non-plant sequences (i.e. trans sequences) in a transgenic plant. The invention further provides a plant that has been provided with additional nucleic acid sequences but which genetically modified plant essentially consists of cis plant sequences, for example a genetically modified potato plant that has been provided with additional (essentially) potato plant (coding) sequences from another potato variety, breeding clones or crossable species. Such a transgenic plant is herein further referred to as a cisgene plant. In a preferred embodiment, commercial interesting potato plants (such as Desiree or Bintje) have been provided with nucleic acid sequences isolated from other potato species (for example from a wild species of potato such as S. bulbocastanum).

Preferably, an obtained genetically modified Solanum plant comprises Solanum 5' and 3' sequences to direct transcription of a cDNA sequence or a genomic sequence. The used cDNA or genomic sequence may be orientated sense or antisense in relation to the used 5' and 3' sequences. The 5' and 3' sequences can be originally linked to the used cDNA or genomic sequence or they can normally be linked to another coding sequence in a Solanum. In a preferred embodiment, the used coding sequence is a genomic sequence (in sense or antisense orientation) and the used 5' and 3' sequences are the ones that are linked to the genomic sequence under normal conditions (i.e. the 5' and 3' sequences are in operable linkage with their natural coding sequence).

In a first embodiment, the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor.

Such a method is very suitable for determining whether a nucleic acid of interest has been successfully transferred to a plant, i.e. whether plant transformation has succeeded.

The term "recombinant nucleic acid" is typically used to describe a double or single stranded DNA or RNA molecule and includes circular as well as linear forms of nucleic acid. In some of the embodiments, the recombinant nucleic acid used in a method of the invention at least comprises (i) a recombinant nucleic acid of interest and (ii) a functional R-gene. However the nucleic acid of interest and the functional R-gene may also be one and the same nucleic acid and hence in such an embodiment, the recombinant nucleic acid used in a method of the invention at least comprises a functional R-gene. In the latter case, said functional R-gene is used as a selection tool as well as to provide (at least partial) protection against a fungal infection/pathogen.

The recombinant nucleic acid used in a method of the invention may also comprise other sequences (i.e. besides a functional R-gene or the combination of a recombinant nucleic acid of interest and a functional R-gene). The choice of these other sequences depends for example on the used method of transfer or on the purpose (i.e. or the goal) of the recombinant nucleic acid. If for example Agrobacterium is the selected transfer method, the recombinant nucleic acid used in a method of the invention further comprises backbone/vector sequences as well as at least one and preferably two (optionally modified) T-DNA border sequences.

Border sequences are well known in the art and they need no further elaborate discussion. For obtaining a maximum number of transformants with (deleted) non-integrated borders, said borders are preferably short with a minimum of 25 bp left and right, however the use of longer borders is also feasible. We have analyzed five transformants obtained with construct pKGBA50mf-IR1.1 (de Vetten et al., 2003) in detail, and observed deletions of the T-DNA sequence from 39-237 bp at the RB side and from 79-1054 bp at the LB side. Zhu et al. (2006) reported similar results. They analyzed 171 left borders and 134 right borders from independent transgenic rice plants and observed deletions of the T-DNA up to 35 bp at the RB side and up to 340 bp at the LB side. They stated that probably some transformants had deletions longer than 611 bp at the LB side. Windels et al. (2003) analyzed 67 T-DNA/plant DNA junctions from *Arabidopsis* transformants. They observed deletions of T-DNA sequences up to 629 bp at the RB side and up to 2309 bp at the LB side.

On the basis of these observations it is expected that transformants can be selected that contain only Solanaceae sequence integrated in the genome.

As disclosed herein within the experiment part, a preferred left border setting is one in which an attenuation region in combination with a double left border is used. This setting prevents more frequently backbone integration. Integration of backbone vector (non-T-DNA) sequences in the plant genome frequently occurs during *Agrobacterium tumefaciens* transformation (Kononov et al. 1997; Ramanathan and Veluthambi, 1995; Van der Graaff et al., 1996; Wenck et al., 1997; Wolters et al., 1998). Wang et al. (1987) reported that "flanking sequences of the border repeats enhance (on the right) or attenuate (on the left) their activity". They defined an 'attenuation region': a 363-bp AT-rich EcoRI/BclI fragment of the T-DNA near the LB of plasmid pTiC58. De Buck et al. (2000) stated that "it is possible that deletion of the inner border region, a piece of T-DNA present in the original Ti plasmid from which the vector was derived, causes inefficient nicking of the LB repeat, which results in read-through past the LB and the transfer of downstream-located vector sequences". Kuraya et al. (2004) reported that "transfer of the 'vector backbone' from the control vectors resulted mainly from inefficient termination of formation of the transfer intermediate of the T-DNA, and additional LB sequences effectively suppressed such transfer".

On the basis of these reports we have cloned in one of our examples an extra nopaline type LB together with the flanking attenuation region outside the LB of a binary vector, to prevent integration of backbone vector DNA in the plant genome.

As described above the recombinant nucleic acid used in a method of the invention may further comprise backbone/vector sequences. The backbone/vector sequences may for example comprise a selection marker for maintaining the recombinant nucleic acid in E.coli and/or in Agrobacterium.

It is clear to the skilled person that if one would like to introduce multiple nucleic acids of interests, the recombinant nucleic acid used in a method of the invention may comprise multiple nucleic acids of interest.

Other sequences present on the recombinant nucleic acid used in a method of the invention are promoter and/or terminator sequences, preferably functionally linked/coupled to a nucleic acid of interest and/or to an R-gene. Preferred embodiments of these promoter and/or terminator sequences are described later on.

It is clear to the skilled person that a vast amount of nucleic acids of interest can be thought of and which are all included herein. In one embodiment, said nucleic acid of interest allows for regulation of the expression of a target gene in said genome. Both up-regulation (or over-expression) and down-regulation can be achieved by "sense" technology. If a full-length copy of the target gene is inserted into the genome a range of phenotypes can be obtained, some over-expressing the target gene, some under-expressing. A population of plants produced by this method may then be screened and individual phenotypes isolated. A preferred embodiment comprises a method wherein said regulation comprises down-regulation. The inhibition of expression of a target gene, commonly referred to as "gene silencing" can be achieved by "antisense down-regulation" and "sense down-regulation" (also, referred to as "co-suppression"). In antisense down-regulation, a DNA fragment which is complementary to all or part of an endogenous target gene is inserted into the genome in reverse orientation. While the mechanism has not been fully elucidated, one theory is that transcription of such an antisense gene produces mRNA which is complementary in sequence to the mRNA product transcribed from the endogenous gene. The antisense mRNA then binds with the naturally produced "sense" mRNA to form a duplex which inhibits translation of the natural mRNA to protein. Antisense down-regulation technology is well-established in the art and used routinely in laboratories around the world. Gene silencing can therefore be achieved by inserting into the genome of a target organism a copy of at least a fragment of the target gene coding sequence which copy may comprise either the whole or part or be a truncated sequence and may be in sense or antisense orientation. Additionally, intron sequences which can be obtained from the genomic gene sequence may be used in the construction of suppression vectors. There have also been reports of gene silencing being achieved within organisms of both the transgene and the endogenous gene where the only sequence identity is within the promoter regions.

In a much preferred embodiment, the invention provides a recombinant nucleic acid wherein said nucleic acid of interest includes an inverted repeat of at least part of a polynucleotide region of said target gene. Although antisense and sense down-regulation can result in complete silencing of the target gene, the efficiency is generally not very high. A maximum of 25% of the antisense transformants displayed complete silencing, while only about 10% of the transformants obtained with sense constructs showed some level of silencing (Smith et al. 2000 Nature 407: 319-320; Wolters and Visser, 2000 Plant Mol Biol 43: 377-386). Recently, it has been observed that the inhibition of a selected target gene within an organism is enhanced, when the gene silencing vector includes a inverted repeat of all or part of a polynucleotide region of the target gene. The inverted repeat sequence may consist of for example a T-DNA with one promoter driving expression of a sense copy of the cDNA together with another promoter in front of an antisense copy of the same cDNA (Chuang and Meyerowitz, 2000 Proc Natl Acad Sci USA 97: 4985-4990) or the T-DNA may contain a cDNA sequence flanked on both ends by a promoter (LaCount et al. 2000 Mol Biochem Parasit 111: 67-76), or the T-DNA may contain a promoter driving transcription of an inverted repeat of (part of) the cDNA (Hamilton et al. 1998; Smith et al. 2000 Nature 407: 319-320; Wang and Waterhouse, 2000 Wang MB, Waterhouse PM (2000) Plant Mol Biol 43: 67-82) or the promoter (Mette et al. 2000 EMBO J 19: 5194-5201) of the gene to be silenced. Some inverted repeat constructs are reported to result in 100% of the transformants displaying silencing of the target gene (Smith et al. 2000 Nature 407: 319-320) in case of using an intron as a spacer between the repeats. The spacer fragment contributes to the stability of the perfect inverted repeat sequences, but is not required for the specificity of the silencing.

In a particularly preferred embodiment, a nucleic acid of interest encodes a granule-bound starch synthase (GBSSI) enzyme or comprises an antisense sequence. The inclusion of a short repeated region of the granule-bound starch synthase (GBSSI) enzyme of potato within a transgene results in a striking increase in the frequency of completely silenced transformants.

Also provided is a nucleic acid of interest that allows for expression of a heterologous polypeptide in a plant cell. Suitable polypeptides are manifold, typical examples of foreign nucleic acid or genes that are of interest to transfer into plants are those encoding for proteins and enzymes that modify metabolic and catabolic processes. Other examples are genes that may encode a protein giving added nutritional value to the plant as a food or crop. Typical examples include plant proteins that can inhibit the formation of anti-nutritive factors and plant proteins that have a more desirable amino acid composition (e.g. a higher lysine content than the non-transgenic plant). In a preferred embodiment, said nucleic acid of interest encodes a polypeptide which comprises an enzyme. The nucleic acid of interest may also code for an enzyme that can be used in food processing such as chymosin, thaumatin and alpha-galactosidase. The nucleic acid of interest may also code for an agent for introducing or increasing pathogen resistance. Preferably, the gene of interest is a gene encoding for a protein or peptide having a high nutritional value, a feedback-insensitive amino acid biosynthesis enzyme such as dihydrodipicolinate synthase (EC 4.2.1.52, DHPS), an enzyme or peptide conferring disease resistance, a sense or antisense transcript such as that for patatin, ADP-glucose pyrophosphorylase, alpha-amylase, branching enzyme, granule-bound starch synthase, soluble starch synthases, a protease or a glucanase.

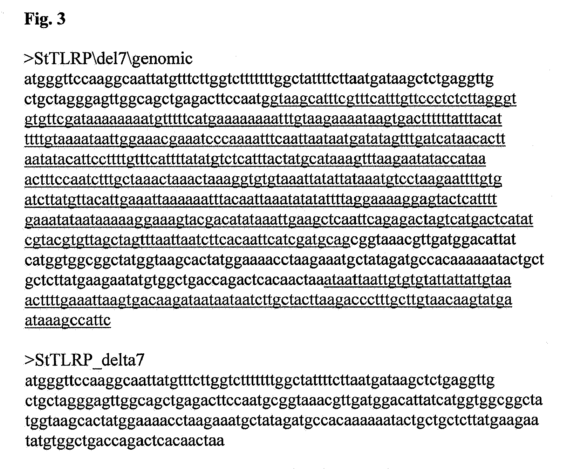

In yet another embodiment, a nucleic acid of interest is a nucleic acid sequence that modulates the texture properties of plant tissues and organs, especially after heat treatment, such as cooking. Preferably, the cooking type of potato tubers can be altered, from example from "mealy" to "firm" or the other way around. An example of such a gene is a sttlrp nucleic acid that encodes a so-called StTLRP protein (Solanum tuberosum tyrosine and lysine rich protein). Figure 3 provides the nucleic acid sequence of such an sttlrp nucleic acid.

StTLRP proteins (and *sttlrp* nucleic acids encoding these) are suitable for modulating texture characteristics of plant cell walls, especially cells (and tissues consisting largely thereof) which lack a rigid (lignified) secondary cell wall. A significant correlation between mRNA expression levels and firmness of potato tubers was found. Potato genoptypes comprising a specific allele of the *sttlrp* gene (the *sttlrp* Δ7 allele) had *sttlrp* mRNA levels which were much higher (e.g. 64-fold upregulated) than genotypes lacking this allele. These genotypes had on average much firmer tissue after cooking. In contrast, genotypes which lacked the Δ7 allele were mealy after cooking. Therefore, particular high expressing alleles are able to confer a 'firm', 'non-mealy' phenotype to the potato tubers, while low expressing alleles (and thus the absence of high expressing alleles) are capable of conferring a 'mealy' phenotype. The genomic and cDNA nucleic acid sequences of the Δ7 allele are shown in Figure 3.

In yet another preferred embodiment, the invention provides a method wherein the nucleic acid of interest is also a functional R-gene. The term R-gene is typically used to describe a DNA sequence that encodes a gene product that is capable of providing a plant with (at least partial) resistance against a pathogen. Even more preferred, the R-gene used in the selection process is also used to provide the obtained plant with resistance. Preferably, the R-gene used in the selection process and the further functional R-gene (as a nucleic acid of interest) are chosen such that they provide complementary (and even more preferably partially overlapping) protection against a pathogen. The use of at least two preferably different R-genes results in so-called stacking of R-genes.

Whether or not multiple, different R-genes provide a complementary and/or overlapping effect is for example tested by ATTA, i,e. Agrobacterium tumefaciens transient expression assay. In this assay the nucleotide sequence coding for an R-gene which is to be tested is introduced into an Agrobacterium strain which is also used in protocols for stable transformation. After incubation of the bacteria with acetosyringon or any other phenolic compound which is known to enhance Agrobacterium T-DNA transfer, a certain amount of the Agrobacterium culture is infiltrated into an in situ plant leaf (for example a tobacco or potato or tomato plant) by injection after which the plants are placed in a greenhouse and infected with a pathogen (for example P. infestans). After 2-5 days the leaves can be scored for occurrence of resistance symptoms. Multiple different R-genes can be tested versus multiple different P. infestans isolates or versus multiple different elicitors.

Another method to test whether or not multiple, different R-genes provide a complementary and/or overlapping effect is by using a detached leaf assay with, preferably well-characterised, isolates (for example P. infestans isolates) or with an effector (such as an elicitor) which disclose that iterative functional allele mining in Solanum and Phytophthora is a powerful tool to identify and characterize R-avr combinations at a much higher discriminatory resolution than was previously possible using only isolate screens.

Wild *Solanum* species have previously been subject to mapping of genes for resistance to *P. infestans.* A summary of mapped R-genes for foliage late blight resistance is given in Table 1. Currently 4 R-genes for late blight resistance have been cloned and all belong to the NB-LRR class of plant R-genes; *R1* and *R3a* from *S*. *demissum* (Ballvora et al., 2002; Huang et al., 2005) and *Rpi-blb1* and *Rpi-blb2* from *S*. *bulbocastanum* (van der Vossen et al, 2003, 2005). Examples of R-genes that can be used as a nucleic acid of interest are provided in Table 1.

However, also R-genes isolated from other plants than potato can be used in a method of the present invention. Preferably the used functional R-gene and the elicitor are matched. For example, if one wants to use Rps1b from soybean plants, the used elicitor is preferably an elicitor of Phytophthora sojae (preferably Avr1b-1). Other examples are provided in the Table below.

| Host | R gene | Avr gene | Pathogen | |
|---|---|---|---|---|
| Potato | *R3a* | *Avr3a* | *Phytophthora infestans* | *Oomycete* |
| Potato | *Rpi-blb1* | *ipiO* | *Phytophthora infestans* | *Oomycete* |
| Soybean | *Rps1b* | *Avr1b* | *Phytophthora* sojae | *Oomycete* |
| *Arabidopsis thaliana* | *Rpp1* | *Atr1* | *Hyaloperonospora parasitica* | *Oomycete* |
| Tomato | *Cf2,* | *Avr2,* | *Cladosporium fulvum* | *Fungus* |
| | *Cf4,* | *Avr4,* | | |
| | *Cf9* | *Avr9* | | |
| Tomato | *Pto* | *AvrPto* | *Pseudomonas syringae pv. tomato* | *Bacteria* |

Typically, the term "functional (R-) gene" is used herein to refer to a gene that is transcribable, i.e. the gene product is expressed (and provides a product that is capable of interacting with at least one Phytophthora elicitor). Suitable genes are provided in Table 1.

There are multiple ways in which the recombinant nucleic acid can be transferred to a plant cell, for example Agrobacterium mediated transformation. However, besides by Agrobacterium infection, there are other means to effectively deliver DNA to recipient plant cells when one wishes to practice the invention. Suitable methods for delivering DNA to plant cells are believed to include virtually any method by which DNA can be introduced into a cell, such as by direct delivery of DNA such as by PEG-mediated transformation of protoplasts (Omirulleh et al., 1993), by desiccation/inhibition-mediated DNA uptake (Potrykus et al., Mol. Gen. Genet., 199:183-188, 1985), by electroporation (U.S. Pat. No. 5,384,253), by agitation with silicon carbide fibers (Kaeppler et al., 1990; U.S. Pat. No. 5,302,523; and U.S. Pat. No. 5,464,765), and by acceleration of DNA coated particles (U.S. Pat. No. 5,550,318; U.S. Pat. No. 5,538,877; and U.S. Pat. No. 5,538,880). Through the application of techniques such as these, certain cells from virtually any plant species may be stably transformed, and these cells developed into transgenic plants.

In case Agrobacterium mediated transfer is used, it is preferred to use a substantially virulent Agrobacterium such as A. *tumefaciens,* as exemplified by strain A281 or a strain derived thereof or another virulent strain available in the art. These Agrobacterium strains carry a DNA region originating from the virulence region of the Ti plasmid pTiBo542 containing the *virB, virC* and *virG* genes. The virulence (vir) gene products of *A. tumefaciens* coordinate the processing of the T-DNA and its transfer into plant cells. Vir gene expression is controlled by *virA* and *virG,* whereby *virA* upon perception of an inducing signal activates *virG* by phosphorylation. *VirG,* in turn, induces the expression of *virB,C,D,E.* These genes code for proteins involved in the transfer of DNA. The enhanced virulence of pTiBo542 is thought to be caused by a hypervirulent *virG* gene on this Ti plasmid (Chen et al. Mol. Gen. Genet 230: 302-309, 1991).

In Microprojectile Bombardment, particles may be coated with nucleic acids and delivered into cells by a propelling force. Exemplary particles include those comprised of tungsten, gold, platinum, and the like. It is contemplated that in some instances DNA precipitation onto metal particles would not be necessary for DNA delivery to a recipient cell using microprojectile bombardment. However, it is contemplated that particles may contain DNA rather than be coated with DNA. For microprojectile bombardment transformation in accordance with the current invention, both physical and biological parameters may be optimized. Physical factors are those that involve manipulating the DNA/microprojectile precipitate or those that affect the flight and velocity of either the macro- or microprojectiles. Biological factors include all steps involved in manipulation of cells before and immediately after bombardment, such as the osmotic adjustment of target cells to help alleviate the trauma associated with bombardment, the orientation of an target tissue relative to the particle trajectory, and also the nature of the transforming DNA, such as linearized DNA or intact supercoiled plasmids. It is believed that pre-bombardment manipulations are especially important for successful transformation. In the preferred embodiment target tissue will be transformed with linearized DNA not containing backbone plasmid DNA sequences.

Accordingly, it is contemplated that one may wish to adjust various bombardment parameters in small-scale studies to fully optimize the conditions. One may particularly wish to adjust physical parameters such as DNA concentration, gap distance, flight distance, tissue distance, and helium pressure. It is further contemplated that the grade of helium may effect transformation efficiency. One also may optimize the trauma reduction factors by modifying conditions which influence the physiological state of the recipient cells and which may therefore influence transformation and integration efficiencies. For example, the osmotic state, tissue hydration and the subculture stage or cell cycle of the recipient cells may be adjusted for optimum transformation.

The transforming DNA used for microprojectile transformation may contain an expression cassette comprising generally of a cDNA, gene or genes which one desires to introduce into the cells, and still further, may include a promoter and 3' region operatively linked to the exogenous gene. The optimized gene construct is described in present invention. The DNA segment may additionally include a second, third, fourth, fifth, sixth, or any additional number of exogenous genes capable of being placed on a single DNA molecule and transformed into a recipient cell.

The recipient plant cells for transformation with the recombinant nucleic acid in a method according to the invention may be from potentially any transformable monocot or dicot plant. Preferred monocot plant cells for use with the invention are from rice, wheat, barley, oats, rye, millet, sorghum, sugarcane, turfgrass and maize. Preferred dicot plant cells for use with the invention include cotton, tomato, citrus, tobacco, soybean and particularly potato and cassava.

After transfer of the recombinant nucleic acid a plant is produced from said genetically modified plant cell. This comprises regeneration methods that are well known in the art and need no further elaborate discussion.

The resulting plant may be subjected at any age to at least one strain or elicitor of Phytophthora. In principle a resulting plant with only one leaf can already be subjected to at least one strain or elicitor of Phytophthora. Moreover, plants can be tested in vitro as well as in vivo. A preferred embodiment, involves testing two weeks after potting from an in vitro culture.

Plant pathogenic oomycetes accomplish parasitic colonization of plants by modulating host cell defense through an array of disease effector proteins. The biology of effectors is poorly understood but tremendous progress has been made in recent years. Two classes of effectors target distinct sites in the host plant: apoplastic effectors are secreted into the plant extracellular space, and cytoplasmic effectors are translocated inside the plant cell, where they target different subcellular compartments.

The term elicitor is used herein to refer to a pathogen molecule that triggers a defense response resulting in enhanced resistance to the invading pathogen.

Elicitors of Phytophthora that may be used in a method of the invention can for example be identified as described by Kamoun (Annu. Rev. Phytopathology 44: 41-60, 2006):
"Biochemical, genetic, and bioinformatic strategies, often in combinations, have been applied to the identification of effector genes from oomycetes. Traditionally, effectors have been identified by biochemical purification and genetic analyses. With the advent of genomics, novel strategies have emerged. The implementation of functional genomics pipelines to identify effectors from sequence data sets has proved particularly successful. A typical pipeline consists of two major steps: use of data mining tools to identify candidate genes that fulfil a list of specific criteria, followed by analysis and validation of these candidate genes by functional assays, such as expression in planta and evaluation for effector-like activities.
A critical milestone in the identification of oomycete effector genes was the validation of the concept that effector proteins must be secreted in order to reach their cellular targets at the intercellular interface between the plant and pathogen or inside the host cell. Identification of candidate secreted proteins was facilitated by the fact that in oomycetes as in other eukaryotes, most secreted proteins are exported through the general secretory pathway via short, N-terminal, amino-acid sequences known as signal peptides. Signal peptides are highly degenerate and cannot be identified using DNA hybridization or PCR-based techniques. Nonetheless, computational tools, particularly the SignalP program that was developed using machine learning methods (Nielson et al, Protein Eng. 12: 3-9, 1999), can assign signal peptide prediction scores and cleavage sites to unknown amino acid sequences with a high degree of accuracy. Therefore, with the accumulation of oomycete cDNA and genome sequences, lists of candidate secreted proteins could be readily generated using bioinformatics tools. For instance, Torto et al. (Genome Res. 13: 1675-1685, 2003) developed PexFinder (with Pex standing for *Phytophthora* extracellular protein), an algorithm based on SignalP v2.0 (Nielson et al, Protein Eng. 12: 3-9, 1999) to identify proteins containing putative signal peptides from expressed sequence tags (ESTs). PexFinder was then applied to ESTs in *P. infestans.* Proteomic identification of secreted proteins collected from culture filtrates of *P. infestans* matched PexFinder predictions, convincingly validating the algorithm performance.
Computational signal peptide predictions help to identify secreted proteins with no significant matches to known sequences. Annotation of cDNA and gene sequences is routinely done by similarity searches to sequences in public databases. However, this approach is limited in that it identifies only proteins with known functions and thus a large number of proteins with unknown functions are ignored. Computational predictions single out unknown secreted proteins that might otherwise be ignored. This results in a manageable list of candidates that can be further processed by sequence annotation and functional analyses.
Building up an annotated collection of computationally predicted candidate proteins that feed into a functional analysis pipeline is by no means a one-directional process. Rather, criteria for annotating and prioritizing candidate genes are continuously refined as new findings emerge and novel sequence-to-function links are established. One illustrative example is the finding that all known avirulence proteins of oomycetes carry a conserved motif (RXLR) closely following a signal peptide (see below for more details) (Rehmany et al.,Plant Cell 17: 1839-1850). Straightforward sequence pattern search tools can thus be used to discover an additional number of candidate effectors of the RXLR family from several *Phytophthora* species.
Identification of genes under diversifying selection (especially when coupled with selection criteria such as presence of secretory signals and in planta expression) can also help to select candidate effector genes (Liu et al., Mol. Biol. Evol. 22: 659-672). Based on the "arms race" model, adaptation and counteradaptation between pathogen and host is likely to drive their antagonistic coevolution and generate the evolutionary forces that shape effectors and their host targets. For instance, diversifying selection (also known as positive selection) can be an indicator of functionally important loci that contribute to the fitness of the organism. Indeed, some oomycete effector genes, e.g., *ATR13* and *scr74,* exhibit extreme levels of amino acid polymorphism with evidence of diversifying selection. In the future, evolutionary analyses will have an even larger role in the identification of effector genes as genome sequences from additional oomycete species become available."

Using the above described strategy Kamoun and co-workers have currently identified about 50 families of genes containing the RXLR motif in *P*. *infestans* This subset of Phytophthora extracellular proteins harbouring the RXLR motif are called RD proteins and this set is constantly being updated and enlarged as criteria for annotating and prioritizing candidate genes are continuously refined. Examples of RD families that harbour functional Avr genes are RD6 (IPI-O) and RD7 (Avr3a).

Although multiple effectors of oomycete pathogen have been cloned and are characterised or are in the process of being characterised the provided examples of effectors and/or elicitors are by no means intended to be limiting. A milestone in the identification of oomycete effector genes was the validation of the concept that effector proteins must be secreted in order to reach their cellular targets at the intracellular interface between the plant and pathogen or inside the host cell. Torto et al. developed PexFinder (with Pex standing for Phytophthora extracellular protein), an algorithm based on SignalP to identify proteins containing putative signal peptides from expressed sequence tags (EST). In the meantime, it has become clear that all known avirulence proteins of oomyctes carry a conserved motif (RXLR) closely following a signal peptide. A corresponding sequence pattern tool is now for example used to identify additional candidate effectors of the RXLR family from several Phytophthora species. It is clear that these newly identified elicitors are suitable applicable in a method of the invention, especially when the counterpart (i.e. the corresponding R-gene) is also identified.

It is clear to the skilled person that also multiple (for example, at least 2 or at least three) elicitors of Phytophthora may be used in a method of the invention. This is for example very useful when multiple R-genes have been introduced, especially when these R-genes provide a partial overlapping spectrum of resistance and/or interact with different effector molecules. In yet another preferred embodiment, a Phytophthora isolate is used.

The selection of a particular elicitor is in particular dependent on the used functional R-gene. It is clear to the skilled person that the used selective, functional R-gene is capable of providing an HR in response to said elicitor. Although an HR is considered to be a local response and the plant in principle does not die as a result of said HR, it is preferred that the presence or absence of a reaction to a particular elicitor is tested on a so-called tester-plant or via a detached leaf of a plant.

Moreover, if a (preferably defined or well characterised) Phytophthora isolate is used instead of an isolated elicitor, the presence or absence of a reaction can also be the presence or absence of fungal growth (i.e. an Phytophthora assay). The used Phytophthora isolate of course produces at least the elicitor that is capable of reaction with the used functional R-gene.

A main advantage of a method according to the invention is that the obtained plants need not be grown in a selective medium, e.g. a medium comprising a herbicide, antibiotic, amino acid analog or the like. Moreover, a method of the invention does not comprise the use of additional equipment which is for example needed in case a GFP or GUS marker is used.

As described above also functional R-genes of plants other than potato can be used in a method of the invention, for example the Rps1b gene of soybean plants or a Cf gene from tomato.

The invention therefore also provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of for example Cladosporium and determining the presence or absence of a reaction to said at least one elicitor. The used plant cell is in this particular example a Lycopersicon cell.

A method of the invention optionally further comprises steps like (i) : testing of copy number and/or (ii) testing for backbone vector sequences and/or (iii) testing for border free transformants.

These kinds of analysis are preferably performed on DNA/RNA level. This includes the isolation of nucleic acid from plantlets or a pool of plantlets according to standard methodologies (Sambrook et al., 1989). The invention provides, for example, the use of a nucleic acid detection method for determining whether a transformed plant cell or progeny thereof comprises backbone seqeunces. The nucleic acid may be genomic DNA, RNA or mRNA. Also rearrangements can be detected. The invention provides the use of a mRNA detection method for determining whether a nucleic acid construct in a transformed plant cell or progeny thereof is sufficiently integrated into a plant genome to be transcribed into a mRNA construct. The specific nucleic acid of interest, being part of the transgene is identified in the sample directly (DNA) or indirectly (RNA) using amplification. Next, the identified product is detected. In certain applications, the detection may be performed by visual means (e.g., ethidium bromide staining of a gel). Alternatively, the detection may involve indirect identification of the product via chemiluminescence, radioactive scintigraphy of radiolabel or fluorescent label or even via a system using electrical or thermal impulse signals. A variety of different assays are contemplated in the screening of transgenic plants created using the methods of the current invention. These techniques can be used to detect for both the presence of particular genes as well as rearrangements that may have occurred in the gene construct. The techniques include but are not limited to, polymerase chain reaction (PCR), fluorescent in situ hybridization (FISH), direct DNA sequencing, PFGE analysis, Southern or Northern blotting, single-stranded conformation analysis (SSCA), RNAse protection assay, allele-specific oligonucleotide (ASO), dot blot analysis, denaturing gradient gel electrophoresis, restriction fragment length polymorphism (RFLP) and PCR-SSCP or chip-based DNA technologies. The invention provides the use of a nucleic acid detection method for determining whether a transformed plant cell or progeny thereof is transformed with a recombinant nucleic acid comprising a T-DNA construct or a functionally equivalent nucleic acid construct allowing integration into a genome of a plant cell. Furthermore, the invention provides the use of a nucleic acid detection method for determining whether a transformed plant cell or progeny thereof is transformed with a recombinant nucleic acid comprising testing said cell or said progeny for the presence or absence of undesired vector material such as vector backbone sequences. For example, a method according to the invention can be used to check whether a transformed plant cell is essentially free of ancillary unwanted nucleic acids. The transformed plantlet, identified by nucleic acid detection methods, will then be allowed to mature into plants. After the regenerating plants have reached the stage of shoot and root development, they may be transferred to a greenhouse for further growth and testing.

Control of the copy number of introduced transgenes and an efficiently production of low-copy transformants can, especially in case for transformation via gun bombardment, be achieved by end-modification of nucleic acid segments by dephosphorylation or by blunting the ends of the nucleic acid segments (WO99/32642).

For production of transformants free of vector DNA sequences it will be desirable to deliver DNA to cells that does not contain DNA sequences necessary for maintenance of the plasmid vector in the bacterial host, e.g., *E*. *coli,* such as antibiotic resistance genes, including but not limited to ampicillin, kanamycin, and tetracycline resistance, and prokaryotic origins of DNA replication. In such case, a DNA fragment containing the transforming DNA may be purified prior to transformation. Purification can be achieved by for example gel electrophoresis on an agarose gel, followed by recovery of a DNA fragment from the agarose gel.

By for example testing whether vector or vector backbone or border sequences are present, the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor., further comprising determining the presence or absence of non-Solanum nucleic acid sequences. In a preferred embodiment, said non-Solanum nucleic acid sequences are non-Solanum T-DNA border sequences, i.e. in a preferred embodiment one tests for the presence or absence of (T-DNA) border sequences, especially in the cases in which at least one non-Solanum T-DNA border sequence is used.

In a preferred embodiment, the obtained plant essentially only comprises nucleic acid sequences that are obtained from a plant and even more preferred from Solanum. Therefore the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said recombinant nucleic acid essentially consists of Solanum nucleic acid sequences, i.e. the to be transferred nucleic acid sequence is essentially a Solanum nucleic acid sequence. If for example the Agrobacterium/binary vector method is selected as the transfer method, the sequences present on the T-DNA (i.e. the part that will be transferred) is essentially a Solanaceae nucleic acid sequence. The border sequences as well as the backbone of the used binary vector may comprise non-Solanaceae nucleic acid sequences. In a preferred embodiment, transformants of Solanaceae will be selected which do not contain these border sequences and backbone sequences. This results in the production of a so-called cisgenic plant, i.e. a plant that has been obtained through genetic modification by using species-own sequences or sequences from crossable species. For example a commercial potato variety that has been provided with a nucleic acid sequence isolated from a wildtype potato species or a different potato variety or from a crossable species. In a preferred embodiment, the cloning of the used sequences on the T-DNA is performed such that only Solanum sequences are present on the T-DNA, for example by using linkers that are derived from or designed based on a Solanumgenome to separate different coding sequences on the T-DNA. It is also possible to use a (modified) multiple cloning site that after cloning only results in the presence of a nucleotide sequence which is already present in the genomic background of the used plant cell. The term "essentially" is used herein to refer to the fact that although all sequences are in at least one (preferably non-genetically modified) plant (preferably a Solanum) naturally present, the genetic surroundings or setting may be somewhat different.

Preferably, the used functional R-gene and/or the used nucleic acid of interest are obtained/isolated from a Solanaceae including its own 5' (promoter) and 3' (terminator) sequences, i.e. the used functional R-gene and/or the used nucleic acid of interest are preferably under regulation of their natural/native promoter and terminator region.

In a preferred embodiment, the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said nucleic acid of interest and said functional R-gene are the same. Such a method does not rely on a selective marker gene and does thus not need selection through treatment with a selective agent. In this embodiment, the functional R-gene has a dual function, i.e. said R-gene is used to determine which plants have been provided with a recombinant nucleic acid and is also used to confer (at least partial) resistance against a pathogen.

In yet another embodiment, the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said nucleic acid of interest is a functional R-gene. Examples of suitable R-genes are provided in Table 1. Such a method is actually a method to provide a plant with at least partial resistance against a pathogen. In this embodiment, the functional R-gene used to determine the presence or absence of a recombinant nucleic acid may also be used to confer resistance. In the latter case, the produced plant comprises at least two functional R-genes. It is clear to the skilled person that a plant can also be provided with three or four or five or six (preferably different) functional R-genes. Preferably the used R-genes are chosen such that they provide complementary protection against a pathogen. Even more preferably the obtained protection is a broad scope protection resulting in a (durable) resistant phenotype.

As described above multiple reactions can be used to determine whether a reaction to an elicitor is present. In a preferred embodiment, said reaction is a hypersensitive response (HR). Whether or not an HR is induced is for example tested with an agroinfection or agroinfiltration. Both techniques are outlined in more detail in the experimental part herein. If a representative Phytophthora strain is used instead of an (isolated) elicitor, the skilled person preferably uses inoculation.

The transferred nucleic acid may be present in the plant cell as an extra-chromosmally (episomal) replicating molecule but more preferably a method according to the invention results in integration of the recombinant nucleic acid into the genome of said plant.

In a preferred the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said nucleic acid of interest and/or said functional R-gene is/are cDNA sequences. In another preferred embodiment, said nucleic acid of interest is an inverted (repeat) sequence. An example of an inverted repeat sequence is an inverted repeat sequence of GBSS.

In yet another preferred embodiment, the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said nucleic acid of interest and said functional R-gene are genomic sequences, more preferably genomic Solanaceae sequences. As a result the genes are present within their normal/native exon/intron context. In a most preferred embodiment, said nucleic acid of interest and said functional R-gene are not only genomic sequences (more preferably genomic Solanaceae sequences) but are also controlled by their native/natural 5' and 3' sequences, i.e. by a promoter and a terminator sequence as present in the original/natural/native setting.

As already described, a method of the invention can be performed by using different functional R-gene/elicitor (or Phytophthora) combinations. In one of the embodiments, the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said functional R-gene is a gene encoding R3a or a gene encoding a functional fragment thereof or a gene encoding a derivative thereof. In an even more preferred embodiment, said elicitor is RD6 (Avr3a). The sequences of R3a and RD6 are published by Huang et al., 2005 and Armstrong et al., 2005.

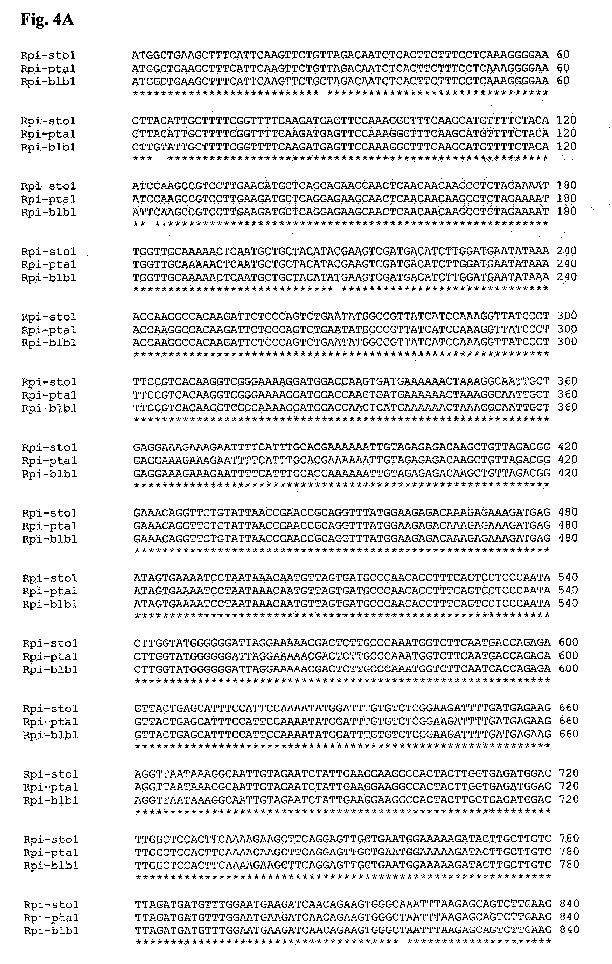

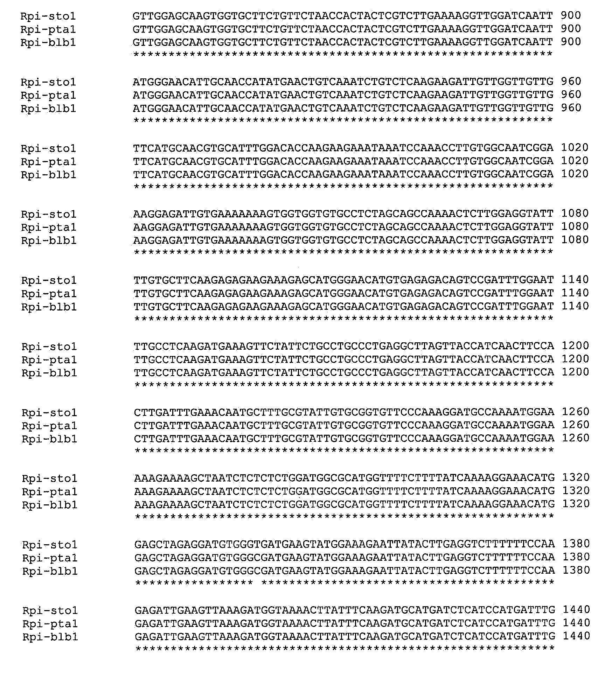

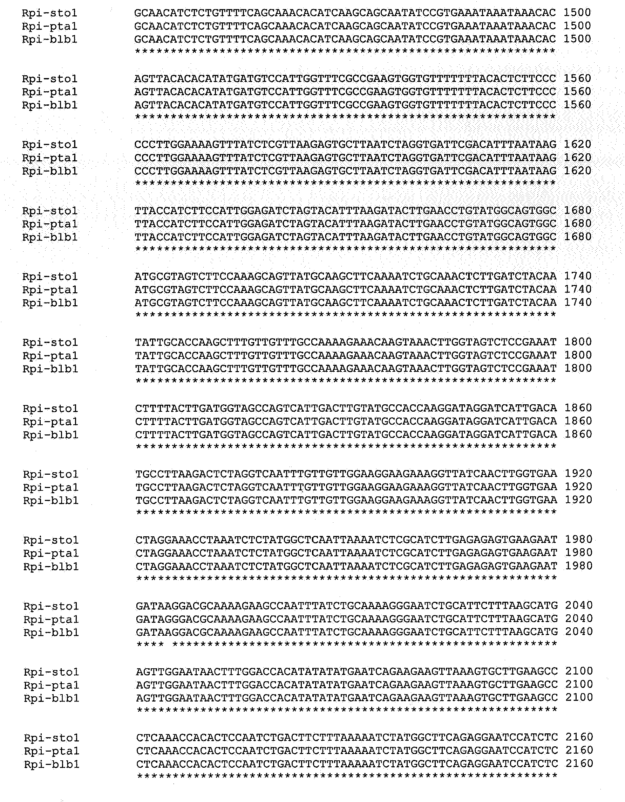

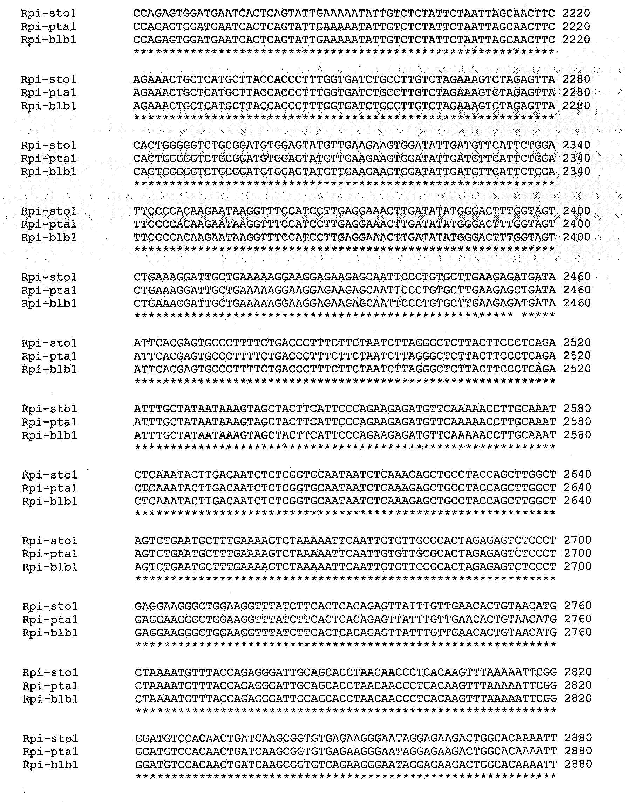

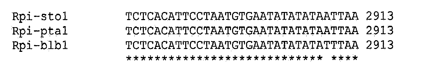

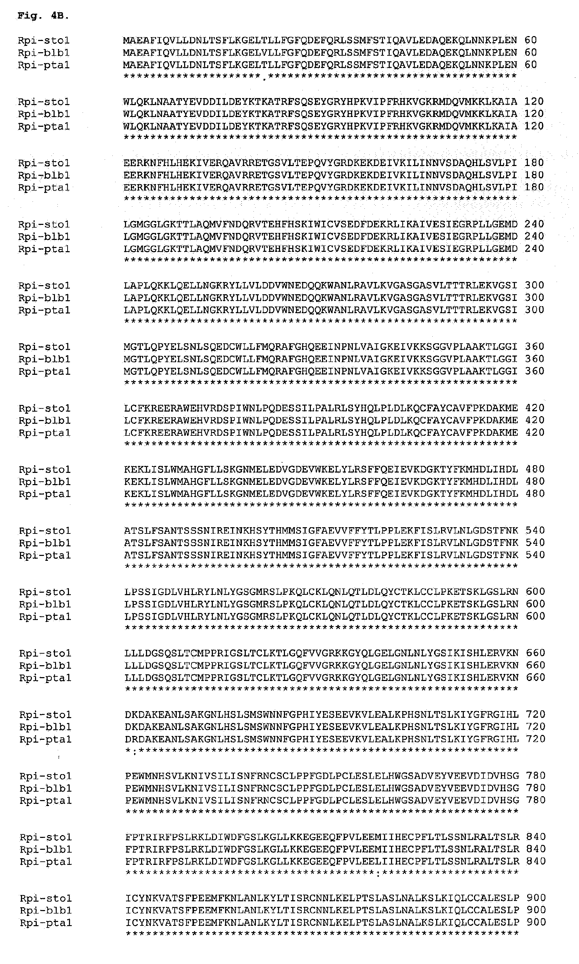

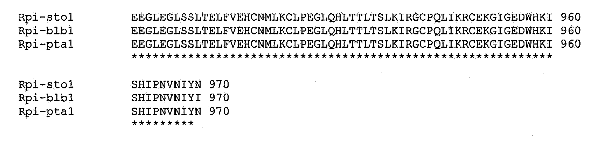

In another embodiment, the functional R-gene is Rpi-blb-1 or Rpi-sto1 or Rpi-pta1 and the used elicitor is RD7 (IPI-0) and hence the invention also provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said functional R-gene is a gene encoding Rpi-blb-1 or Rpi-sto1 or Rpi-pta1 or a gene encoding a functional fragment thereof or a gene encoding a derivative thereof. In an even more preferred embodiment, said elicitor is RD7 (IPI-0). As disclosed in more detail in one of our co-pending applications, Rpi-sto1 and Rpi-pta1 are homologues of Rpi-blb-1 and can interact with RD7 (IPI-0). Figure 4 shows an alignment between Rpi-blb-1 and Rpi-sto1 and Rpi-pta1.

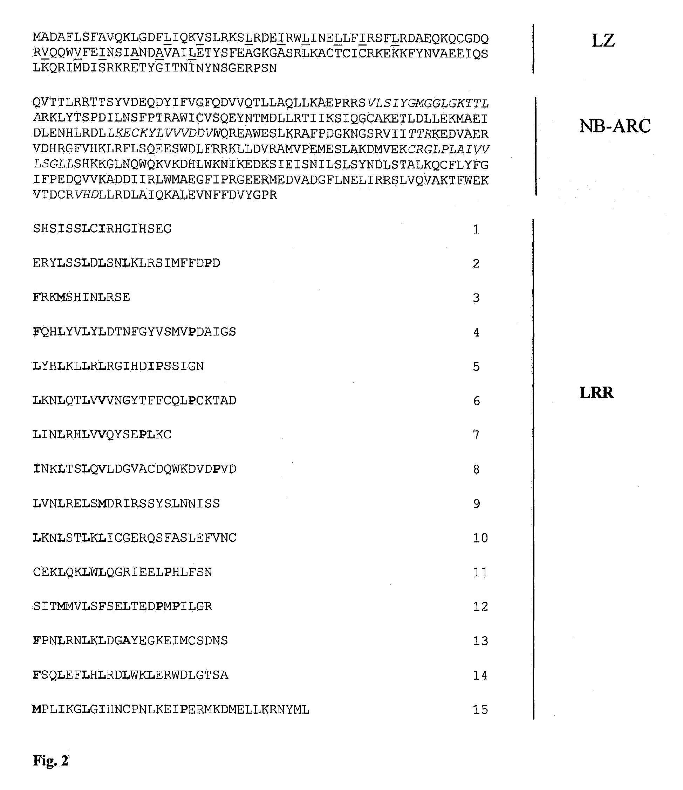

In yet another embodiment, the used functional R-gene is a gene encoding blb-3 as depicted in Figure 2 and hence in another embodiment, the invention provides a method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor of Phytophthora and determining the presence or absence of a reaction to said at least one elicitor, wherein said functional R-gene is a gene encoding blb3 or a gene encoding a functional fragment thereof or a gene encoding a derivative thereof. In yet another embodiment, said functional R-gene is a gene encoding blb-3 as depicted in Figure 2.

Blb3 is a LZ-NBS-LRR type of R-gene and as described in a co-pending application, provides resistance to a range of P.infestans isolates.

A functional fragment of a blb-3 nucleic acid sequence is a truncated (n-terminal, C-terminal, internally or a combination thereof) sequence of the complete blb-3 nucleic acid sequence. The truncated sequence has a comparable function and/or activity if compared to the full-length sequence, i.e. a functional fragment is capable of providing a member of the Solanaceae family with race non-specific (at least partial) resistance against an oomycete pathogen. Such a truncated sequence is for example tested in the herein already outlined ATTA methodology or by an effector screen with the matching effector (elicitor).

Examples of a derivative are allelic variants of blb-3.

In yet another embodiment, the invention provides use of a functional Phytophthora R-gene as a selection marker in plant transformation. Preferably, said R-gene is a gene encoding blb3 or a gene encoding a functional fragment thereof or a gene encoding a derivative thereof or a gene encoding blb-3 as depicted in Figure 2.

The invention further provides a plant obtainable by a method of the invention. In a preferred embodiment, said plant is a commercially interesting potato variety such as Bintje, Desiree or Premiere, Spunta, Nicola, Favorit, Russet Burbank, Aveka or Lady Rosetta. The identity of plants of the invention is for example determined by performing a PCR to determine whether a functional R-gene is present and whether selection markers such as kanamycin are absent. The R-gene is preferably present in its non-natural background, e.g. Rpi-blb1 or 3 in a non S. bulbocastanum background or R3 in a non S. demissum background or Rpi-sto1 in a non S. stoloniferum background or Rpi-pta1 in a non S. patita background. The same is true for the used nucleic acid of interest e.g. if a gene of interest is isolated from S. microdontum, said gene of interest is preferably introduced in a non-microdontum background. In other words, the to be introduced traits are preferably introduced in a variety different from the species from which they were cloned or isolated.

In yet another preferred embodiment, the herein used R-gene and/or nucleic acid of interest is foreign to the plant cell. The term "foreign" is herein used to describe the situation in which the R-gene and/or nucleic acid of interest is heterologous with respect to the host cell (i.e. derived from a cell or organism with a different genomic/genetic background if compared to the cell used for transferring) or the R-gene and/or nucleic acid of interest is homologous with respect to the used host cell but located in a different genomic environment than the naturally occurring counterpart of said R-gene and/or nucleic acid of interest (for example surrounded by different genes if compared to the natural gene layout).

As described above, the used R-gene and the used nucleic acid of interest are preferably controlled by their own 5' (promoter) and 3' (terminator) nucleic acid sequences. The presence of such 5' (promoter) and 3' (terminator) nucleic acid sequences may also be determined by for example PCR or sequence analysis. As is also described above, the used R-gene and the used nucleic acid of interest are genomic sequences, i.e. optionally intron and exon sequences are present. Again, the presence of such intron and/or exon sequences can also be determined by using PCR or sequence analysis.

Moreover, the plants of the invention are preferably, non-chimeric and/or marker-free and/or comprise no backbone sequences and/or comprise no border sequences. A plant obtained by a method of the invention does not need any recombination event (to for example excise a selection marker) and does also not need any crossing event (to for example cross out a selection marker), i.e. a plant obtainable by a method of the invention does not need any additional recombination events or sexual cycle. However, an obtained plant can be used a crossing parent for new varieties. The thus obtained plant have a genomic background which is essentially identical to the genetic background of the plant cell used for transformation and said obtained plant does also not contain any traces of recombination events.

As described a method of the invention may result in a so-called cisgene plant, preferably a potato cisgene plant. Such a cisgene (potato) plant may also be obtained by an alternative method (for example a marker-free method, i.e a method devoid of a selection marker such as an R-gene). Hence the invention also provides a method for obtaining a plant that has been provided with a nucleic acid of interest comprising providing a recombinant nucleic acid comprising said nucleic acid of interest transferring said recombinant nucleic acid to a plant cell producing a plant from said cell and determining the presence or absence of said nucleic acid of interest, wherein said nucleic acid of interest (or the to be transferred DNA, for example T-DNA) is essentially a plant nucleic acid.

The to be transferred nucleic acid sequence is free of nucleic acid sequences encoding a selective marker or a reporter gene. Moreover, said method does not need selection through treatment with a selective agent or at least one elicitor. Instead, selection for the desired transformant is achieved by testing for the presence of the nucleic acid of interest, for example by using commonly known nucleic acid techniques, such as detection by Polymerase Chain Reaction or by hybridisation with complementary sequences in routine Southern blotting experiments. It will also be apparent to those skilled in the art that the presence of a desired heterologous gene or gene fragment can be assayed by monitoring the presence or the absence or change in amount of the expression product of the gene. For example, an expressed protein allowing detection by ELISA (enzyme-linked immunosorbent assay) the presence of such a protein can be assayed by ELISA. Furthermore, the method provided herein may involve a bioassay or a chemical analytical method such as gas chromatography/mass spectometry (GC/MS).

Details of such a transformation and screening method is described in our co-pending application WO03/010319.

The phrase "said nucleic acid of interest is essentially a plant nucleic acid" is herein defined to refer to sequences obtained/derived/isolated from a plant (preferably a Solanum) i.e. the to be transferred nucleic acid sequence is essentially a Solanum nucleic acid sequence. If for example the Agrobacterium/binary vector method is selected as the transfer method, the sequences present on the T-DNA (i.e. the part that will be transferred) is essentially a Solanaceae nucleic acid sequence. The border sequences as well as the backbone of the used binary vector may comprise non-Solanaceae nucleic acid sequences. In a preferred embodiment, the used plant cell is a Solanaceae. This results in the production of a so-called cis(genic) plant, i.e. a plant that has been obtained through genetic modification by using species-own sequences or sequences from crossable species. For example a commercial potato variety that has been provided with a nucleic acid sequence isolated from a wildtype potato variety or from a crossable species. In a preferred embodiment, the cloning of the used sequences on the T-DNA is performed such that only Solanum sequences are present on the T-DNA, for example by using linkers that are derived from a Solanum to separate different coding sequences on the T-DNA. It is also possible to use a (modified) multiple cloning site that after cloning only results in the presence of a nucleotide sequence which is already present in the genomic background of the used plant cell. The term "essentially" is used herein to refer to the fact that although all sequences are in at least one (preferably non-genetically modified) plant (preferably a Solanum) naturally present, the genetic surroundings or setting may be somewhat different.

In a preferred embodiment, the invention provides a method for obtaining a plant that has been provided with a nucleic acid of interest comprising providing a recombinant nucleic acid comprising said nucleic acid of interest transferring said recombinant nucleic acid to a plant cell producing a plant from said cell and determining the presence or absence of said nucleic acid of interest, wherein said nucleic acid of interest is essentially a plant nucleic acid, wherein said plant nucleic acid is a Solanaceae nucleic acid. Even more preferred the used plant cell is a Solanceae cell.

In yet another preferred embodiment, the invention provides a method for obtaining a plant that has been provided with a nucleic acid of interest comprising providing a recombinant nucleic acid comprising said nucleic acid of interest transferring said recombinant nucleic acid to a plant cell producing a plant from said cell and determining the presence or absence of said nucleic acid of interest, wherein said nucleic acid of interest is essentially a plant nucleic acid, wherein said nucleic acid of interest is a cDNA sequence or wherein said nucleic acid of interest is an inverted (repeat) sequence or wherein said nucleic acid of interest is a genomic sequences.

In a more preferred embodiment, said plant nucleic acid is an open reading frame that is under control of natural/native 5' (promoter) and 3' (terminator) nucleic acid sequences and in an even more preferred embodiment, said nucleic acid sequences is a genomic sequence. Use of this kind of sequences in combination with a Solanaceae plant cell results in the production of a so-called cisgenic plant, i.e. a plant that has been genetically modified by using species own sequences.

A further preferred embodiment is a method wherein said nucleic acid of interest is an R-gene. This way the invention provides for example cisgenic potato plants which are at least partial resistant against a pathogen (preferably P. infestans). Such plants are considered to be more easily excepted by ethic committees as well as by consumer groups and such plants at the same time reduce the use of for example fungicides.

The here-described method may further be complemented by determining the presence or absence of non-Solanum nucleic acid sequences. This confirms the absence of any non-plant nucleic acid sequences. Preferably said non-Solanum nucleic acid sequences are non-Solanum T-DNA border sequences.

The nucleic acid of interest may be present in the plant cell as an extra-chromosmally (episomal) replicating molecule but more preferably a method according to the invention results in integration of the recombinant nucleic acid (the to be transferred nucleic acid sequence) into the genome of said plant.

The invention further provides a plant obtainable by the method for obtaining a plant that has been provided with a nucleic acid of interest comprising providing a recombinant nucleic acid comprising said nucleic acid of interest transferring said recombinant nucleic acid to a plant cell producing a plant from said cell and determining the presence or absence of said nucleic acid of interest, wherein said nucleic acid of interest is essentially a plant nucleic acid.

The invention also provides a genetically modified plant that has been provided with a cisgene, i.e. a gene obtained/derived/isolated from a same species but different variety. Preferably, said cisgene is under control of native 5' (promoter) and 3' (terminator) sequences and even more preferably said cis gene is a genomic sequence. Said genomic sequence can contain intron and exon sequences. Preferably, the invention provides a plant which is free from (non-Solanum) T-DNA border sequences.

The described methods can be applied to multiple types of plants. Preferred monocot plants (cells for use with the invention) are from rice, wheat, barley, oats, rye, millet, sorghum, sugarcane, turfgrass and maize. Preferred dicot plant (cells for use with the invention) include cotton, tomato, citrus, tobacco, soybean and particularly potato and cassava.

The invention will be explained in more detail in the following, nonlimiting examples.

### Experimental part

### Materials and methods

### Agroinfection

Recombinant A. tumefaciens GV3101 strains carrying candidate effectors (obtained from OSU) in pGR106 were used to screen for a response in *Solanum.* The pGR106-CRN2 and the pGR106 empty vector (Jones *et al*., 1999; Takken *et al.,* 2000; Torto *et al.,* 2003) were used as a positive and negative control respectively. Cultures were grown for 2 days at 28°C on solid agar LB medium supplemented with antibiotics. Excess of bacteria was inoculated by piercing the leaf at both sides of the mid-vein. Local and systemic symptoms were visually scored every 2-4 days. For mature plant inoculations, usually three leaves from three to four week old plants was used, and the leaf age was rotated for replicates of the various treatments.

### Application of agroinfection in Solanum

Resistance to PVX is known to occur in *Solanum* and would interfere with large-scale screenings with the binary PVX vector. To determine the extent to which the assay is applicable to the diverse *Solanum* germplasm, we inoculated plants corresponding to 80 *Solanum* clones from 31 species with A. *tumefaciens* carrying pGR106 and pGR106-CRN2 (Vleeshouwers *et al.,* 2006). Treatment with the pGR106-CRN2 strain consistently caused necrosis around the inoculation sites on 50 plant clones (63%) whereas the empty vector strain caused no symptoms. We concluded that these plants are suitable for PVX agroinfection assays. The remainder of the *Solanum* clones tested were not suitable for the assay. Responses to both the positive and negative controls occurred in 20 clones and were regarded as nonspecific reactions to PVX. Ten other clones showed no necrotic response to pGR106-CRN2 possibly because the gene insert was not expressed *in planta.* In summary, it appears that the PVX assay is suitable for about 80% of the species examined and 60% of the clones. Therefore, before each screening with a large set of candidate effectors from *P*. *infestans,* a pre-screening with pGR106-CRN2 and the pGR106 empty vector is performed.

### Agroinfiltration

Coinfiltrations of Agrobacterium strains carrying the R gene and the AVR gene were performed in *N. benthamiana*. Recombinant A. tumefaciens cultures were grown in LB medium (10 gram bacteriological peptone, 10 gram NaCl and 5 gram yeast extract in 1 liter MQ water) supplemented with 50 mg/L Rifamplicin and 50 mg/L Kanamycin for the LBA4404 constructs. The COR308 was supplemented with 5 mg/ L tetracycline and 50 mg/L kanamycin (the empty COR308 was only grown on tetracycline). After one or two days a calculated amount of culture (according to OD 0.5 at 600 nm) was transferred to YEB medium (5 gram beef extract, 5 gram bacteriological peptone, 5 gram sucrose, 1 gram yeast extract, 2ml 1 M MgSO₄ in 1 liter MQ water) supplemented with Kanamycin for all strains, but for COR308 empty vector tetracycline was used. After 1 day overnight cells were centrifuged at 3500 rpm and resuspended in MMA medium (20 gram sucrose, 5 gram MS salts and 1.95 gram MES) supplemented with 1 ml 200 mM acetosyringone to an final OD of 0.5. Both constructs (R-gene and PEX) were mixed 1:1 before infiltration into 4-6 weeks old plants with a 3ml syringe. Symptom development was monitored after 5 to 6 days.

### Example 1.

### Selection of cisgene R3a transformants

### Construction of vector pBINmf::R3a

pBI121-derived binary vector pPGB-1S (Kuipers et al, 1995) was digested with enzymes PmeI and ClaI to remove the NptII gene. The ClaI sticky end was made blunt-ended by Klenow polymerase treatment, after which the vector DNA was circularized by blunt-end ligation using T4 DNA ligase. This resulted in vector pPGBmf (marker-free) containing in the T-DNA the potato GBSSI promoter followed by the NOS terminator as an 1140-bp HindIII/EcoRI fragment. Construct pPGBmf was digested with HindIII and EcoRI, resulting in two fragments of 1140 bp and 9681 bp. The 9681-bp fragment containing the inner LB (left border) sequence, LB, backbone vector DNA, RB and inner RB sequence was isolated from an agarose gel.
A double stranded oligo was made by annealing primers AWO1 (5'-AGCTTGGCGCGCCCGGGTTAATTAAG-3') and AWO2 (5'-AATTCTTAATTAACCCGGGCGCGCCA-3'). This sequence contains HindIII and EcoRI sticky ends and restriction sites for AscI, SmaI and PacI. The oligo was ligated to the 9681-bp HindIII/EcoRI fragment of pPGBmf, resulting in vector pBINmf (9707 bp).

Vector pBINmf::R3a was developed by the cloning of a PacI/AscI genomic DNA fragment of SH23-2 into PacI/AscI-digested pBINmf. SH23-2 is a subclone of the Bacterial Artificial Chromosome (BAC) SH23 (Huang et al. 2005). This BAC was partially digested with Sau3AI and the 7-10 kb fraction was ligated into the BamHI site of vector pBINPLUS.

The T-DNA in vector pBINmf::R3a contains a 9-kb fragment of the SH23-2 pBINPLUS subclone, in which the Coding Sequence (CDS) of gene R3a is situated. The 3849-bp CDS is (after transformation of the plant) regulated by the original promoter and terminator of R3a which are both present on SH23-2. The genomic fragment SH23-2 is isolated from the diploid potato clone SH83-92-488 (Huang et al. 2005).

The binary vector pBINmf::R3a was transformed into *Agrobacterium tumefaciens* strain COR308 by triparental mating, using helper plasmid pRK2013 (Figurski and Helinski, 1979).

### Transformation of potato and selection of transformants

Internodal cuttings *from in vitro* grown plants of potato cultivar 'Desiree' were used for transformation by *Agrobacterium tumefaciens* co-cultivation, according to the protocol described by Visser et al. (1991a).

Potato cultivar 'Desiree' was transformed with pBINmf::R3a in *A*. *tumefaciens* COR308. No selection was performed. After four weeks the first shoots were harvested and harvesting of shoots continued for about three months. No more than two regenerants per stem explant were harvested and shoots were allowed to grow on MS30 medium in a glass jars (diameter 10 cm) or plastic jars (diameter 15 cm). Each jar contained 5 cuttings. A 2 to 3 cm space was left between the cuttings and the inner wall of the jars.

### In vitro selection with avr3

*In vitro* plantlets were grown at 24°C and 16 h light/8 h dark. Plantlets with 3 to 4 fully developed leaves were used for *in vitro* inoculation. Depending on the potato genotype, after 2 to 4 weeks plantlet were ready for inoculation.

The 3 largest leaves of *each in vitro* plantlet were inoculated (1 spot per leaf) by pipetting 10 µl droplets of a zoospore suspension of 2.5 x 10⁴ spores/ml on the adaxial side. Inoculum preparation and inoculation were performed in sterilized conditions. Leaves touching the inner wall or the lid of the jars or the medium were excluded because we found these leaves to be more susceptible than others of the same plants. The jars with inoculated in *vitro* plantlets were incubated in the same climate chamber as the trays with inoculated detached leaves.
Non-transformed plantlet showing a susceptible interactions were scored as S (spreading lesion with massive sporulation) or class SQ (spreading lesion with no or little sporulation). Putative transformants showing a resistant interactions were scored as class R (no symptom or localized HR-like necrosis) or class RQ (trailing HR necrosis).
Putative transformants were transferred to new media and/or transferred to potting soil in the greenhouse.

### DNA analyses

DNA was isolated *from in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1, 2, 5, 6, 7 and 8; Wolters et al. 1998a), to study the presence of backbone vector DNA in the transformants.

Four µg of DNA of the transformants was digested with HindIII, fragments were separated by gel electrophoresis, and Southern blots were made. Blots were hybridized with the R3a probe, to check the number of T-DNA insertions.

### Border-free transformants

### PCR on the border sequences

The right border and left border sequences of the T-DNA are not derived from *Solanum* sp., but obtained from pBIN19 (pTiT37 borders). To analyze whether these border sequences are present or absent in the transformants PCRs were performed. For the analysis of the right border the following primers were used: primer RBcheck2 (5'-CCAATATATCCTGTCAAACA-3') and primer SHcis1 (5'-CATCATCATCCCAAGTACAA-3'). With these primers a fragment of 1221 bp was expected when DNA of vector pBINmf::R3a was used as template. For the analysis of the left border primer SHcis2 (5'-AAGGGCAAACATAACCATTC-3') was used in combination with primer LBcheck1 (5'-GGATATATTGTGGTGTAAAC-3'). With these primers a fragment of 1712 bp was expected when DNA of vector pBINmf::R3a was used as template.

### Universal genomic walking

To obtain T-DNA flanking sequences from the transformants the Universal GenomeWalker Kit (Clontech) was used. DNA of the transformants was digested with DraI, EcoRV, PvuII, ScaI and StuI. GenomeWalker Adaptors were ligated to the obtained fragments to create enzyme-specific libraries. To obtain RB-flanking DNA the first PCR was performed with adaptor primer AP1 (5'-GTAATACGACTCACTATAGGGC-3') and primer SHGW1 (5'-TAAGTTTAATCAGAAGTTGGGTAGGAA-3'). The nested PCR was performed with adaptor primer AP2 (5'-ACTATAGGGCACGCGTGGT-3') and primer SHGW2 (5'-GTTGGGTAGGAAGCCTGCTCTTGGAAA-3'). To obtain LB-flanking DNA the first PCR was performed with adaptor primer AP1 and primer SHGW3 (5'-GTATGTATGTGTAGTTAATGGGGTAGT-3'). The nested PCR was performed with adaptor primer AP2 and primer SHGW4 (5'-ACGGTTTCTAAATTAACGTAGCCAATA-3').

### Example 2

### Selection of cisgene R3a transformants

Construction of pBINAW2b::R3a with reduced T-DNA borders A new backbone vector including the RB and LB was constructed using pBIN19 as starting material. Primers for the RB and LB were designed. Primers URB (5'-GCGGTCCTGATCAATCGTCAC-3') and RBK (5'-GGTACCTGACAGGATATATTGGCGGGTAAA-3'; with KpnI site) were used to amplify an RB upstream DNA sequence from pBIN19 of 1156 bp. Primers LBKX (5'-GGTACCTCTAGAGTTTACACCACAATATATCC-3'; with KpnI and XbaI sites) and DLB (5'-GCGGGTTTAACCTACTTCCTTT-3') were used to amplify an LB downstream DNA sequence from pBIN19 of 627 bp. Both PCR products were cloned into pGEM-T, and sequenced. The RB upstream sequence was released from the pGEM-T vector by digestion with SacI and KpnI. The LB downstream sequence was released from the pGEM-T vector by digestion with KpnI and NsiI. Fragments were isolated from agarose gels. The SacI/KpnI RB upstream sequence and the KpnI/NsiI LB downstream sequence were ligated into SacI/NsiI digested pUC28 vector (Bene 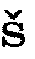 et al., 1993), resulting in plasmid pUC28-LBRB. Through this ligation the RB and LB were ligated to each other, separated only by a KpnI and an XbaI recognition sequence. A 644-bp BglII/Nsi fragment was released from this vector, and ligated to the 6966-bp BclI/Nsil backbone vector sequence from pBIN19, resulting in plasmid pBINAW2. By this ligation the tetR gene flanking the RB was deleted.
To obtain a vector with more than two unique restriction sites between the RB and LB plasmid pUC28-LBRB was digested with KpnI, and subsequently self-ligated, resulting in plasmid pUC28-LBRB1. A 562-bp XbaI fragment from plasmid pUC28-LBRB was ligated into XbaI-digested pUC28-LBRB1, resulting in plasmid pUC28-LBRB2. This plasmid contains the RB upstream sequence and the LB downstream sequence, separated by a multiple cloning site for KpnI, SmaI, BamHI and XbaI. Plasmid pUC28-LBRB2 was digested with KpnI and NsiI. The 526-bp fragment was isolated from an agarose gel and ligated into KpnI/NsiI-digested pBINAW2, resulting in vector pBINAW2a. A double stranded oligo was made by annealing primers MNO1 (5'-CGGCGCGCCCGGGTTAATTAAG-3') and MNO2 (5'-GATCCTTAATTAACCCGGGCGCGCCGGTAC-3'). This sequence contains KpnI and BamHI sticky ends and restriction sites for AscI, SmaI and PacI. The oligo was ligated into KpnI/BamHI-digested pBINAW2a, resulting in vector pBINAW2b.

Vector pBINAW2b::R3a was developed by the cloning of a PacI/AscI genomic DNA fragment of SH23-2 into PacI/AscI-digested pBINAW2b. SH23-2 is a subclone of the Bacterial Artificial Chromosome (BAC) SH23 (Huang et al. 2005). This BAC was partially digested with Sau3AI and the 7-10 kb fraction was ligated into the BamHI site of vector pBINPLUS.

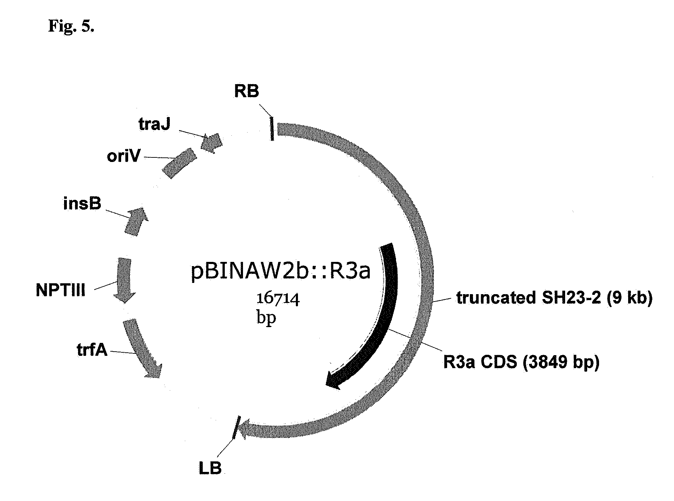

The T-DNA in vector pBINAW2b::R3a contains a 9-kb fragment of the SH23-2 pBINPLUS subclone, in which the Coding Sequence (CDS) of gene R3a is situated. The 3849-bp CDS is (after transformation of the plant) regulated by the original promoter and terminator of R3a which are both present on SH23-2 (Fig. 5). The genomic fragment SH23-2 is isolated from the diploid potato clone SH83-92-488 (Huang et al. 2005).

The binary vector pBINAW2b::R3a was transformed into *Agrobacterium tumefaciens* strain COR308 by triparental mating, using helper plasmid pRK2013 (Figurski and Helinski, 1979).

### Transformation of potato and selection of transformants

Internodal cuttings from in *vitro* grown plants of potato cultivar 'Desiree' were used for transformation by *Agrobacterium tumefaciens* co-cultivation, according to the protocol described by Visser et al. (1991a).

Potato cultivar 'Desiree' was transformed with pBINAW2b::R3a in *A*. *tumefaciens* COR308. No selection was performed. After four weeks the first shoots were harvested and harvesting of shoots continued for about three months. No more than two regenerants per stem explant were harvested and shoots were allowed to grow on MS30 medium in a glass jars (diameter 10 cm) or plastic jars (diameter 15 cm). Each jar contained 5 cuttings. A 2 to 3 cm space was left between the cuttings and the inner wall of the jars.

### In vitro selection with avr3

*In vitro* plantlets were grown at 24°C and 16 h light/8 h dark. Plantlets with 3 to 4 fully developed leaves were used for *in vitro* inoculation. Depending on the potato genotype, after 2 to 4 weeks plantlet were ready for inoculation.

The 3 largest leaves of *each in vitro* plantlet were inoculated (1 spot per leaf) by pipetting 10 µl droplets of a zoospore suspension of 2.5 x 10⁴ spores/ml on the adaxial side. Inoculum preparation and inoculation were performed in sterilized conditions. Leaves touching the inner wall or the lid of the jars or the medium were excluded because we found these leaves to be more susceptible than others of the same plants. The jars with inoculated *in vitro* plantlets were incubated in the same climate chamber as the trays with inoculated detached leaves.
Non-transformed plantlet showing a susceptible interactions were scored as S (spreading lesion with massive sporulation) or class SQ (spreading lesion with no or little sporulation). Putative transformants showing a resistant interaction were scored as class R (no symptom or localized HR-like necrosis) or class RQ (trailing HR necrosis).
Putative transformants were transferred to new media and/or transferred to potting soil in the greenhouse.

### DNA analyses

DNA was isolated from *in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1, 2, 5, 6, 7 and 8; Wolters et al. 1998a), to study the presence of backbone vector DNA in the transformants.

Four µg of DNA of the transformants was digested with HindIII, fragments were separated by gel electrophoresis, and Southern blots were made. Blots were hybridized with the R3a probe, to check the number of T-DNA insertions.

### Border-free transformants

### PCR on the border sequences

The right border and left border sequences of the T-DNA are not derived from *Solanum* sp., but obtained from pBIN19 (pTiT37 borders). To analyze whether these border sequences are present or absent in the transformants PCRs were performed. For the analysis of the right border the following primers were used: primer RBcheck1 (5'-CCAATATATCCTGTCAGGTA-3') and primer SHcis1 (5'-CATCATCATCCCAAGTACAA-3'). With these primers a fragment of 795 bp was expected when DNA of vector pBINAW2b::R3a was used as template. For the analysis of the left border primer SHcis2 (5'-AAGGGCAAACATAACCATTC-3') was used in combination with primer LBcheck1 (5'-GGATATATTGTGGTGTAAAC-3'). With these primers a fragment of 1084 bp was expected when DNA of vector pBINAW2b::R3a was used as template.

### Universal genomic walking

To obtain T-DNA flanking sequences from the transformants the Universal GenomeWalker Kit (Clontech) was used. 13NA of the transformants was digested with DraI, EcoRV, PvuII, ScaI and StuI. Genome Walker Adaptors were ligated to the obtained fragments to create enzyme-specific libraries. To obtain RB-flanking DNA the first PCR was performed with adaptor primer AP1 (5'-GTAATACGACTCACTATAGGGC-3') and primer SHGW1 (5'-TAAGTTTAATCAGAAGTTGGGTAGGAA-3'). The nested PCR was performed with adaptor primer AP2 (5'-ACTATAGGGCACGCGTGGT-3') and primer SHGW2 (5'-GTTGGGTAGGAAGCCTGCTCTTGGAAA-3'). To obtain LB-flanking DNA the first PCR was performed with adaptor primer AP1 and primer SHGW3 (5'-GTATGTATGTGTAGTTAATGGGGTAGT-3'). The nested PCR was performed with adaptor primer AP2 and primer SHGW4 (5'-ACGGTTTCTAAATTAACGTAGCCAATA-3').

### Example 3

### Selection of cisgene R3a transformants via PCR selection.

The binary vector pBINAW2b::R3a as described in example 2 was transformed into *Agrobacterium tumefaciens* strain COR308 by triparental mating, using helper plasmid pRK2013 (Figurski and Helinski, 1979).

### Transformation of potato and selection of transformants

Internodal cuttings *from in vitro* grown plants of potato cultivar 'Desiree' were used for transformation by *Agrobacterium tumefaciens* co-cultivation, according to the protocol described by Visser et al. (1991a).

Potato cultivar 'Desiree' was transformed with pBINAW2b::R3a in A. *tumefaciens* COR308. No selection was performed. After four weeks the first shoots were harvested and harvesting of shoots continued for about three months. No more than two regenerants per stem explant were harvested and shoots were allowed to grow on MS20 medium. After 1 to 2 weeks leaf or stem material of 8 or more independent shoots was harvested and pooled (pool size depended on the frequency of transformants expected). DNA of these pools of shoots was isolated in 96-wells microtiter plates using CTAB DNA isolation method or the Magnesil genomic DNA isolation kit from Promega. PCR analyis was performed on DNA isolated from the pools of regenerants with the primers R3aF (5'-AGCTGTGAAGGCAAAGATGAGG-3') and R3aR (5'-CGTCACTGCCATCATGGTAGAT-3') yielding a 1106-bp PCR product to check for the presence of transformants. Of the PCR-positive pools leaf and / or stem material of individual shoots was harvested in 96-wells microtiter plates and genomic DNA was isolated using the Magnesil genomic DNA isolation kit from Promega. PCR analysis was performed on DNA isolated from the individual regenerants with the primers BINMCS and GBSS-0, to check for the presence of transformants.

### DNA analyses

DNA was isolated *from in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1, 2, 5, 6, 7 and 8; Wolters et al. 1998a), to study the presence of backbone vector DNA in the transformants.

Four µg of DNA of the transformants was digested with HindIII, fragments were separated by gel electrophoresis, and Southern blots were made. Blots were hybridized with the R3a probe, to check the number of T-DNA insertions.

### Border-free transformants

### PCR on the border sequences

The right border and left border sequences of the T-DNA are not derived from *Solanum* sp., but obtained from pBIN19 (pTiT37 borders). To analyze whether these border sequences are present or absent in the transformants PCRs were performed. For the analysis of the right border the following primers were used: primer RBcheck1 (5'-CCAATATATCCTGTCAGGTA-3') and primer SHcis1 (5'-CATCATCATCCCAAGTACAA-3'). With these primers a fragment of 795 bp was expected when DNA of vector pBINAW2b::R3a was used as template. For the analysis of the left border primer SHcis2 (5'-AAGGGCAAACATAACCATTC-3') was used in combination with primer LBcheck1 (5'-GGATATATTGTGGTGTAAAC-3'). With these primers a fragment of 1084 bp was expected when DNA of vector pBINAW2b::R3a was used as template.

### Universal genomic walking

To obtain T-DNA flanking sequences from the transformants the Universal GenomeWalker Kit (Clontech) was used. DNA of the transformants was digested with DraI, EcoRV, PvuII, ScaI and StuI. GenomeWalker Adaptors were ligated to the obtained fragments to create enzyme-specific libraries. To obtain RB-flanking DNA the first PCR was performed with adaptor primer AP1 (5'-GTAATACGACTCACTATAGGGC-3') and primer SHGW1 (5'-TAAGTTTAATCAGAAGTTGGGTAGGAA-3'). The nested PCR was performed with adaptor primer AP2 (5'-ACTATAGrGGCACGCGTGGT-3') and primer SHGW2 (5'-GTTGGGTAGGAAGCCTGCTCTTGGAAA-3'). To obtain LB-flanking DNA the first PCR was performed with adaptor primer AP1 and primer SHGW3 (5'-GTATGTATGTGTAGTTAATGGGGTAGT-3'). The nested PCR was performed with adaptor primer AP2 and primer SHGW4 (5'-ACGGTTTCTAAATTAACGTAGCCAATA-3').

### Example 4.

### Marker-free R3a - GBSS-IR construct

### Construction of an all potato R3a-GBSS-IR T-DNA vector.

The T-DNA sequence is completely derived from potato GBSSI genomic sequences. To clone an extended GBSSI promoter the sequence upstream of the HindIII site of the commonly used promoter (Visser et al., 1991b; van der Leij et al., 1991a; accession number X58453) until the BglII site 0.6 kb upstream (van der Leij et al., 1991a) was determined. For this a PCR was performed with primers GBSS-0 (5'-TACCGCTACCACTTGACATTC-3') and BINMCS (5'-GCACCCCAGGCTTTACACTTT-3') using DNA of plasmid pWAM101 (van der Leij et al., 1991b) as template. The 738-bp sequence between BglII and HindIII was highly homologous (98% identical) to the sequence published by Dai et al. (1996), accession number X83220. Primers were designed to amplify the GBSSI promoter and upstream region: primer UPGBX (5'-CTCTACrAAGTTCGAGACACTGGCTACG-3'; with XbaI site) and primer PGBB (5'-GGATCCTGGAGGAGATGAGTAAAAGTTA-3'; with BamHI site). These primers were used in a PCR with pWAM200 DNA as template. Vector pWAM200 contains the same 6.5-kb BglII fragment of GBSSI genomic DNA as vector pWAM100 (van der Leij et al., 1991a), but cloned in pMTL24 (Chambers et al., 1988). The 1528-bp PCR product was cloned in pGEM-T and sequenced.
For the GBBSI terminator and downstream sequences primers were designed on the basis of the sequence published by van der Leij et al. (1993) (accession number X66826). Primers TGBB (5'-GGATCCAAACGTATTTACTAGCGAACT-3'; with BamHI site) and DTGBK (5'-GGTACCAAACrAGACAGGTGCCGTTAT-3'; with KpnI site) amplified a 658-bp PCR product containing the GBSSI terminator plus downstream sequences from plasmid pWAM200. This PCR produkt was cloned into pGEM-T and sequenced.
The extended GBSSI promoter was released from the pGEM-T vector by digestion with XbaI and BamHI. The extended GBSSI terminator was released from the pGEM-T vector by digestion with BamHI and KpnI. Both fragments were ligated into XbaI/KpnI-digested pUC28 (Bene 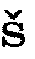 et al., 1993), resulting in plasmid pUC28-PTGB. Subsequently, plasmid pUC28-PTGB was digested with XbaI and KpnI, and the released fragment was ligated into XbaI/KpnI-digested pBINAW2a vector (see Example 1). In the resulting vector pBINAW3 the extended GBSSI promoter is flanked by the LB and the extended GBSSI terminator is flanked by the RB.

A 1390-bp BamHI fragment containing an inverted repeat of the middle part of the GBSSI cDNA, with a spacer sequence consisting of potato GBSSI cDNA was isolated from vector pIRMAS (Heilersig et al., 2006). This fragment was cloned into BamHI-digested pBINAW3. The resulting binary vector pBINAW4 contained an antisense-sense inverted repeat of the GBSSI gene between an extended GBSSI promoter and an extended GBSSI terminator flanked immediately by the LB and RB sequences of pBIN19.

Vector pBINAW4a was derived from vector pBINAW4. A double-stranded oligo with two KpnI sticky ends was ligated into the KpnI site of pBINAW4. This double-stranded oligo harbours restriction sites for AscI, SmaI and PvuI. Two orientations of the oligo are possible: one with the AscI site closest to the RB, and one with the PvuI site closest to the RB. The first orientation was selected.

pBINAW4a::R3a was developed by the cloning of a PacI/AscI genomic DNA fragment of SH23-2 into PvuI/AscI-digested pBINAW4a. SH23-2 is a subclone of the Bacterial Artificial Chromosome (BAC) SH23 (Huang et al. 2005). This BAC was partially digested with Sau3AI and the 7-10 kb fraction was ligated into the BamHI site of vector pBINPLUS. Using the restriction enzymes PacI and AscI, a 9-kb fragment (truncated SH23-2) was cut out of pBINPLUS::R3a and the fragment was ligated into the PvuI and AscI sites of pBINAW4a.

The T-DNA contains a fragment of SH23-2, in which the Coding Sequence (CDS) of the R3a gene is present. This CDS is (after transformation of the plant) regulated by the original promoter and terminator of R3a which are also present on SH23-2. The genomic fragment SH23-2 is isolated from the diploid potato clone SH83-92-488 (Huang et al., 2005).

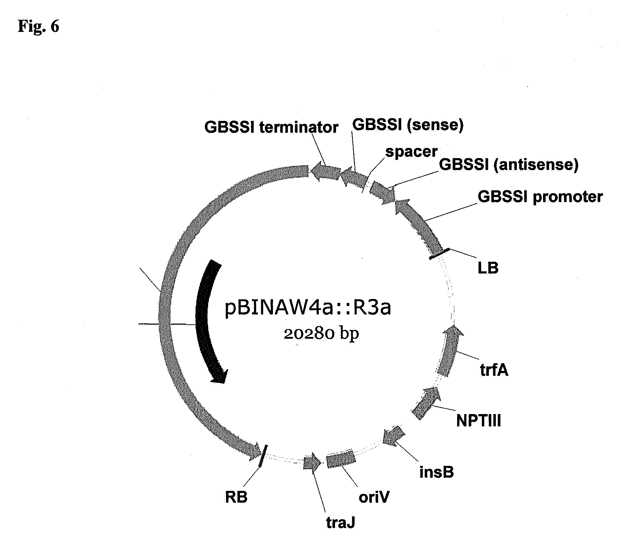

The binary vector pBINAW4a::R3a (Fig. 6) was transformed into *Agrobacterium tumefaciens* strain COR308 by triparental mating, using helper plasmid pRK2013 (Figurski and Helinski, 1979).

### Transformation of potato and selection of transformants

Internodal cuttings from *in vitro* grown plants of potato cultivars 'Desiree', 'Aventra' or 'Aveka' were used for transformation by *Agrobacterium tumefaciens* co-cultivation, according to the protocol described by Visser (1991a).
Potato cultivar 'Desiree' was transformed with pBINAW4a::R3a in A. *tumefaciens* COR308 according to the same protocol. No selection was performed. After four weeks the first shoots were harvested and harvesting of shoots continued for about three months. No more than two regenerants per stem explant were harvested and shoots were allowed to grow on MS30 medium in a glass jars (diameter 10 cm) or plastic jars (diameter 15 cm). Each jar contained 5 cuttings. A 2 to 3 cm space was left between the cuttings and the inner wall of the jars.

### In vitro assay for disease testing

*In vitro* plantlets were grown at 24°C and 16 h light/8 h dark. Plantlets with 3 to 4 fully developed leaves were used for *in vitro* inoculation. Depending on the potato genotype, after 2 to 4 weeks plantlet were ready for inoculation.

The 3 largest leaves of each *in vitro* plantlet were inoculated (1 spot per leaf) by pipetting 10 µl droplets of a zoospore suspension of 2.5 x 10 ⁴ spores/ml on the adaxial side. Inoculum preparation and inoculation were performed in sterilized conditions. Leaves touching the inner wall or the lid of the jars or the medium were excluded because we found these leaves to be more susceptible than others of the same plants. The jars with inoculated *in vitro* plantlets were incubated in the same climate chamber as the trays with inoculated detached leaves.
Non-transformed plantlet showing a susceptible interactions were scored as S (spreading lesion with massive sporulation) or class SQ (spreading lesion with no or little sporulation). Putative transformants showing resistant interactions were scored as class R (no symptom or localized HR-like necrosis) or class RQ (trailing HR necrosis).
Putative transformants were transferred to new media and/or transferred to potting soil in the greenhouse.

### In vitro tuberization

To PCR-positive shoots 20 ml of a liquid medium was added, consisting of KI medium ('knolinducerend' (= tuber inducing) medium, Duchefa) with 325 g/L sucrose and 1.75 g/L CCC (chlorocholine chloride, or cycocel.). The pots were placed in a dark growth chamber at a temperature of 18°C. After 2 to 4 weeks microtubers had developed on most shoots.

### Starch staining

Microtubers were cut and stained with an iodine solution to assess the presence of amylose in the starch granules. Staining of the starch granules was inspected with a microscope.

### DNA analyses

DNA was isolated *from in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1, 2, 5, 6, 7 and 8; Wolters et al. 1998a), to study the presence of backbone vector DNA in the transformants.

Four µg of DNA of the transformants was digested with HindIII, fragments were separated by gel electrophoresis, and Southern blots were made. Blots were hybridized with an R3a probe, to check the number of T-DNA insertions.

### Border-free transformants

### PCR on the border sequences

The right border and left border sequences of the T-DNA are not derived from *Solanum* sp., but obtained from pBIN19 (pTiT37 borders). To analyze whether these border sequences are present or absent in the transformants PCRs were performed. For the analysis of the right border the following primers were used: primer RBcheck1 (5'-CCAATATATCCTGTCAGGTA-3') and primer SHcis1 (5'-CATCATCATCCCAAGTACAA-3'). With these primers a fragment of 795 bp was expected when DNA of vector pBINAW4a::R3a was used as template. For the analysis of the left border primer GBSSIcis1 (5'-CTCTGTCAACAGCCAAATAG-3') was used in combination with primer LBcheck1 (5'-GGATATATTGTGGTGTAAAC-3'). With these primers a fragment of 836 bp was expected when DNA of vector pBINAW4a::R3a was used as template.

### Universal genomic walking

To obtain T-DNA flanking sequences from the transformants the Universal GenomeWalker Kit (Clontech) was used. DNA of the transformants was digested with DraI, EcoRV, PvuII, ScaI and StuI. GenomeWalker Adaptors were ligated to the obtained fragments to create enzyme-specific libraries. To obtain RB-flanking DNA the first PCR was performed with adaptor primer AP1 (5'-GTAATACGACTCACTATAGGGC-3') and primer SHGW1 (5'-TAAGTTTAATCAGAAGTTGGGTAGGAA-3'). The nested PCR was performed with adaptor primer AP2 (5'-ACTATAGGGCACGCGTGGT-3') and primer SHGW2 (5'-GTTGGGTAGGAAGCCTGCTCTTGGAAA-3'). To obtain LB-flanking DNA the first PCR was performed with adaptor primer AP1 and primer GBSSIGW1 (5'-TTTACTCATCTCCTCCAGGATCCTTCT-3'). The nested PCR was performed with adaptor primer AP2 and primer GBSSIGW2 (5'-ATCTCCTCCAGGATCCTTCTGCTCCTC-3').

### Example 5.

### Marker-free cisgene R3a - blbl construct

Vector pBINAW2b::blb1-R3a is a binary plasmid without a selection gene in the T-DNA, and is derived from vector pBINAW2b::R3a. Vector pBINAW2b::R3a was developed by cloning the SH23-2 fragment with the restriction enzymes PacI and AscI from a subclone, and ligating it into the PacI and AscI sites of pBINAW2b (see Example 1). SH23-2 is a pBINPLUS subclone developed by subcloning the Bacterial Artificial Chromosome (BAC) SH23 (Huang et al. 2005). This BAC was partially digested with Sau3AI and the 7-10 kb fraction was ligated into the BamHI site of pBINPLUS.
After the development of pBINAW2b::R3a, a RGC2 fragment was placed between the AscI site and the right border using the restriction enzymes AscI and Sbfl. RGC2 is a pBINPLUS subclone and is, just like SH23-2, developed by cloning the 7-10 kb fraction of Sau3AI partially digested BAC (SPB4) (van der Vossen et al., 2003) in the BamHI site of pBINPLUS.
A 6.5-kb fragment of RGC2 was amplified using the Polymerase Chain Reaction (PCR). The used primers were extended with an AscI and SbfI site. The amplified fragment could therefore be digested with these enzymes and this fragment was then ligated into the AscI and SbfI sites of pBINAW2b::R3a.

The total pBINAW2b::blb1-R3a construct has a size of 23,147 bp. The T-DNA is flanked by borders; Left Border (LB) and Right Border (R). These borders originate from Agrobacterium and serve as a signal for Agrobacterium to discriminate the border between vector and T-DNA. The T-DNA is integrated by Agrobacterium into the potato genome.
The T-DNA contains a fragment of the SH23-2 and RGC2 pBINPLUS subclones, in which the Coding Sequence (CDS) of R3a is situated on SH23-2 and the CDS of Rpi-blb1 is situated on RGC2. These CDS are (after transformation of the plant) regulated by the original promoter and terminator of R3a and Rpi-blb1 which are also present on both SH23-2 and RGC2. The genomic fragment SH23-2 is isolated from the diploid potato clone SH83-92-488 (Huang et al. 2005) and the genomic fragment RGC2 is isolated from *Solanum bulbocastanum.*
There is no selection gene (like NPTII) present in the T-DNA for the selection of transgenic plants. Only the genomic SH23-2 fragment, the genomic RGC2 fragment and the border sequences will be integrated into the plant genome.

The binary vector pBINAW2b::blb1-R3a was transformed into *Agrobacterium tumefaciens* strain COR308 by triparental mating, using helper plasmid pRK2013 (Figurski and Helinski, 1979).

### Transformation of potato and selection of transformants

Internodal cuttings *from in vitro* grown plants of potato cultivar 'Desiree' were used for transformation by *Agrobacterium tumefaciens* co-cultivation, according to the protocol described by Visser et al. (1991a).

Potato cultivar 'Desiree' was transformed with pBINAW2b::b1b1-R3a in A. *tumefaciens* strain COR308. No selection was performed. After four weeks the first shoots were harvested and harvesting of shoots continued for about three months. No more than two regenerants per stem explant were harvested and shoots were allowed to grow on MS30 medium in a glass jars (diameter 10 cm) or plastic jars (diameter 15 cm). Each jar contained 5 cuttings. A 2 to 3 cm space was left between the cuttings and the inner wall of the jars.

### In vitro selection with avr3

*In vitro* plantlets were grown at 24°C and 16 h light/8 h dark. Plantlets with 3 to 4 fully developed leaves were used for *in vitro* inoculation. Depending on the potato genotype, after 2 to 4 weeks plantlet were ready for inoculation.

The 3 largest leaves of each *in vitro* plantlet were inoculated (1 spot per leaf) by pipetting 10 µl droplets of a zoospore suspension of 2.5 x 10 ⁴ spores/ml on the adaxial side. Inoculum preparation and inoculation were performed in sterilized conditions. Leaves touching the inner wall or the lid of the jars or the medium were excluded because we found these leaves to be more susceptible than others of the same plants. The jars with inoculated *in vitro* plantlets were incubated in the same climate chamber as the trays with inoculated detached leaves.
Non-transformed plantlet showing a susceptible interaction were scored as S (spreading lesion with massive sporulation) or class SQ (spreading lesion with no or little sporulation). Putative transformants showing a resistant interaction were scored as class R (no symptom or localized HR-like necrosis) or class RQ (trailing HR necrosis).
Putative transformants were transferred to new media and/or transferred to potting soil in the greenhouse.

### Putative transformants were re-analysed for the expression of the blb1 gene. Therefore, either in vitro plants or leaves of greenhouse plants were inoculated with IpO1.

For the detached leaf assay, the 3rd to 5th fully developed leaves (counted from the top) were cut from greenhouse-grown plants and placed in water saturated florists foam (Oasis®, Grünstadt, Germany) in a tray. Depending on the shape and size of the *Solanum* leaves, one or more leaves were used for inoculation. In general, 10 spots were inoculated per genotype by pipetting 10 µl droplets of a zoospore suspension of 5 x 10⁴ spores/ml on the abaxial side. The trays were then covered with transparent lids (covered trays), transferred into a climate chamber, and incubated at a 16h/8h day/night photoperiod at 16°C.

Resistance level was determined by determining the lesion size (LS), macroscopic scoring, or a combination of both. LS was measured usually at day 6 after spot inoculation using an electronic caliper connected to a computer. The mean LS was calculated from 10 replicates. Based on the average lesion size, a relative score from 0-9 was assigned, relative to Bintje (Table 2). Also the lesion phenotype was examined. In addition macroscopic scoruing was carried out as follows.
Compatible interactions were classified as S (susceptible, spreading lesion with massive sporulation) or SQ (spreading lesion with no or little sporulation). Incompatible interactions were scored as R (Resistant, no symptom or localized HR-like necrosis) or RQ (trailing HR necrosis). Also intermediate phenotypes (Q) between compatible and incompatible ones were observed, such as sporulation on 2 to 3 leaflets per compound leaf and localized HR on the other leaflets.

### DNA analyses

DNA was isolated *from in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1, 2, 5, 6, 7 and 8; Wolters et al. 1998a), to study the presence of backbone vector DNA in the transformants.

Four µg of DNA of the transformants was digested with HindIII, fragments were separated by gel electrophoresis, and Southern blots were made. Blots were hybridized with the R3a probe, to check the number of T-DNA insertions.

### Border-free transformants

### PCR on the border sequences

The right border and left border sequences of the T-DNA are not derived from *Solanum* sp., but obtained from pBIN19 (pTiT37 borders). To analyze whether these border sequences are present or absent in the transformants PCRs were performed. For the analysis of the right border the following primers were used: primer RBcheck1 (5'-CCAATATATCCTGTCAGGTA-3') and primer RGCcis1 (5'-CGCTTTCAGAATCTATTACT-3'). With these primers a fragment of 693 bp was expected when DNA of vector pBINAW2b::b1b1-R3a was used as template. For the analysis of the left border primer SHcis2 (5'-AAGGGCAAACATAACCATTC-3') was used in combination with primer LBcheck1 (5'-GGATATATTGTGGTGTAAAC-3'). With these primers a fragment of 1084 bp was expected when DNA of vector pBINAW2b::blb1-R3a was used as template.

### Universal genomic walking

To obtain T-DNA flanking sequences from the transformants the Universal GenomeWalker Kit (Clontech) was used. DNA of the transformants was digested with DraI, EcoRV, PvuII, ScaI and StuI. GenomeWalker Adaptors were ligated to the obtained fragments to create enzyme-specific libraries. To obtain RB-flanking DNA the first PCR was performed with adaptor primer AP1 (5'-GTAATACGACTCACTATAGGGC-3') and primer RGCGW1 (5'-ATTTCATGCGCATATTCCCGATCAAAC-3'). The nested PCR was performed with adaptor primer AP2 (5'-ACTATAGGGCACGCGTGGT-3') and primer RGCGW2 (5'-TCCCGATCAAACTTAAATTACTAGACT-3'). To obtain LB-flanking DNA the first PCR was performed with adaptor primer AP1 and primer SHGW3 (5'-GTATGTATGTGTAGTTAATGGGGTAGT-3'). The nested PCR was performed with adaptor primer AP2 and primer SHGW4 (5'-ACGGTTTCTAAATTAACGTAGCCAATA-3').

### Example 6.

### Marker-free R3a::cooking type

### Marker-free transformation - selection for amylose-free - selection of vector DNA, border-free transformants

### Marker-free R3a::cooking type gene StTLRP

### Marker-free transformation- selection for R3a - selection of vector DNA-free, border-free transformants

The coding sequence of cooking type gene StLTRP (Kloosterman 2006) was amplified with PCR primers LTF1 (5'-GGATCCATGGGTTCCAAGGCAATTATGrTT-3') and LTR1 (5'-GGATCCGAATGGCTTTATTCATACTTGTT-3'). The 360-bp PCR fragment was cloned into pGEM-T vector, and subsequently digested with BamHI. The BamHI insert was isolated from an agarose gel, and ligated to the large BamHI fragment of vector pBINAW4 (see Example 2), thereby replacing the GBSSI cDNA inverted repeat with the StTLRP cDNA sequence. A clone with the StTLRP cDNA in sense orientation between the GBSSI promoter and GBSSI terminator was selected and named pBINAW7.
A double-stranded oligo with two KpnI sticky ends was ligated into the KpnI site of pBINAW7. This double-stranded oligo harbours restriction sites for AscI, SmaI and PvuI. Two orientations of the oligo are possible: one with the AscI site closest to the RB, and one with the PvuI site closest to the RB. The first orientation was selected. The resulting vector was named pBINAW7a. pBINAW7a::R3a was developed by the cloning of a PacI/AscI genomic DNA fragment of SH23-2 into PvuI/AscI-digested pBINAW7a. SH23-2 is a subclone of the Bacterial Artificial Chromosome (BAC) SH23 (Huang et al. 2005). This BAC was partially digested with Sau3AI and the 7-10 kb fraction was ligated into the BamHI site of vector pBINPLUS. Using the restriction enzymes PacI and AscI, a 9-kb fragment (truncated SH23-2) was cut out of pBINPLUS::R3a and the fragment was ligated into the PvuI and AscI sites of pBINAW7a.

The T-DNA contains a fragment of SR23-2, in which the Coding Sequence (CDS) of the R3a gene is present. This CDS is (after transformation of the plant) regulated by the original promoter and terminator of R3a which are also present on SH23-2. The genomic fragment SH23-2 is isolated from the diploid potato clone SH83-92-488 (Huang et al., 2005).

The binary vector pBINAW7a::R3a was transformed into *Agrobacterium tumefaciens* strain COR308by triparental mating, using helper plasmid pRK2013 (Figurski and Helinski, 1979).

### Transformation of potato and selection of transformants

Internodal cuttings *from in vitro* grown plants of potato cultivars 'Desiree', 'Aventra' or 'Aveka' were used for transformation by *Agrobacterium tumefaciens* co-cultivation, according to the protocol described by Visser (1991a).
Potato cultivar 'Desiree' was transformed with pBINAW4a::R3a in A. *tumefaciens* COR308 according to the same protocol. No selection was performed. After four weeks the first shoots were harvested and harvesting of shoots continued for about three months. No more than two regenerants per stem explant were harvested and shoots were allowed to grow on MS30 medium in a glass jars (diameter 10 cm) or plastic jars (diameter 15 cm). Each jar contained 5 cuttings. A 2 to 3 cm space was left between the cuttings and the inner wall of the jars.

### In vitro assay for disease testing

*In vitro* plantlets were grown at 24°C and 16 h light/8 h dark. Plantlets with 3 to 4 fully developed leaves were used for *in vitro* inoculation. Depending on the potato genotype, after 2 to 4 weeks plantlet were ready for inoculation.

The 3 largest leaves of *each in vitro* plantlet were inoculated (1 spot per leaf) by pipetting 10 µl droplets of a zoospore suspension of 2.5 x 10 spores/ml on the adaxial side. Inoculum preparation and inoculation were performed in sterilized conditions. Leaves touching the inner wall or the lid of the jars or the medium were excluded because we found these leaves to be more susceptible than others of the same plants. The jars with inoculated *in vitro* plantlets were incubated in the same climate chamber as the trays with inoculated detached leaves.
Non-transformed plantlet showing a susceptible interactions were scored as S (spreading lesion with massive sporulation) or class SQ (spreading lesion with no or little sporulation). Putative transformants showing a resistant interactions were scored as class R (no symptom or localized HR-like necrosis) or class RQ (trailing HR necrosis).
Putative transformants were transferred to new media and/or transferred to potting soil in the greenhouse.

### Analysis of the cooking-type

Three tubers of each sample were peeled and steam cooked for 20 minutes after texture was visually scored and categorized on a nominal scale ranging from firm/non-mealy (1) to extremely mealy tubers (6).

### DNA analyses

DNA was isolated *from in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1, 2, 5, 6, 7 and 8; Wolters et al. 1998a), to study the presence of backbone vector DNA in the transformants.

Four µg of DNA of the transformants was digested with HindIII, fragments were separated by gel electrophoresis, and Southern blots were made. Blots were hybridized with an R3a probe, to check the number of T-DNA insertions.

### Border-free transformants

### PCR on the border sequences

The right border and left border sequences of the T-DNA are not derived from *Solanum* sp., but obtained from pBIN19 (pTiT37 borders). To analyze whether these border sequences are present or absent in the transformants PCRs were performed. For the analysis of the right border the following primers were used: primer RBcheck1 (5'-CCAATATATCCTGTCAGGTA-3') and primer SHcis1 (5'-CATCATCATCCCAAGTACAA-3'). With these primers a fragment of 795 bp was expected when DNA of vector pBINAW4a::R3a was used as template. For the analysis of the left border primer GBSSIcis1 (5'-CTCTGTCAACAGCCAAATAG-3') was used in combination with primer LBcheck1 (5'-GGATATATTGTGGTGTAAAC-3'). With these primers a fragment of 836 bp was expected when DNA of vector pBINAW4a::R3a was used as template.

### Universal genomic walking

To obtain T-DNA flanking sequences from the transformants the Universal GenomeWalker Kit (Clontech) was used. DNA of the transformants was digested with DraI, EcoRV, PvuII, Seal and StuI. Genome Walker Adaptors were ligated to the obtained fragments to create enzyme-specific libraries. To obtain RB-flanking DNA the first PCR was performed with adaptor primer AP1 (5'-GTAATACGACTCACTATAGGGC-3') and primer SHGW1 (5'-TAAGTTTAATCAGAAGTTGGGTAGGAA-3'). The nested PCR was performed with adaptor primer AP2 (5'-ACTATAGGGCACGCGTGGT-3') and primer SHGW2 (5'-GTTGGGTAGGAAGCCTGCTCTTGGAAA-3'). To obtain LB-flanking DNA the first PCR was performed with adaptor primer AP1 and primer GBSSIGW3 (5'-ATTGCCTTGGAACCCATGGATCCTTCT-3'). The nested PCR was performed with adaptor primer AP2 and primer GBSSIGW4 (5'-TGGAACCCATGGATCCTTCTGCTCCTC-3').

### Example 7.

### Marker-free cisgene R3a GBSS -IR contruct with attenuation region.

### Construction of an all potato T-DNA vector with sequences outside the LB to prevent integration of backbone vector DNA

Integration of backbone vector (non-T-DNA) sequences in the plant genome frequently occurs during *Agrobacterium tumefaciens* transformation (Kononov et al. 1997; Ramanathan and Veluthambi, 1995; Van der Graaff et al., 1996; Wenck et al., 1997; Wolters et al., 1998b). Wang et al. (1987) reported that "flanking sequences of the border repeats enhance (on the right) or attenuate (on the left) their activity". They defined an 'attenuation region': a 363-bp AT-rich EcoRI/BcII fragment of the T-DNA near the LB of plasmid pTiC58. De Buck et al. (2000) stated that "it is possible that deletion of the inner border region, a piece of T-DNA present in the original Ti plasmid from which the vector was derived, causes inefficient nicking of the LB repeat, which results in read-through past the LB and the transfer of downstream-located vector sequences". Kuraya et al. (2004) reported that "transfer of the 'vector backbone' from the control vectors resulted mainly from inefficient termination of formation of the transfer intermediate of the T-DNA, and additional LB sequences effectively suppressed such transfer".
On the basis of these reports we have cloned an extra nopaline type LB together with the flanking attenuation region outside the LB of binary vector pBINAW2a, to prevent integration of backbone vector DNA in the plant genome. Ti plasmid pTiC58C1 was isolated from *Agrobacterium tumefaciens* strain LBA958 (kindly provided by Dr. P. Hooykaas, Leiden University, The Netherlands). The complete sequence of the T-DNA region of this Ti plasmid is published by Gielen et al. (1999). A 885-bp fragment was amplified from this Ti plasmid by PCR with primers LB2 (5'-TAAGCGAGAAATGAATAAGAAG-3') and LBatt (5'-GCGAGACAGATGAAACGAAGTA-3'). This fragment was cloned in pGEM-T and sequenced. This plasmid (pAtt1-1) was subsequently transformed to *E*. *coli* strain GM2163 (Fermentas), which is dam⁻ and dcm⁻. Plasmid DNA isolated from this strain was digested with BclI, releasing a 677-bp BclI fragment containing the Attenuation Region and the LB sequence. This fragment was cloned into BclI-digested and Alkaline Phosphatase-treated pBINAW2a plasmid DNA, isolated from an *E*. *coli* strain GM2163 transformed culture. Several colonies were analysed for the presence of the BclI fragment in the right orientation. This resulted in binary vector pBINAW5. Subsequently, a XbaI/KpnI fragment from vector pBINAW4 (see Example 2) containing the extended GBSSI promoter, GBSSI inverted repeat, and extended GBSSI terminator sequences, were cloned into the XbaI and KpnI sites of pBINAW5, resulting in binary vector pBINAW6.

Vector pBINAW6a was derived from vector pBINAW6. A double-stranded oligo with two KpnI sticky ends was ligated into the KpnI site of pBINAW6. This double-stranded oligo harbours restriction sites for AscI, SmaI and PvuI. Two orientations of the oligo are possible: one with the AscI site closest to the RB, and one with the PvuI site closest to the RB. The first orientation was selected.

pBINAW6a::R3a was developed by the cloning of a PacI/AscI genomic DNA fragment of SH23-2 into PvuI/AscI-digested pBINAW6a. SH23-2 is a subclone of the Bacterial Artificial Chromosome (BAC) SH23 (Huang et al. 2005). This BAC was partially digested with Sau3AI and the 7-10 kb fraction was ligated into the BamHI site of vector pBINPLUS. Using the restriction enzymes PacI and AscI, a 9-kb fragment (truncated SH23-2) was cut out of pBINPLUS::R3a and the fragment was ligated into the PvuI and AscI sites of pBINAW6a.

The T-DNA contains a fragment of SH23-2, in which the Coding Sequence (CDS) of the R3a gene is present. This CDS is (after transformation of the plant) regulated by the original promoter and terminator of R3a which are also present on SH23-2. The genomic fragment SH23-2 is isolated from the diploid potato clone SH83-92-488 (Huang et al., 2005).

The binary vector pBINAW6a::R3a was transformed into *Agrobacterium tumefaciens* strain COR308 by triparental mating, using helper plasmid pRK2013 (Figurski and Helinski, 1979).

### Transformation of potato and selection of transformants

Internodal cuttings from *in vitro* grown plants of potato cultivars 'Desiree', 'Aventra' or 'Aveka' were used for transformation by *Agrobacterium tumefaciens* co-cultivation, according to the protocol described by Visser (1991).

Potato cultivar 'Desiree' was transformed with pBINAW6a::R3a in A. *tumefaciens* strain COR308. No selection was performed. After four weeks the first shoots were harvested and harvesting of shoots continued for about three months. No more than two regenerants per stem explant were harvested and shoots were allowed to grow on MS30 medium in a glass jars (diameter 10 cm) or plastic jars (diameter 15 cm). Each jar contained 5 cuttings. A 2 to 3 cm space was left between the cuttings and the inner wall of the jars.

### In vitro assay for disease testing

*In vitro* plantlets were grown at 24°C and 16 h light/8 h dark. Plantlets with 3 to 4 fully developed leaves were used for *in vitro* inoculation. Depending on the potato genotype, after 2 to 4 weeks plantlet were ready for inoculation.
The 3 largest leaves of *each in vitro* plantlet were inoculated (1 spot per leaf) by pipetting 10 µl droplets of a zoospore suspension of 2.5 x 10⁴ spores/ml on the adaxial side. Inoculum preparation and inoculation were performed in sterilized conditions. Leaves touching the inner wall or the lid of the jars or the medium were excluded because we found these leaves to be more susceptible than others of the same plants. The jars with inoculated *in vitro* plantlets were incubated in the same climate chamber as the trays with inoculated detached leaves.
Non-transformed plantlet showing a susceptible interactions were scored as S (spreading lesion with massive sporulation) or class SQ (spreading lesion with no or little sporulation). Putative transformants showing a resistant interactions were scored as class R (no symptom or localized HR-like necrosis) or class RQ (trailing HR necrosis).
Putative transformants were transferred to new media and/or transferred to potting soil in the greenhouse.

### In vitro tuberization

To PCR-positive shoots 20 ml of a liquid medium was added, consisting of KI medium ('knolinducerend' (= tuber inducing) medium, Duchefa) with 325 g/L sucrose and 1.75 g/L CCC (chlorocholine chloride, or cycocel.). The pots were placed in a dark growth chamber at a temperature of 18°C. After 2 to 4 weeks microtubers had developed on most shoots.

### Starch staining

Microtubers were cut and stained with an iodine solution to assess the presence of amylose in the starch granules. Staining of the starch granules was inspected with a microscope.

### DNA analyses

DNA was isolated *from in vitro* shoots by a CTAB DNA isolation protocol as described by Rogers and Bendich (1988). PCRs were performed with primers NPT1 and NPT2, trfA1 and trfA2, insB1 and insB2 (Sequence nos. 1, 2, 5, 6, 7 and 8; Wolters et al., 1998a), to study the presence of backbone vector DNA in the transformants.

Four µg of DNA of the transformants was digested with HindIII, fragments were separated by gel electrophoresis, and Southern blots were made. Blots were hybridized with an R3a probe, to check the number of T-DNA insertions.

### Border-free transformants

### PCR on the border sequences

The right border and left border sequences of the T-DNA are not derived from *Solanum* sp., but obtained from pBIN19 (pTiT37 borders). To analyze whether these border sequences are present or absent in the transformants PCRs were performed. For the analysis of the right border the following primers were used: primer RBcheck1 (5'-CCAATATATCCTGTCAGGTA-3') and primer SHcis1 (5'-CATCATCATCCCAAGTACAA-3'). With these primers a fragment of 795 bp was expected when DNA of vector pBINAW6a::R3a was used as template. For the analysis of the left border primer GBSSIcis1 (5'-CTCTGTCAACAGCCAAATAG-3') was used in combination with primer LBcheck1 (5'-GGATATATTGTGGTGTAAAC-3'). With these primers a fragment of 836 bp was expected when DNA of vector pBINAW6a::R3a was used as template.

### Universal genomic walking

To obtain T-DNA flanking sequences from the transformants the Universal GenomeWalker Kit (Clontech) was used. DNA of the transformants was digested with DraI, EcoRV, PvuII, ScaI and StuI. GenomeWalker Adaptors were ligated to the obtained fragments to create enzyme-specific libraries. To obtain RB-flanking DNA the first PCR was performed with adaptor primer AP1 (5'-GTAATACGACTCACTATAGGGC-3') and primer SHGW1 (5'-TAAGTTTAATCAGAAGTTGGGTAGGAA-3'). The nested PCR was performed with adaptor primer AP2 (5'-ACTATAGGGCACGCGTGGT-3') and primer SHGW2 (5'-GTTGGGTAGGAAGCCTGCTCTTGGAAA-3'). To obtain LB-flanking DNA the first PCR was performed with adaptor primer AP1 and primer GBSSIGW1 (5'-TTTACTCATCTCCTCCAGGATCCTTCT -3'). The nested PCR was performed with adaptor primer AP2 and primer GBSSIGW2 (5'- ATCTCCTCCAGGATCCTTCTGCTCCTC-3').

### Tables

**Table 1. R-genes and quantitative trait loci for late blight resistance reported for wild Solanum species**

| **Wild species** | **Locus type or** | **Chromosome** |
|---|---|---|
| | **name** | |
| S. berthaultii | QTLs (4) | I, III, VII and XI |
| | *Rpi-ber 1* | X |
| | *Rpi-ber2* | VII |
| S. bulbocastanum | *RB*/*Rpi-blbl* | VIII |
| | *Rpi-blb2* | VI |
| | *Rpi-blb3* | IV |
| S. caripense | QTL (2) | unassigned |
| S. demissum | *R1* | V |
| | *R2* | IV |
| | *R3, R6, R7* | XI |
| | *R3a* | XI |
| | *R3b* | XI |
| | *R5 - R11* | XI |
| | *R10, R11* | XI |
| S. microdontum | QTLs (3) | IV, V and X |
| | QTL | Unassigned |
| S. mochiquense | *Rpi-mcgl (Rpi-* | IX |
| | *moc1)* | |
| S. paucissectum | QTLs (3) | X, XI and XII |
| S. phureja | *Rpi-phu1* | IX |
| S. pinnatisectum | *Rpi-pnt1* | VII |
| | *(Rpi1)* | |
| S. vernei | QTLs (several) | VI, VIII, IX |
| Hybrids with S. | *Rpi-abpt* | IV |
| tuberosum | | |
| | *R2-like* | IV |
| | QTLs (several) | several |
| | QTLs | IV |

### References

Bene 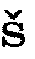 V, Hostomský Z, Arnold L, Pa 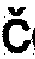 es V (1993) M13 and pUC vectors with new unique restriction sites for cloning. Gene 130: 151-152
De Buck S, De Wilde C, Van Montagu M, Depicker A (2000) T-DNA vector backbone sequences are frequently integrated into the genome of transgenic plants obtained by Agrobacterium-mediated transformation. Mol Breed 6: 459-468
Chambers SP, Prior SE, Barstow DA, Minton NP (1988) The pMTL nic-cloning vectors. I. Improved pUC polylinker regions to facilitate the use of sonicated DNA for nucleotide sequencing. Gene 68: 139-149
Dai WL, Deng W, Cui WY, Zhao SY, Wang XM (1996) Molecular cloning and sequence of potato granule-bound starch synthase gene. Acta Botanica Sinica 38: 777-784
Figurski DH, Helinski DR (1979) Replication of an origin-containing derivative of plasmid RK2 dependent on a plasmid function provided in trans. Proc Natl Acad Sci USA 76: 1648-1652
Gielen J, Terryn N, Villarroel R, Van Montagu M (1999) Complete nucleotide sequence of the T-DNA region of the plant tumour-inducing Agrobacterium tumefaciens Ti plasmid pTiC58. J Exp Bot 50: 1421-1422
Heilersig HJB, Loonen A, Bergervoet M, Wolters AMA, Visser RGF (2006) Post-transcriptional gene silencing of GBSSI in potato: effects of size and sequence of the inverted repeats. Plant Mol Biol 60: 647-662
Huang S, van der Vossen EAG, Kuang H, Vleeshouwers VGAA, Zhang N, Borm TJA, van Eck HJ, Baker B, Jacobsen E, Visser RGF (2005) Comparative genomics enabled the isolation of the R3a late blight resistance gene in potato. Plant J 42: 251-261.
Kloosterman B (2006) Transcriptomic analysis of potato tuber development and tuber quality traits using microarray technology. In search of candidate genes. PhD Thesis, Wageningen University, The Netherlands. ISBN 90-8504-454-5
Kononov ME, Bassuner B, Gelvin SB (1997) Integration of T-DNA binary vector 'backbone' sequences into the tobacco genome: evidence for multiple complex patterns of integration. Plant J 11: 945-957
Kuraya Y, Ohta S, Fukuda M, Hiei Y, Murai N, Hamada K, Ueki J, Imaseki H, Komari T (2004) Suppression of transfer of non-T-DNA 'vector backbone' sequences of higher plants mediated by Agrobacterium tumefaciens. Mol Breed 14: 309-320
Ramanathan V, Veluthambi K (1995) Transfer of non-T-DNA portions of the Agrobacterium tumefaciens Ti plasmid pTiA6 from the left terminus of TL-DNA. Plant Mol Biol 28: 1149-1154
Rogers SO, Bendich AJ (1988) Extraction of DNA from plant tissues. In: Plant Molecular Biology Manual (Gelvin SB, Schilperoort RA, eds). Kluwer Academic Publ, Dordrecht, pp. A6/1-A6/10
Torto T. et al. (2003) EST mining and functional expression assays identify extracellular effector proteins from Phytophthorta. Genome Res. 13:1675-1685.
Van der Graaff E, den Dulk-Ras A, Hooykaas PJJ (1996) Deviating T-DNA transfer from Agrobacterium tumefaciens to plants. Plant Mol Biol 31: 677-681
Van der Leij FR, Visser RGF, Ponstein AS, Jacobsen E, Feenstra WJ (1991a) Sequence of the structural gene for granule-bound starch synthase of potato (Solanum tuberosum L.) and evidence for a single point deletion in the amf allele. Mol Gen Gent 228: 240-248
Van der Leij FR, Visser RGF, Oosterhaven K, van der Kop DAM, Jacobsen E, Feenstra WJ (1991b) Complementation of the amylose-free starch mutant of potato (Solanum tuberosum) by the gene encoding granule-bound starch synthase. Theor Appl Genet 82: 289-295
Van der Leij FR, Abeln ECA, Hesseling-Meinders A, Feenstra WJ (1993) A putative β-glucanase pseudogene behind the potato GBSS gene. Plant Mol Biol 21: 567-571
Van der Vossen E, Sikkema A, te Lintel Hekkert B, Gros J, Stevens P, Muskens M, Wouters D, Pereira A, Stiekema W, Allefs S (2003) An ancient R gene from the wild potato species Solanum bulbocastanum confers broad-spectrum resistance to Phytophthora infestans in cultivated potato and tomato. Plant J 36: 867-882
Visser RGF, Somhorst I, Kuipers GJ, Ruys NJ, Feenstra WJ, Jacobsen E (1991a) Inhibition of the expression of the gene for granule-bound starch synthase in potato by antisense constructs. Mol Gen Genet 225: 289-296
Visser RGF, Stolte A, Jacobsen E (1991b) Expression of a chimaeric granule-bound starch synthase-GUS gene in transgenic potato plants. Plant Mol Biol 17: 691-699
Wang K, Genetello C, Van Montagu M, Zambryski PC (1987) Sequence context of the T-DNA border repeat element determines its relative activity during T-DNA transfer to plant cells. Mol Gen Gent 210: 338-346
Wenck A, Czakó M, Kanevski I, Márton L (1997) Frequent collinear long transfer of DNA inclusive of the whole binary vector during Agrobacteraum-mediated transformation. Plant Mol Biol 34: 913-922
Windels P, De Buck S, Van Bockstaele E, De Loose M, Depicker A (2003) T-DNA integration in Arabidopsis chromosomes. Presence and orgin of filler DNA sequences. Plant Physiol 133: 2061-2068
Wolters AMA, Janssen EM, Rozeboom-Schippers MGM; Jacobsen E, Visser RGF (1998a) Composition of endogenous alleles can influence the level of antisense inhibition of granule-bound starch synthase gene expression in tetraploid potato plants. Mol Breeding 4: 343-358
Wolters AMA, Trindade LM, Jacobsen E, Visser RGF (1998b) Fluorescence in situ hybridization on extended DNA fibres as a tool to analyse complex T-DNA loci in potato. Plant J 13: 837-847
Zhu QH, Ramm K, Eamens AL, Dennis ES, Upadhyaya NM (2006) Transgene structures suggest that multiple mechanisms are involved in T-DNA integration in plants. Plant Science 171: 308-322

### Description of figures

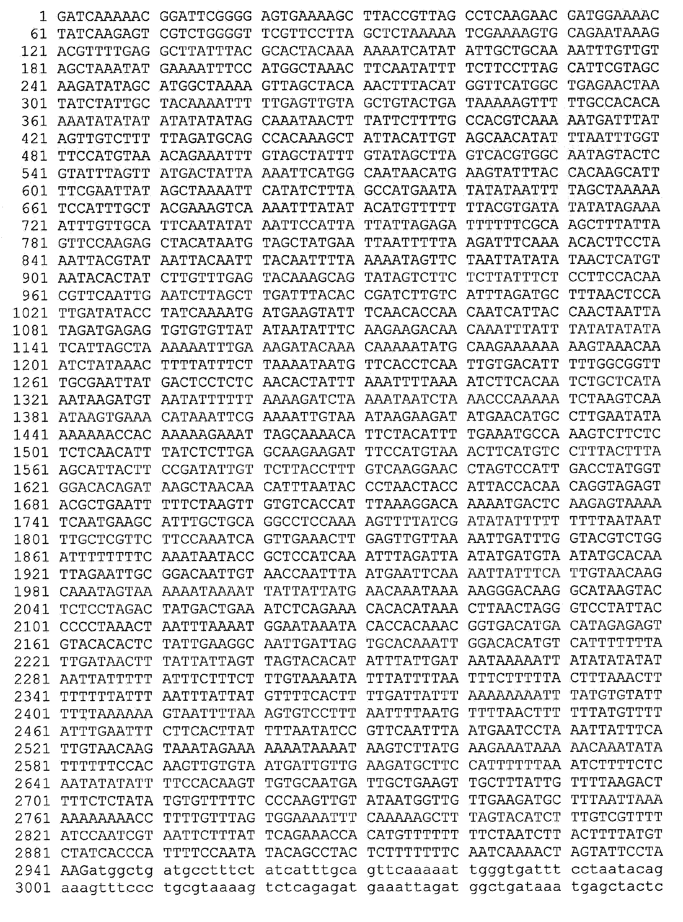

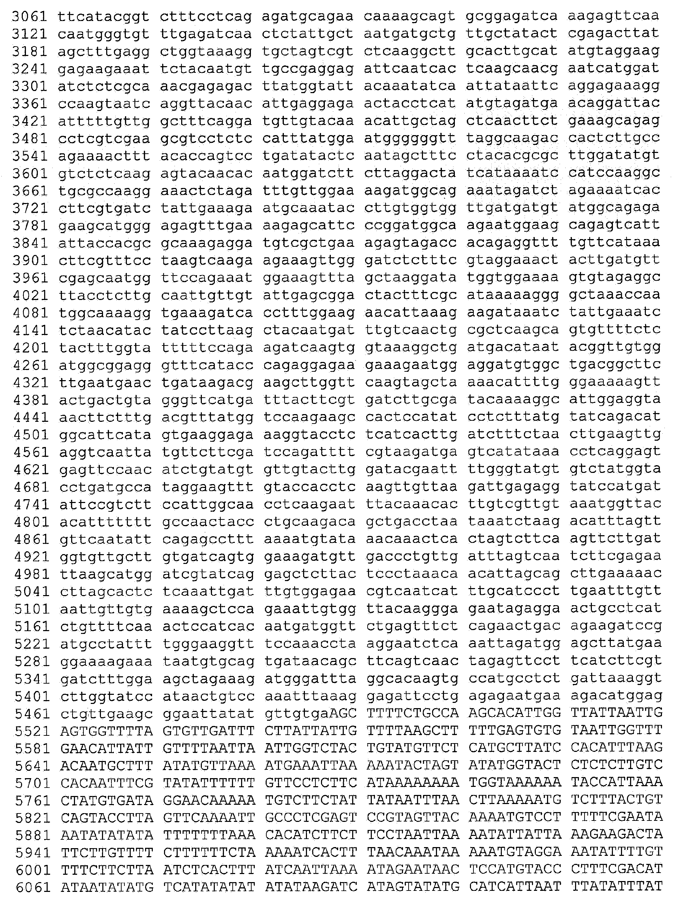

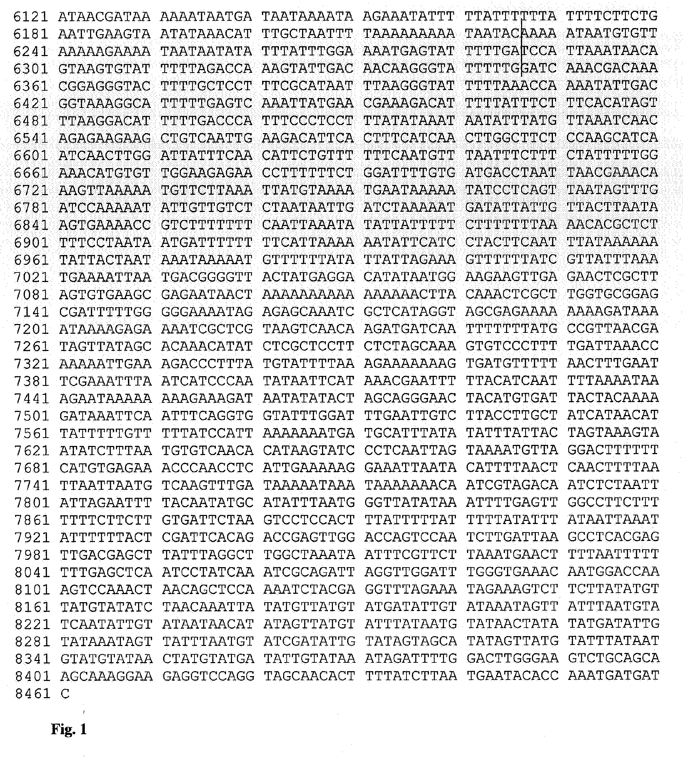

**Figure 1****.** Nucleotide sequence of clone Blb25-B2 (8461 bp) containing the *Rpi-blb3* gene and regulatory sequences. The Rpi-blb3 coding region of 2544 bp is highlighted in lower case (2944-5487). The upstream 2732 nt (211-2942) and the downstream 882 nt (5488-6370) harbour the regulatory sequences.

**Figure 2****.** Deduced Rpi-blb3 protein sequence. The amino-acid sequence deduced from the DNA sequence of Rpi-blb3 is divided into three domains (CC, NB-ARC and LRR).
Hydrophobic residues in the CC domain are underlined. Conserved motifs in R proteins are written in italic in the NBS domain. Residues matching the consensus of the cytoplasmic LRR are indicated in bold in the LRR domain.

**Figure 3****.** shows the StTLRP delta 7 sequences; the intron and the 3'UTR are underlined.

**Figure 4****.** Sequence alignment of Rpi-sto1, Rpi-pta1 and Rpi-blb1. A. DNA sequence alignment. B. Amino acid sequence alignment.

**Figure 5****.** Binary vector pBINAW2b::R3a with reduced T-DNA borders.

**Figure 6****.** Binary vector pBINAW4a::R3a containing the GBSS inverted repeat cDNA and the Phytophthora infestans resistance gene R3a.

## Claims

1. A method for determining whether a plant has been provided with a recombinant nucleic acid that comprises a nucleic acid of interest, comprising further providing said recombinant nucleic acid with a functional R-gene, transferring said recombinant nucleic acid to a plant cell, producing a plant from said cell, and exposing the resulting plant to at least one elicitor (or strain) of Phytophthora and determining the presence or absence of a reaction to said at least one elicitor (or strain).

2. A method according to claim 1, wherein said recombinant nucleic acid essentially consists of Solanum nucleic acid sequences.

3. A method according to claim 1 or 2, further comprising determining the presence or absence of non-Solanum nucleic acid sequences in the resulting plant.

4. A method according to claim 3, wherein said non-Solanum nucleic acid sequences are non-Solanum T-DNA border sequences.

5. A method according to any one of claims 1 to 4, wherein said nucleic acid of interest and said functional R-gene are the same.

6. A method according to any one of claims 1 to 5, wherein said nucleic acid of interest is a functional R-gene.

7. A method according to any one of claims 1 to 6, wherein said reaction is a hypersensitive response (HR).

8. A method according to any one of claims 1 to 7, wherein said recombinant nucleic acid is integrated into the genome of said plant.

9. A method according to any one of claims 1 to 8, wherein said nucleic acid of interest and/or said functional R-gene is/are cDNA sequences.

10. A method according to any one of claims 1 to 8, wherein said nucleic acid of interest is an inverted (repeat) sequence.

11. A method according to any one of claims 1 to 78, wherein said nucleic acid of interest and/or said functional R-gene is/are genomic sequences.

12. A method according to any one of claims 1 to 11, wherein said nucleic acid of interest and/or said functional R-gene is/are under control of native 5' and 3' nucleic acid sequences.

13. A method according to any one of claims 1 to 12, wherein said functional R-gene is a gene encoding Rpi-blb3 or a gene encoding a functional fragment thereof or a gene encoding a derivative thereof.

14. A method according to claim 13, wherein said functional R-gene is a gene encoding Rpi-blb-3 as depicted in Figure 2.

15. Use of a functional Phytophthora R-gene as a selection marker in plant transformation.

16. Use according to claim 15, wherein said R-gene is a gene encoding Rpi-blb3 or a gene encoding a functional fragment thereof or a gene encoding a derivative thereof.

17. Use according to claim 16, wherein said functional R-gene is a gene encoding Rpi-blb-3 as depicted in Figure 2.

18. A plant obtainable by a method according to any one of claims 1 to 17.

19. A method for obtaining a plant that has been provided with a nucleic acid of interest comprising providing
a recombinant nucleic acid comprising said nucleic acid of interest transferring said recombinant nucleic acid to a plant cell
producing a plant from said cell
and determining the presence or absence of said nucleic acid of interest, wherein said nucleic acid of interest is essentially a plant nucleic acid.

20. A method according claim 19, wherein said nucleic acid of interest is a cDNA sequence.

21. A method according to claim 19 or 20, wherein said nucleic acid of interest is an inverted (repeat) sequence.

22. A method according to any one of claims 19 to 21, wherein said nucleic acid of interest is a genomic sequences.

23. A method according to any one of claims 19 to 22, wherein said plant nucleic acid is a Solanaceae nucleic acid.

24. A method according to any one of claims 19 to 23, wherein said plant nucleic acid is an open reading frame that is under control of native 5' and 3' nucleic acid sequences.

25. A method according to any one of claims 19 to 24, wherein said nucleic acid of interest is an R-gene.

26. A method according to any one of claim 19 to 25, further comprising determining the presence or absence of non-Solanum nucleic acid sequences in said plant or cell.

27. A method according to claim 26, wherein said non-Solanum nucleic acid sequences are non-Solanum T-DNA border sequences.

28. A method according to any one of claims 19 to 27, wherein said recombinant nucleic acid is integrated into the genome of said plant.

29. A plant obtainable by a method according to any one of claims 19 to 28.

30. A transgenic plant that has been provided with a cisgene.

31. A plant according to claim 30, wherein said cisgene is under control of native 5' and 3' sequences.

32. A plant according to claim 30 or 31, wherein said cisgene is a genomic sequence.

33. A plant according to any one of claims 29 to 32 which plant is free from non-Solanum T-DNA border sequences.
